# EUROPEAN PATENT APPLICATION

(11) **EP 4 650 349 A1**
(43) Date of publication of application: **19.11.2025**
(21) Application number: 24741366.9
(22) Date of filing: 12.01.2024
(51) Int. Cl.: C07D 295/13, C07C 237/22, C07C 323/52, A61K 9/127, A61K 47/18

(54) **COMPOUND, PREPARATION METHOD, AND LIPID NANOPARTICLES AND PHARMACEUTICAL COMPOSITION THEREOF**

(30) Priority: 13.01.2023 CN 202310038713
(71) Applicant: Suzhou Healirna Biotechnology Co., Ltd., Suzhou, Jiangsu 215000 (CN)
(72) Inventor: ZHONG, Tianyi, Suzhou, Jiangsu 215000 (CN); JIA, Kan, Suzhou, Jiangsu 215000 (CN); LIU, Bing, Suzhou, Jiangsu 215000 (CN); XU, Zebin, Suzhou, Jiangsu 215000 (CN); ZHOU, Wujun, Suzhou, Jiangsu 215000 (CN); SUN, Jingbo, Suzhou, Jiangsu 215000 (CN); DENG, Shuang, Suzhou, Jiangsu 215000 (CN); HU, Hongpeng, Suzhou, Jiangsu 215000 (CN); LU, Qingqing, Suzhou, Jiangsu 215000 (CN); JIANG, Jianhao, Suzhou, Jiangsu 215000 (CN)
(74) Representative: Gille Hrabal Partnerschaftsgesellschaft mbB Patentanwälte
(86) International application number: PCT/CN2024/071975
(87) International publication number: WO 2024/149370

(57) **Abstract**

The invention provides a compound, its preparation method, and lipid nanoparticles, as well as a pharmaceutical composition. The compound includes at least one structural formula (I-1) and may be provided as a pharmaceutically acceptable salt, prodrug, or isomer. It increases structural diversity among lipid molecules and is biodegradable. The L1a, L1b, and L1c groups in the compound can be interchanged with L2, enabling the integration of other molecules like steroids or peptides. This interchangeability expands the functionality of cationic lipid compounds, allowing for the direct coupling of functional molecules to the lipid compound to enhance its functional scope. The replaceable L groups offer greater flexibility in selecting L1a, L1b, L1c, and L2.

## Description

### Field of the Invention

The present invention generally relates to lipidic substances similar to dipptides, which can be used in combination with other lipid components, such as neutral lipids, cholesterol, and polymer-bound lipids, to form lipid nanoparticles. These nanoparticles are designed for delivering therapeutic agents, both *in vitro* and *in vivo.* The therapeutic agents include nucleic acid molecules and nucleic acid analogs like locked nucleic acid (LNA), peptide nucleic acid (PNA), and morpholino. The delivery aims to achieve therapeutic or preventive purposes, including vaccination.

### Background technology

RNA-based therapies have drawn considerable attention for their potential in treating various diseases. Over the past decade, several RNA-based therapies have been approved for different indications, such as macular degeneration, spinal muscular atrophy, hypercholesterolemia, and TTR-mediated amyloidosis. Recently, lipid nanoparticle formulations of mRNA, BNT162b2 and mRNA-1273, received Emergency Use Authorization (EUA) from the FDA and EMA as COVID-19 vaccines. RNA molecules, including ASOs, siRNA, miRNA, and mRNA, play key roles in genetic information transcription and protein synthesis.

mRNA, in particular, is crucial for encoding specific proteins and regulating post-translational modifications. Its short half-life allows for transient protein expression, making mRNA a promising therapeutic agent for vaccine development, allergen tolerance, and disease treatment, including veterinary vaccines.

However, successful delivery of these RNA-based therapies to target cells faces multiple biological barriers, including extracellular and intracellular obstacles. The extracellular matrix (ECM) is the first line of defense against foreign substances and can hinder RNA transfer into target cells. Intracellular barriers include the cell membrane and endosomal entrapment. Once administered intravenously, RNA-carrying nanoparticles must protect RNA from rapid degradation by serum ribonucleases (RNases), escape phagocytosis, cross vascular endothelial cells, and reach the extracellular space of target cells. Cells can internalize RNA-carrying nanoparticles through various mechanisms, after which the nanoparticles must escape the endosome before lysosome formation to avoid enzymatic degradation.

Effective cellular delivery of RNA molecules is crucial for successful RNA-based therapies. Ideal RNA delivery carriers should offer high efficiency, low toxicity, and high cell specificity. Lipid nanoparticles (LNPs) are considered ideal carriers and typically consist of ionizable lipids, phospholipids, cholesterol, and PEG-lipids. Each component is essential for effective nucleic acid delivery and particle stability. However, ionizable lipids can cause in vivo toxicity. Incorporating biodegradable groups like ester, amide, and disulfide bonds into compounds is a common strategy to mitigate this issue.

Designing carriers with reduced in vivo toxicity and improved targeting is crucial for LNP delivery. Multi-component reactions (MCRs) have been used to synthesize peptidic compounds, such as ceramide sphingolipids and derivatives. This invention uses a "one-pot" MCR to assemble ionizable head groups and bioactive tails on a dipeptide scaffold or to form multimers. This approach leverages the high yields, broad substrate scope, and mild conditions of MCRs to incorporate elements beneficial for nucleic acid delivery and targeting. Furthermore, this patent describes the one-pot synthesis of dipeptide-like lipids via MCRs, introducing ester bonds into the final product using unique ester-containing components. These dipeptide-like lipids, with two amide groups on their scaffolds, provide additional biodegradable structures, potentially reducing in vivo toxicity.

### SUMMARY OF THE INVENTION

In one embodiment, this article provides dipptide-like lipid compounds, including their pharmaceutically acceptable salts, prodrugs, or stereoisomers, which can be used alone or in combination with other lipid components, such as neutral lipids, charged lipids, sterols (including all sterols), and/or their analogs and/or polymer-bound lipids and/or polymers, to form lipid nanoparticles for delivering therapeutic agents (e.g., nucleic acid molecules, including nucleic acid analogs like LNA, PNA, and morpholino). In some cases, these lipid nanoparticles are used to deliver nucleic acids, such as antisense and/or messenger RNA. Methods of using these lipid nanoparticles to treat various diseases or conditions, such as those caused by infections and/or protein deficiencies, are also provided.

In one aspect, this patent provides a compound that comprises at least one structural formula (I-1): 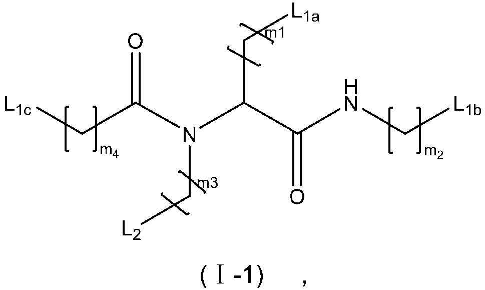
or the compound is a pharmaceutically acceptable salt, prodrug, or isomer thereof; wherein,
m1, m2, m3, and m4 are each independently selected from integers between 1 and 18, inclusive;
L1a, L1b, and L1c are each independently selected from the group consisting of: -H, -S-SR1, -SR1, -C(=O)OR1, -OC(=O)R1, -N(R2)R1, -C(=O)N(R2)R1-, -N(R2)C(=O)R1-, -C(=O)NR1, and -OR1;
R1 and R2 are each independently selected from the group consisting of: -H, alicyclic hydrocarbons, 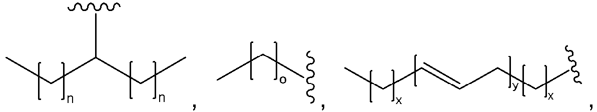 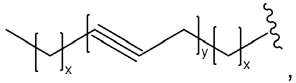 in a composition of the present disclosure, n, x and o are each independently selected from integers of 0 to 12, y is independently selected from 1, 2, 3 or 4;
L₂ is independently selected from the group consisting of: -R3XR4Y, -R3Y, -R3YR5, -Y, -R3XR4(Z)Y;
R₃, R₄ and R₅ are each independently selected from the group consisting of: C1-C5 alkane, C2-C5 alkene;
X is independently selected from the group consisting of: -O-, -N(Rx)-, -S-, -S-S-, -OC(=O)-, -C(=O)O-, -OP(=O)O-, -OS(=O)O-, -C(=O)N-, -N(C(=O))-;
Rx is independently selected from the group consisting of: -H, -(CH2)m-OH, -(CH2)m-CN, -(CH2)m and alicyclic hydrocarbons, wherein m is independently selected from 0, 1, 2, 3, 4 or 5;
Y is independently selected from the group consisting of: -(CH2)m-OH, -(CH2)m-CN, -(CH2)m-N(R**)R*, -(CH2)m-G-(CH2)n, wherein m and n are each independently selected from 0, 1, 2, 3, 4 or 5;
G is independently selected from aryl or heterocyclic;
R* and R** are each independently selected from the group consisting of: H, C1-C3 alkane, -(CH2)mQ, wherein m is independently selected from 1, 2, 3 or 4;
Q is independently selected from the group consisting of: -OH, -CN;
Z is independently selected from the group consisting of: -(CH2)m-OH, -(CH2)m-CN, -(CH2)m-N(R**)R*, -(CH2)m-G-(CH2)n, wherein m and n are each independently selected from 0, 1, 2, 3, 4 or 5;
G is independently selected from aryl or heterocyclic;
R* and R** are each independently selected from the group consisting of: H, C1-C3 alkane, -(CH2)aQ, wherein a is independently selected from 1, 2, 3 or 4;
Q is independently selected from the group consisting of: -OH, -CN.

The present patent also provides a compound which comprises at least one structural formula (I-1): 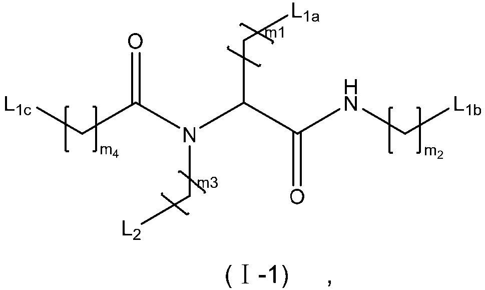 The compound herein may also be provided as its pharmaceutically acceptable salts, prodrugs, or isomers. In the compound:
m1, m2, m3, and m4 are each independently selected from integers between 1 and 18, inclusive.
L1a, L1b, and L1c are each independently selected from the group consisting of: -H, -S-SR1, -SR1, -C(=O)OR1, -OC(=O)R1, -N(R2)R1, -C(=O)N(R2)R1, -N(R2)C(=O)R1, -C(=O)NR1,-OR1;
R1 and R2 are each independently selected from the group consisting of: -H, alicyclic hydrocarbons, 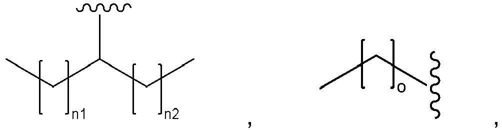 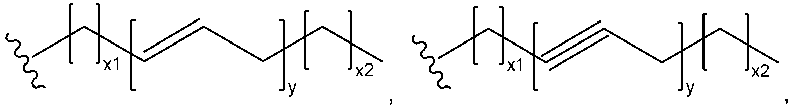 in the compound,
n1, n2, x1, x2, and o are each independently selected from integers of 0 to 12, and y is independently selected from 1, 2, 3, or 4;
L2 is independently selected from the group consisting of: -R3XR4M, -R3XR4Y-H, -R3M, -R3YR5, -M, -R3XR4(Z)M;
R3, R4, and R5 are each independently selected from the group consisting of: C1-C5 alkane and C2-C5 alkene;
X is independently selected from the group consisting of: -O-, -N(Rx)-, -S-, -S-S-, -OC(=O)-, -C(=O)O-, -OP(=O)O-, -OS(=O)O-, -C(=O)N-, -N(C(=O))-;
Rx is independently selected from the group consisting of: -H, -(CH2)m-OH, -(CH2)m-CN, -(CH2)mH, and alicyclic hydrocarbons, wherein m is independently selected from 0, 1, 2, 3, 4, or 5.
M is independently selected from the group consisting of: -(CH2)m-OH, -(CH2)m-CN, -(CH2)m-N(R**)R*, wherein m is independently selected from 0, 1, 2, 3, 4, or 5.
Y is independently selected from the group consisting of: -(CH2)m-G-(CH2)n-, wherein m and n are each independently selected from 0, 1, 2, 3, 4, or 5.
G is independently selected from aryl or heteroaryl.
R* and R** are each independently selected from the group consisting of: H, C1-C3 alkyl, and -(CH2)mQ, wherein m is independently selected from 1, 2, 3, or 4.
Q is independently selected from the group consisting of: -OH, -CN, 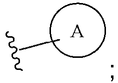 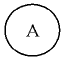 is independently selected from the group consisting of: 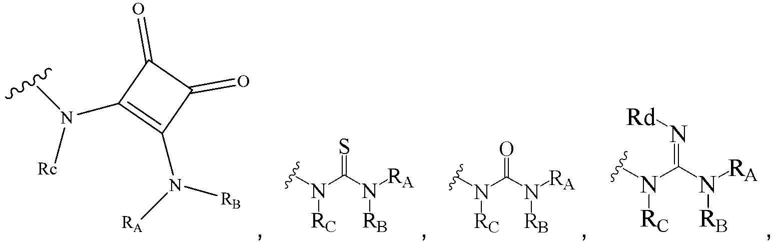 aryl or heterocyclic compounds;
RA, RB, and RC are each independently selected from the group consisting of: -H, -(CH2)m-H;
m is independently selected from 1, 2, and 3;
RD is independently selected from the group consisting of: -H, -(CH2)n-H, -CN, -NO2, -NH2, -N(CH3)CH3;
n is independently selected from 1, 2, and 3;
Z is independently selected from the group consisting of: -(CH2)m-OH, -(CH2)m-CN, -(CH2)m-N(R**)R*, -(CH2)m-G-(CH2)n-H, wherein m and n are each independently selected from 0, 1, 2, 3, 4, or 5;
G is independently selected from aryl or heteroaryl;
R* and R** are each independently selected from the group consisting of: H, C1-C3 alkyl, -(CH2)aQ, wherein a is independently selected from 1, 2, 3, or 4;
Q is independently selected from the group consisting of: -OH, -CN; 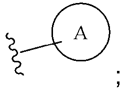 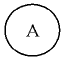 is independently selected from the group consisting of: 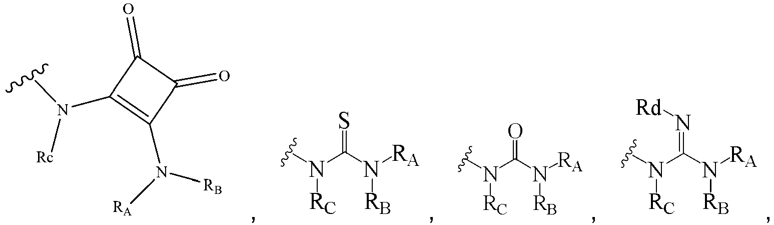 aryl or heterocyclic compounds;
RA, RB, and RC are each independently selected from the group consisting of: -H, -(CH2)m-H;
m is independently selected from 1, 2, and 3.
RD is independently selected from the group consisting of: -H, -(CH2)n-H, -CN, -NO2, -NH2, -N(CH3)CH3;
n is independently selected from 1, 2, and 3.
As a preferred embodiment of the compounds described in this patent, the positions of L1a, L1b, and L1c can be interchangeably or replaceably connected with L2, and/or the position of L2 can be interchangeably or replaceably connected with L1a, L1b, or L1c;

The number of L2 groups in the structural formula is at least one and no more than two.

As another preferred embodiment of the compounds described in this patent, Rx is alicyclic, and Rx is alicyclic having at least one of the structures (II-1), (II-2), (II-3), and (II-4). The structures (II-1), (II-2), (II-3), and (II-4) are as follows:

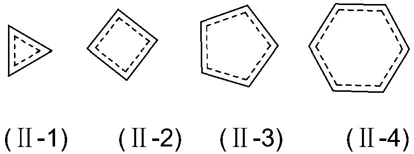

**As** a preferred embodiment of the compounds described in this patent, Rx may be a pharmaceutically acceptable salt, prodrug, or isomer of the structural formula (II-1), (II-2), (II-3) or (II-4);
wherein the "=" bond in the structural formula (II-1), (II-2), (II-3) and (II-4) is a single or double bond.

As a preferred embodiment of the compounds described in this patent, R1 is alicyclic, and R1 is a vitamin E succinate, adamantane or its derivative;
R2 is alicyclic, and R2 is a vitamin E succinate, adamantane or its derivative.

As a preferred embodiment of the compounds described in this patent, G is a heterocycle, and G is a nitrogen-containing heterocycle, oxygen-containing heterocycle or sulfur-containing heterocycle compound.

More preferably, G is acridine, acetidine, furan, pyrrole, thiophene, pyrazole, imidazole, oxazole, isoxazole, thiazole, isothiazole, pyridine, pyran, thiopyran, pyridazine, pyrimidine, pyrazine, piperazine, indole, quinoline, pteridine or morpholine.

As a preferred embodiment of the compounds described in this patent, the parent structure of the compound is prepared by the Ugi reaction (i.e., Ugi reaction) from amine, carboxylic acid, aldehyde and isonitrile.

As a preferred embodiment of the compounds described in this patent, the structures L1a, L1b, and L1c are each interchangeably or replaceably connectable with L2, and/or the structure L2 is interchangeably or replaceably connectable with L1a, L1b, or L1c;
wherein the number of L2 groups in each structural formula is at least one and no more than two.

As a preferred embodiment of the compounds described in this patent, Rx is alicyclic, and Rx is alicyclic having at least one of the structures (II-1), (II-2), (II-3), and (II-4). The structures (II-1), (II-2), (II-3), and (II-4) are as follows:

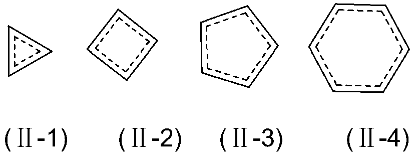

**As** a preferred embodiment of the compounds described in this patent, Rx may be a pharmaceutically acceptable salt, prodrug, or isomer of the structural formula (II-1), (II-2), (II-3) or (II-4),
wherein the "------" bond in the structural formula (II-1), (II-2), (II-3) and (II-4) is a single or double bond.

As a preferred embodiment of the compounds described in this patent, R1 is alicyclic, and R1 is a vitamin E succinate, adamantane or its derivative;
**R2** is alicyclic, and R2 is a vitamin E succinate, adamantane or its derivative.

As a preferred embodiment of the compounds described in this patent, G is a heterocycle, and G is a nitrogen-containing heterocycle, oxygen-containing heterocycle or sulfur-containing heterocycle compound.

More preferably, G is acridine, acetidine, furan, pyrrole, thiophene, pyrazole, imidazole, oxazole, isoxazole, thiazole, isothiazole, pyridine, pyran, thiopyran, pyridazine, pyrimidine, pyrazine, piperazine, indole, quinoline, pteridine or morpholine.

As a preferred embodiment of the compounds described in this patent, the parent structure of the compound is prepared by the Ugi reaction (i.e., Ugi reaction) from amine, carboxylic acid, aldehyde and isonitrile.

In another aspect, the present patent also provides another compound which comprises one or more structural formula (I-2):

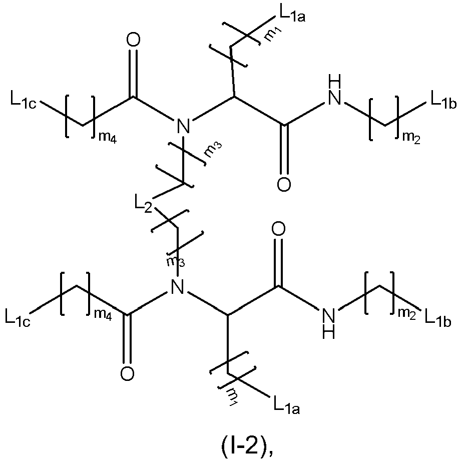

As a preferred embodiment of the compounds described in this patent, the compound may be provided as its pharmaceutically acceptable salts, prodrugs, or isomers.

In the compound:
m1, m2, m3, and m4 are each independently selected from integers between 1 and 18, inclusive. This means that m1, m2, m3, and m4 can each be different integers or the same integer.
L1a, L1b, and L1c are each independently selected from the group consisting of: -H, -S-SR1, -SR1, -C(=O)OR1, -OC(=O)R1, -N(R2)R1, -C(=O)N(R2)R1-, -N(R2)C(=O)R1-, -C(=O)NR1, and -OR1.
R1 and R2 are each independently selected from the group consisting of: -H, alicyclic hydrocarbons, 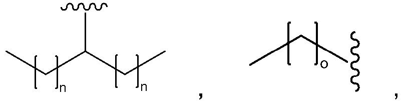 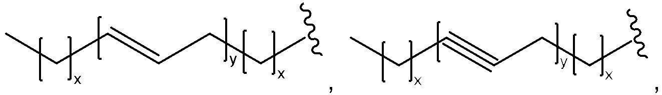 wherein n, x, and o are each independently selected from integers of 0 to 12, and y is independently selected from 1, 2, 3, or 4.
**L2** is independently selected from the group consisting of: -R3XR4Y, -R3Y, -R3YR5, -Y, -R3XR4(Z)Y.
**R3,** R4, and R5 are each independently selected from the group consisting of: C1-C5 alkane and C2-C5 alkene.
**X** is independently selected from the group consisting of: -O-, -N(Rx)-, -S-, -S-S-, -OC(=O)-, -C(=O)O-, -OP(=O)O-, -OS(=O)O-, -C(=O)N-, -N(C(=O))-.
**Rx** is independently selected from the group consisting of: -H, -(CH2)m-OH, -(CH2)m-CN, -(CH2)m, and alicyclic hydrocarbons, wherein m is independently selected from 0, 1, 2, 3, 4, or 5.
**Y** is independently selected from the group consisting of: -(CH2)m-OH, -(CH2)m-CN, -(CH2)m-N(R**)R*, -(CH2)m-G-(CH2)n, wherein m and n are each independently selected from 0, 1, 2, 3, 4, or 5.
G is independently selected from aryl or heteroaryl.
**R*** and R** are each independently selected from the group consisting of: H, C1-C3 alkyl, and -(CH2)mQ, wherein m is independently selected from 1, 2, 3, or 4.
**Q** is independently selected from the group consisting of: -OH and -CN.
**Z** is independently selected from the group consisting of: -(CH2)m-OH, -(CH2)m-CN, -(CH2)m-N(R**)R*, -(CH2)m-G-(CH2)n, wherein m and n are each independently selected from 0, 1, 2, 3, 4, or 5.
G is independently selected from aryl or heteroaryl.
**R*** and R** are each independently selected from the group consisting of: H, C1-C3 alkyl, and -(CH2)aQ, wherein a is independently selected from 1, 2, 3, or 4.
**Q** is independently selected from the group consisting of: -OH and -CN.
**R** is independently selected from the group consisting of: -(CH2)m-, -N-, -S-, -C(=O)O-, -OC(=O)-, -N((CH2)m)-, -C(=O)N-, and -O-, wherein m is independently selected from 1, 2, 3, or 4.

The advantage of formula (I-2) is its high nitrogen content, which allows for tighter encapsulation of therapeutic agents, potentially reducing the amount of lipid compound required.

**As** a preferred embodiment of the compounds described in this patent, the parent structure of the compound is prepared by the Ugi reaction (i.e., Ugi reaction) from amine, carboxylic acid, aldehyde, and isonitrile.

**As** a preferred embodiment of the compounds described in this patent, R1 is alicyclic, and R1 is a vitamin E succinate, adamantane, or its derivative;

**R2** is alicyclic, and R2 is a vitamin E succinate, adamantane, or its derivative.

**In** another aspect, the present invention provides a method for preparing the compound, which specifically comprises the following steps:

Dissolve a first compound in a reaction vessel containing a first solvent. Add a second substance to the reaction vessel and stir for 0.5 to 2 hours. Then, add a third substance and continue stirring for an additional 0.5 to 2 hours. Next, add a fourth substance and stir for 4 to 48 hours. Once the reaction is complete, as determined by appropriate analytical techniques, terminate the reaction. Remove the organic solvent from the reaction mixture under reduced pressure. Finally, purify the resulting product to obtain the desired compound.

The first compound is an amine, the first solvent is ethanol or dichloromethane/methanol, the second substance is an aldehyde, the third substance is a carboxylic acid, and the fourth substance is an isonitrile.

**In** another aspect, the present invention provides a composition comprising steroids, structural lipids, polymer-bound lipids, and the compounds described herein. The composition serves as a carrier for intracellular delivery of therapeutic agents and is preferably a nanoparticle.As a preferred embodiment of the composition described in this patent, the steroid is a steroid of formula III-1.

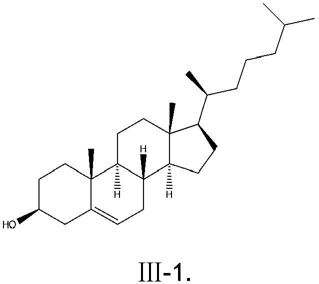

**As** a preferred embodiment of the composition described in this patent, the molar ratio of the compound to the steroid is in the range of approximately 1:2 to 6:1.

More preferably, the molar ratio ranges from 1:0.5 to 1:1. Even more preferably, the molar ratio is 1:0.77.

**As** a preferred embodiment of the composition described in this patent, the polymer-bound lipid includes one or more types of polymer-bound lipids, such as polyinosinic acid, DSPE-PCB20, DMG-PEG2000, DMPE-PEG2000, or C14-PEG2000.

**As** a preferred embodiment of the composition described in this patent, the molar ratio of the compound to the polymer-bound lipid is in the range of approximately 10:1 to 100:1.

More preferably, the molar ratio ranges from 20:1 to 40:1. Even more preferably, the molar ratio is 20:1 or 33:1.

**As** a preferred embodiment of the composition described in this patent, the structural lipid includes at least one of the structures represented by formulas III-2, III-3, and III-4:

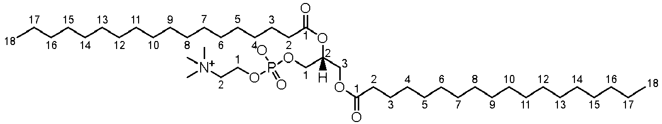

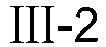

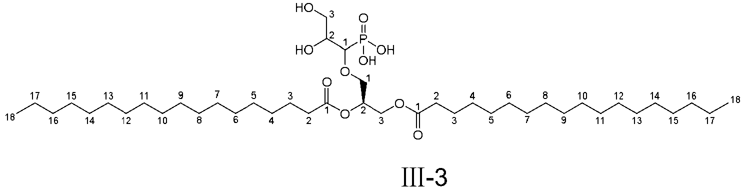

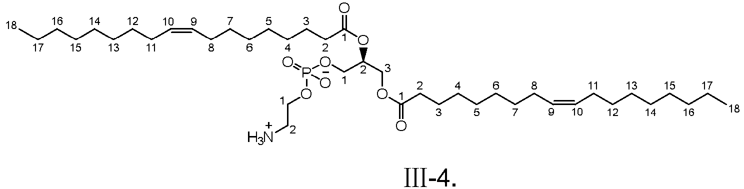

**As** a preferred embodiment of the composition described in this patent, the molar ratio of the compound to the structural lipid is in the range of approximately 2:1 to 10:1.

**As** a preferred embodiment of the composition described in this patent, the composition further comprises a therapeutic or prophylactic agent;

the therapeutic or prophylactic agent comprises a nucleic acid-based drug or a nucleic acid-like drug.

**As** a preferred embodiment of the composition described in this patent, the nucleic acid-based drug includes aptamers, circular RNA, ASO, siRNA, sgRNA, tRNA, or mRNA. Preferably, the mRNA comprises at least one mRNA encoding an antigen or its fragment or epitope. More preferably, the antigen is a pathogenic antigen or a tumor-related antigen. Preferably, the mRNA is a monocistronic or bicistronic mRNA.

**As** a preferred embodiment of the composition described in this patent, the nucleic acid-like drug comprises Locked Nucleic Acid (LNA), Peptide Nucleic Acid (PNA), or Morpholino.

**As** a preferred embodiment of the composition described in this patent, the mRNA comprises one or more functional nucleotide analogs.

Preferably, the functional nucleotide analogs include pseudouridine, 1-methyl-pseudouridine, and 5-methylcytidine.

It should be noted that in the molecules mentioned in this invention, the -(CH2)m type molecule is -(CH2)mH when at the end of a molecule and -(CH2)m- when not at the end.

It should be noted that in the molecules mentioned in this invention, the -(CH2)n type molecule is -(CH2)nH when at the end of a molecule and -(CH2)n- when not at the end.

In another aspect, the present invention provides a lipid nanoparticle comprising the compound or composition as described above.

In another aspect, the present invention provides a pharmaceutical composition comprising the compound, composition, or lipid nanoparticle as described above, and a pharmaceutically acceptable excipient or diluent.

Compared to the prior art, the present invention has at least one of the following advantages:

The compounds of the invention increase structural diversity, thereby increasing the diversity of lipid molecules.

The structure of the compound directly provides biodegradability, which can reduce in vivo toxicity and accelerate metabolism.

The L1a, L1b, L1c, and L2 of the compound can be interchangeably replaced, allowing for the direct incorporation of other types of molecules, such as steroids and peptides, for rapid screening. Steroids can participate in in vivo metabolism to enhance the targeting of the delivery vehicle. In contrast, the prior art typically incorporates steroids as a fifth component of lipid nanoparticles, resulting in weak interactions. This invention directly couples steroids to cationic lipid compounds, strengthening interactions with cargo molecules. Peptides can assist in delivery, enhancing the effectiveness of the composition or lipid nanoparticles, thereby expanding the functionality of cationic lipids. Functional molecules can be directly coupled to cationic lipids without affecting their function, as the other three L structures already fulfill the basic functions of cationic lipids.

The replaceability of L1a, L1b, L1c, and L2 in the compound offers greater flexibility in selecting these groups.

### BRIEF DESCRIPTION OF THE DRAWINGS

The accompanying drawings serve to further illustrate the present invention and should not be construed as limiting the subject matter of the invention.

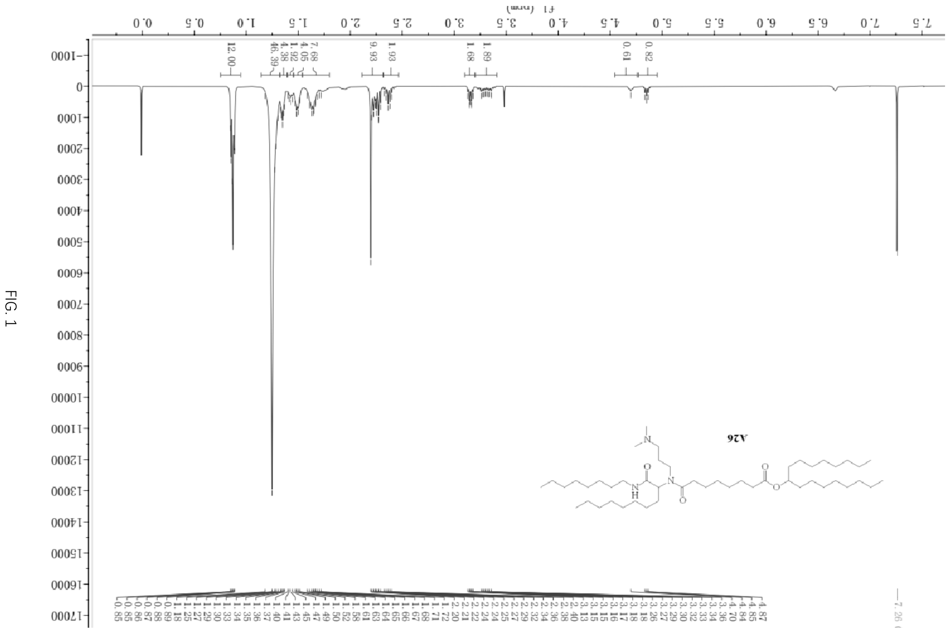

Figure 1: ¹ H NMR spectrum of compound A26 (400 MHz, CDCl₃).

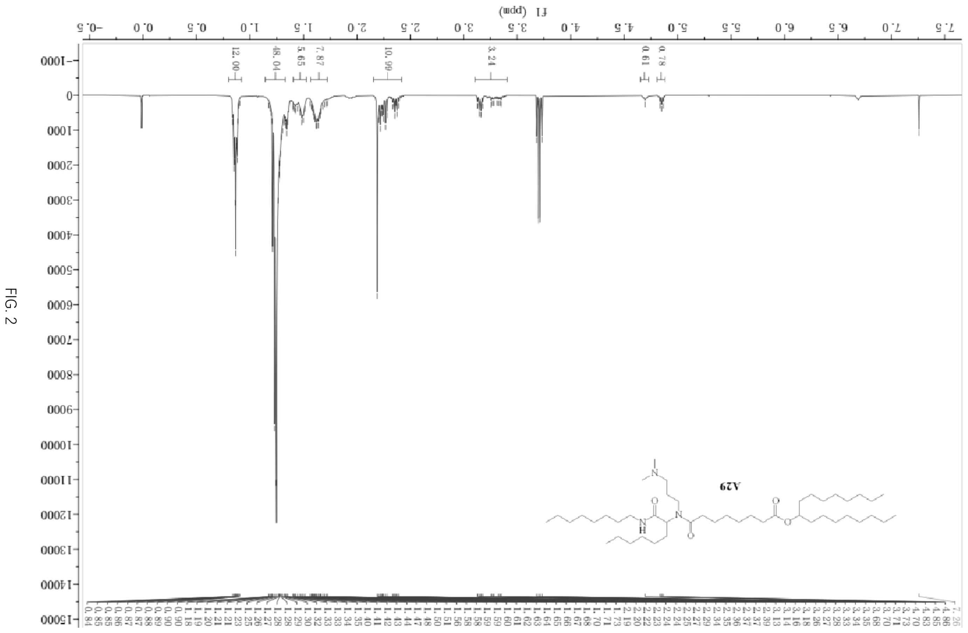

Figure 2: ¹H NMR spectrum of compound A29 (400 MHz, CDCl₃).

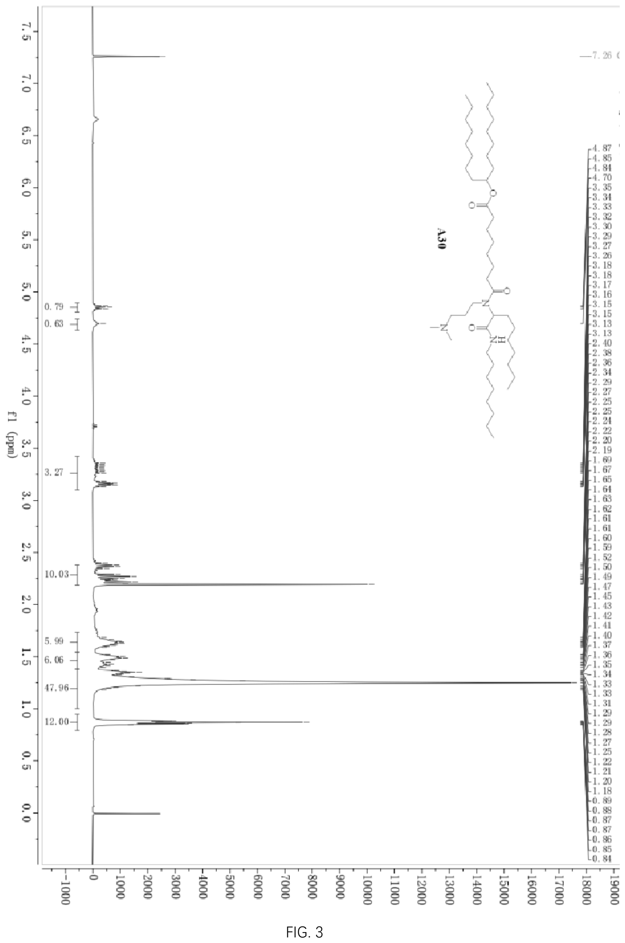

Figure 3: ¹H NMR spectrum of compound A30 (400 MHz, CDCl₃).

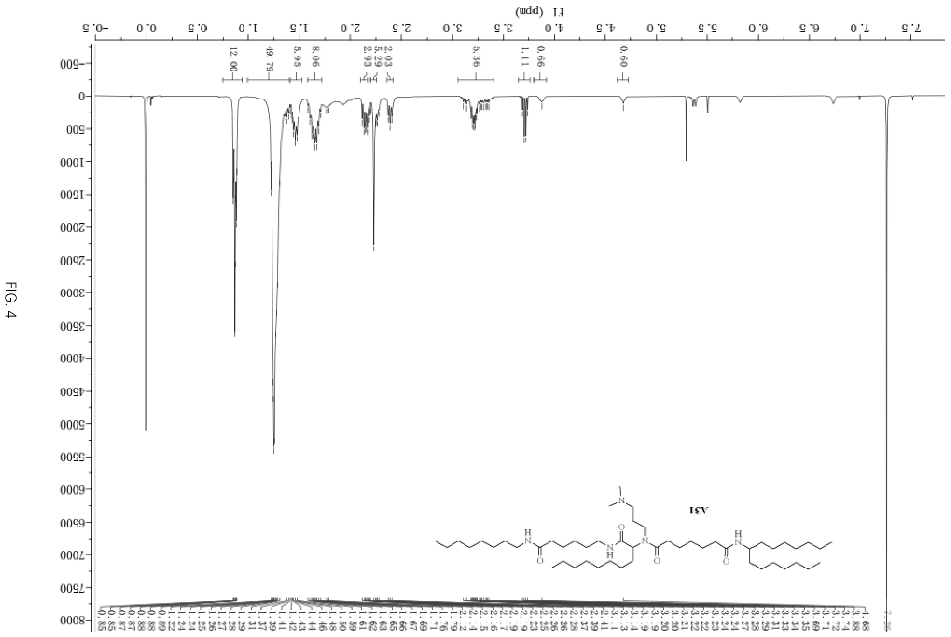

Figure 4: ¹H NMR spectrum of compound A31 (400 MHz, CDCl₃).

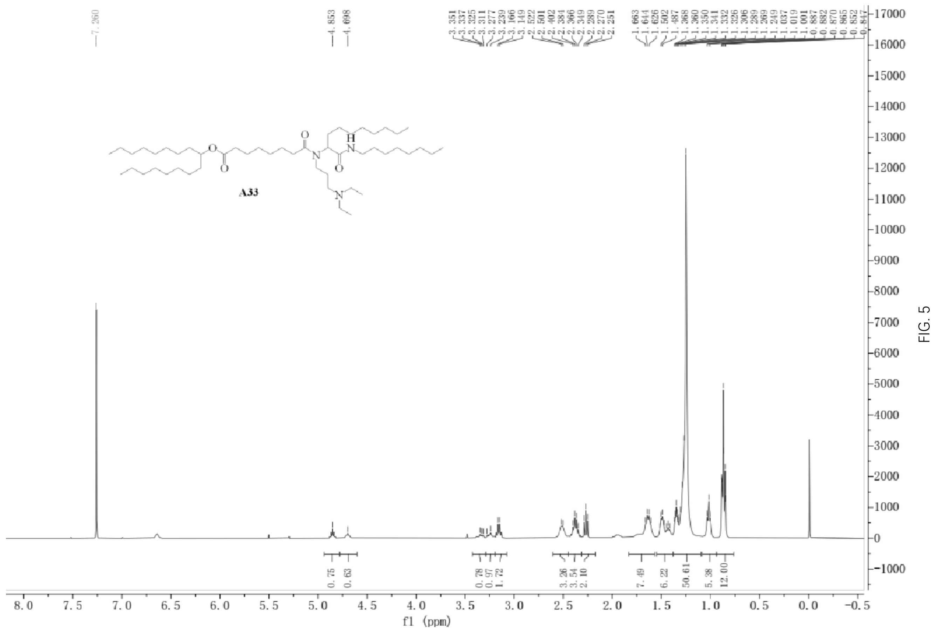

Figure 5: ¹H NMR spectrum of compound A33 (400 MHz, CDCl₃).

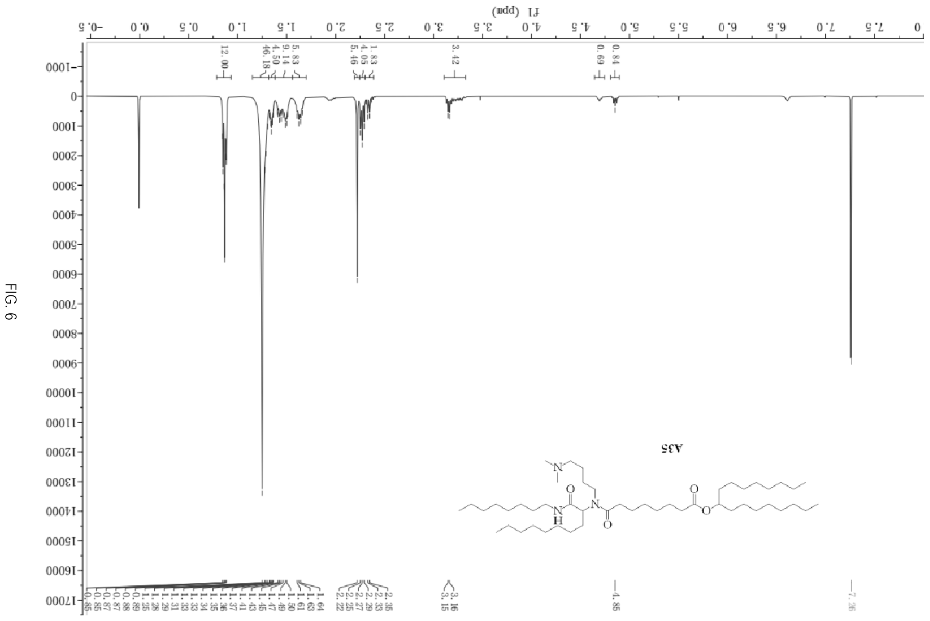

Figure 6: ¹H NMR spectrum of compound A35 (400 MHz, CDCl₃).

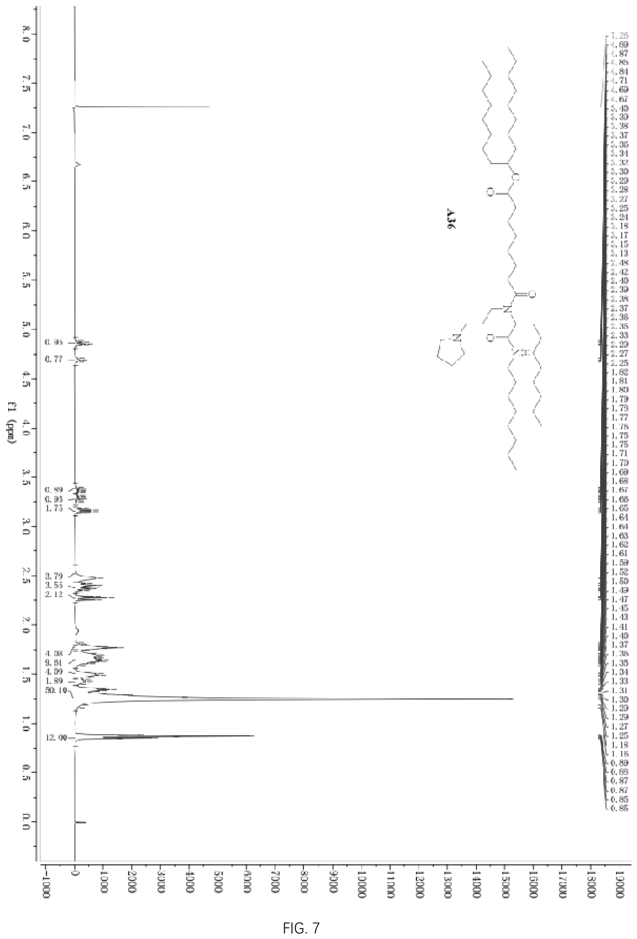

Figure 7: ¹H NMR spectrum of compound A36 (400 MHz, CDCl₃).

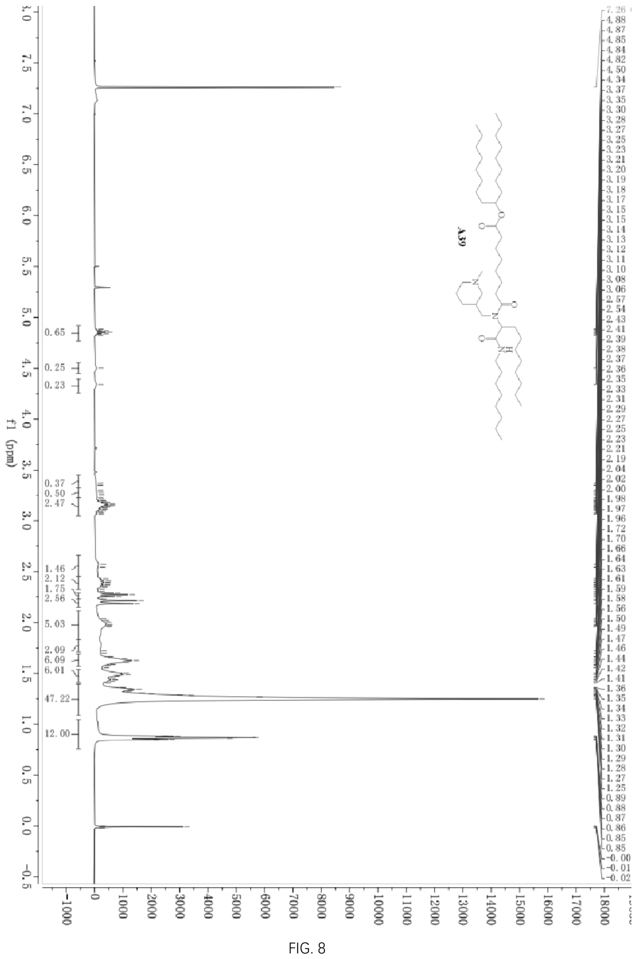

Figure 8: ¹H NMR spectrum of compound A39 (400 MHz, CDCl₃).

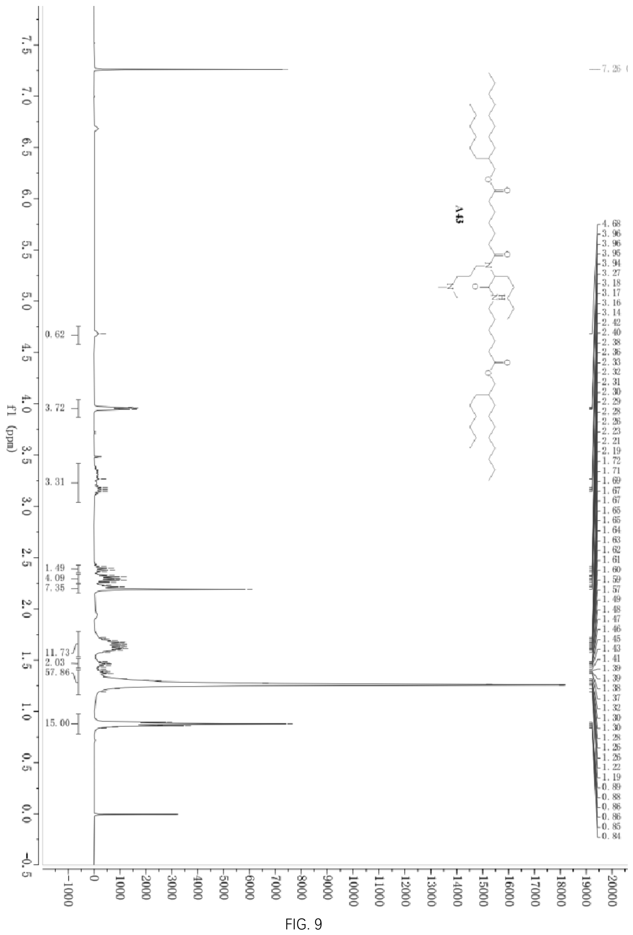

Figure 9: ¹H NMR spectrum of compound A43 (400 MHz, CDCl₃).

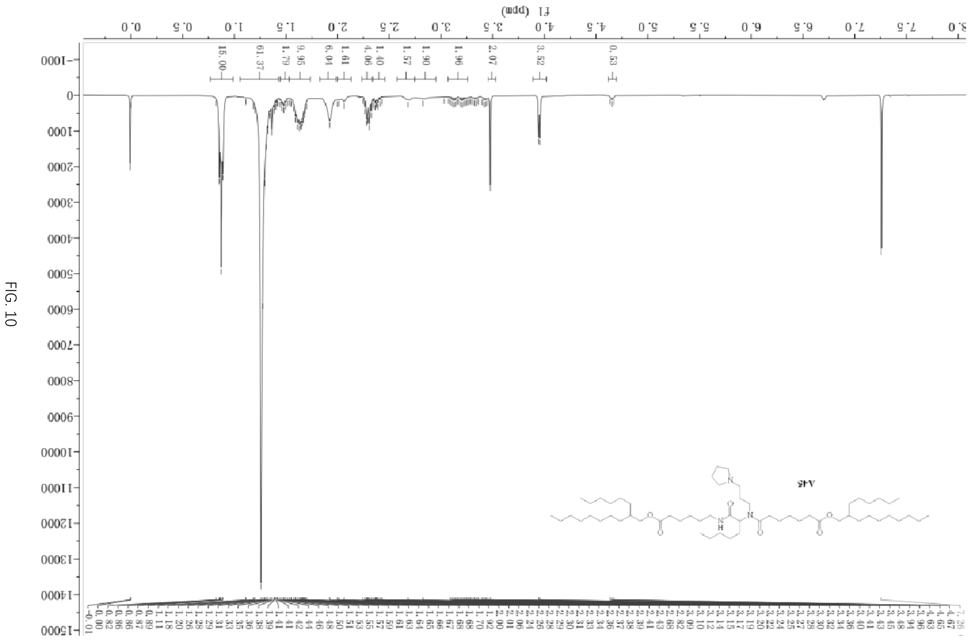

Figure 10: ¹H NMR spectrum of compound A45 (400 MHz, CDCl₃).

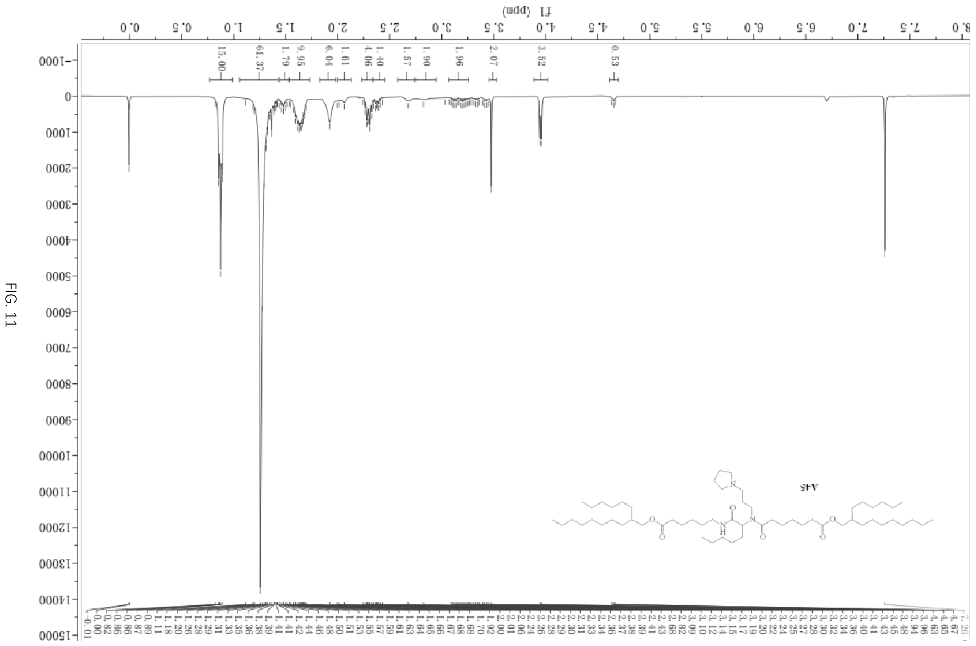

Figure 11: ¹H NMR spectrum of compound A47 (400 MHz, CDCl₃).

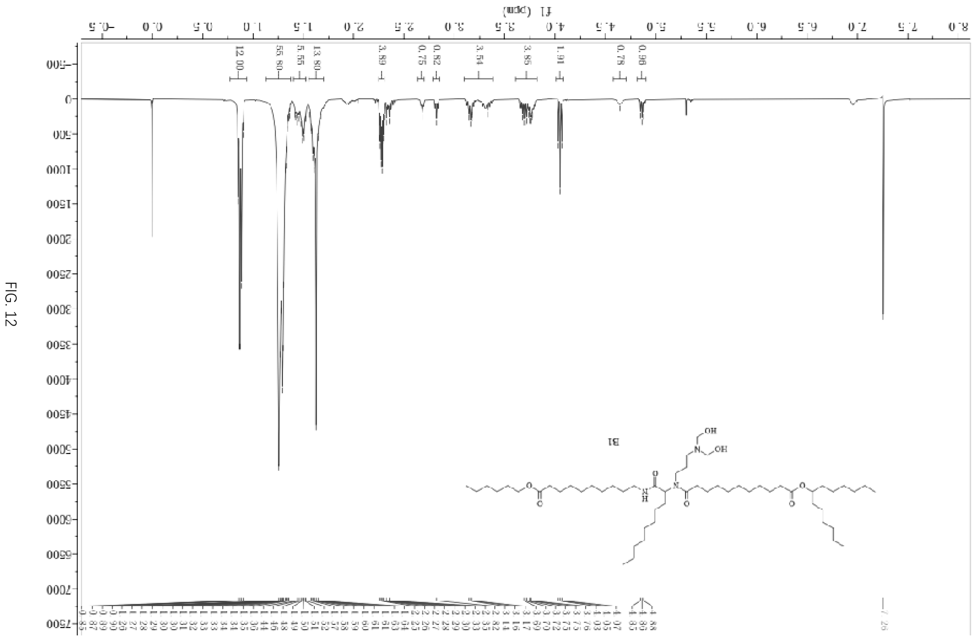

Figure 12: ¹H NMR spectrum of compound B1 (400 MHz, CDCl₃).

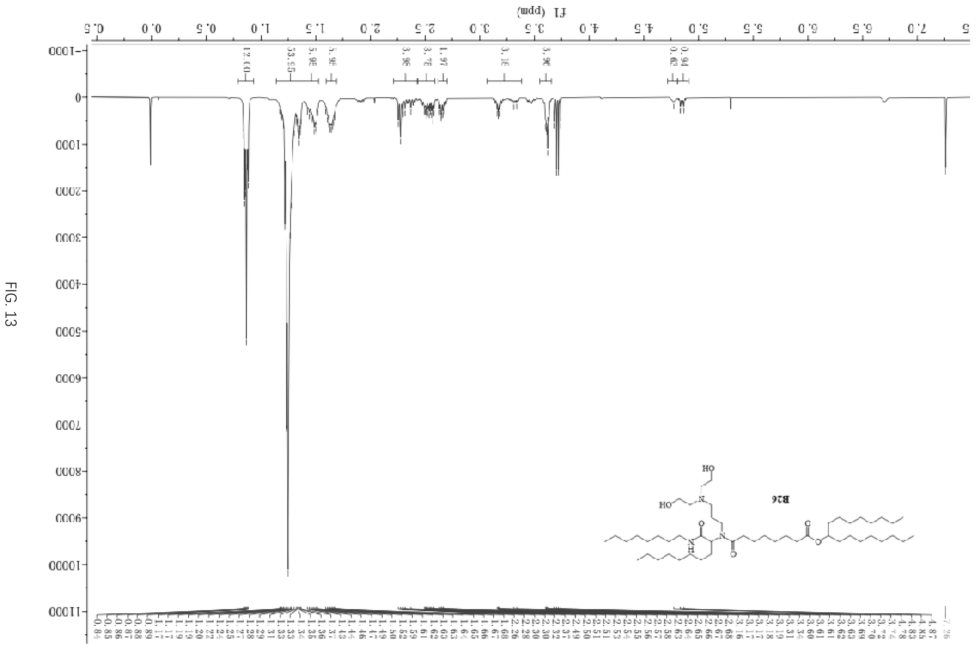

Figure 13: ¹H NMR spectrum of compound B26 (400 MHz, CDCl₃).

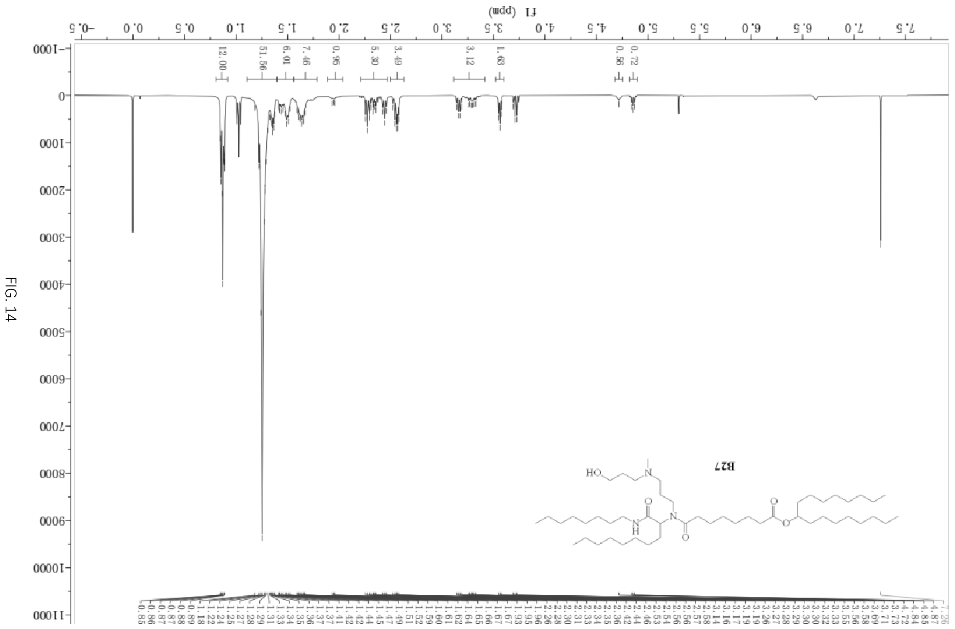

Figure 14: ¹H NMR spectrum of compound B27 (400 MHz, CDCl₃).

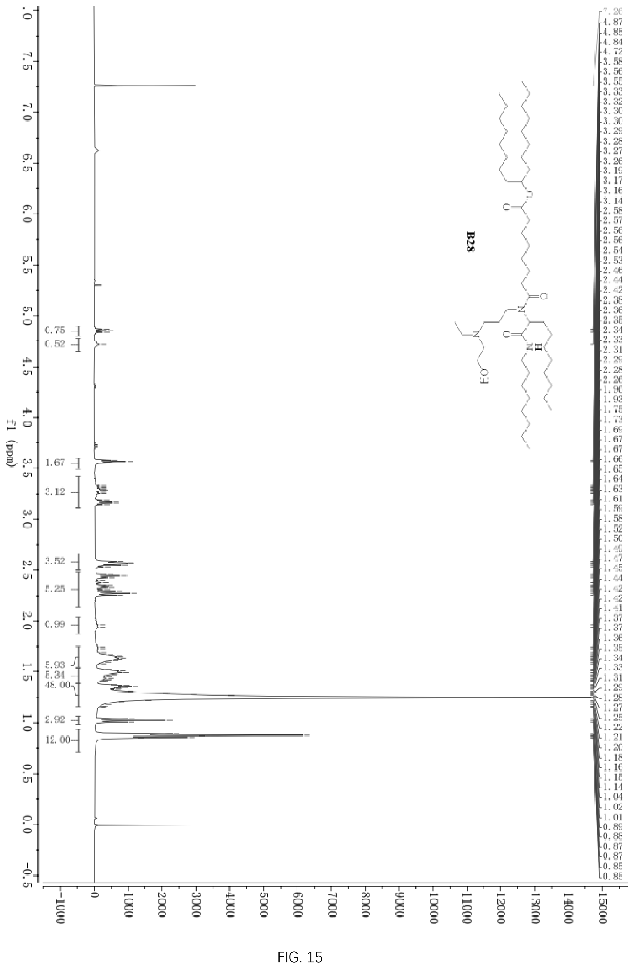

Figure 15: ¹H NMR spectrum of compound B28 (400 MHz, CDCl₃).

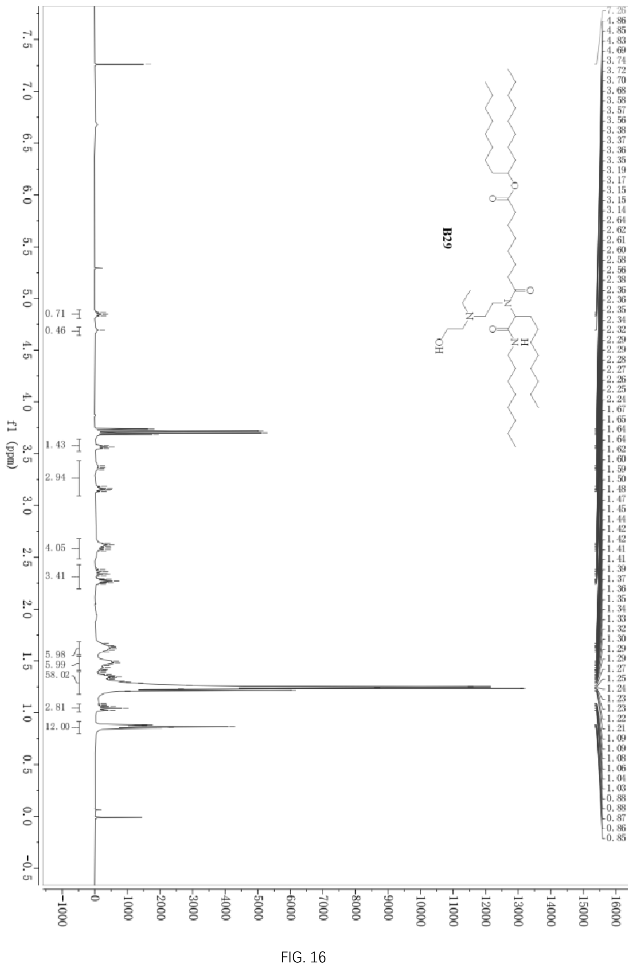

Figure 16: ¹H NMR spectrum of compound B29 (400 MHz, CDCl₃).

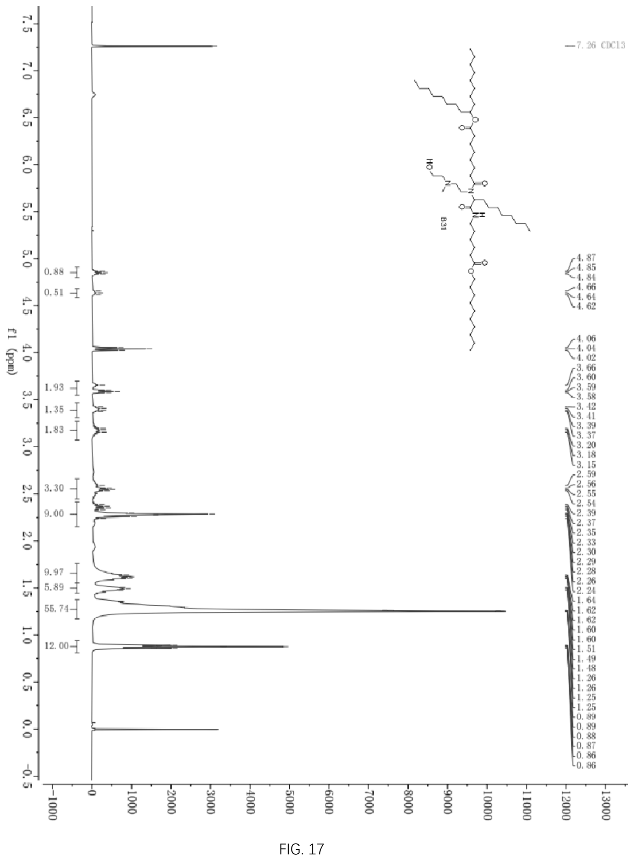

Figure 17: ¹H NMR spectrum of compound B31 (400 MHz, CDCl₃).

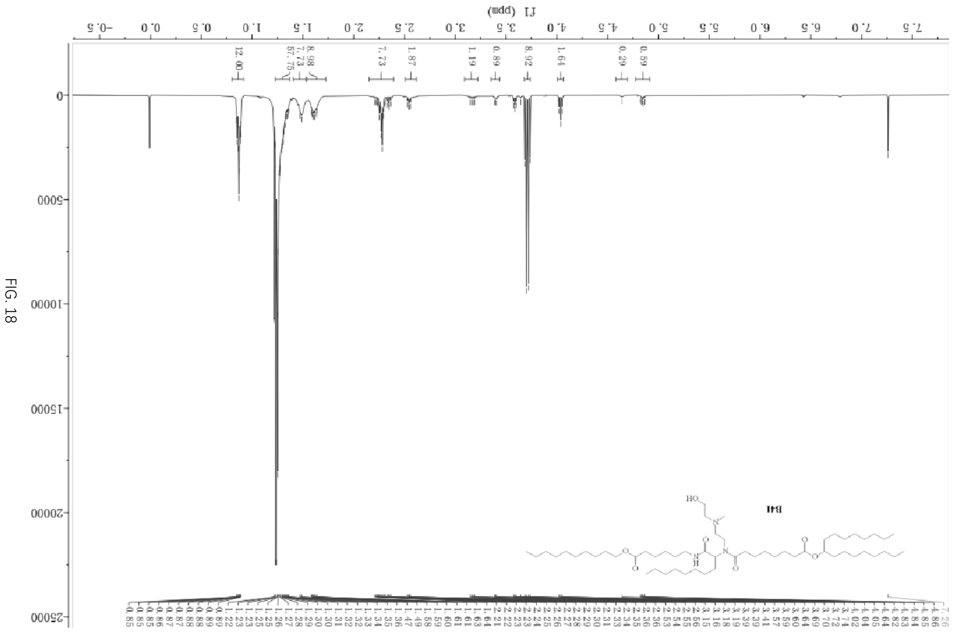

Figure 18: ¹H NMR spectrum of compound B39 (400 MHz, CDCl₃).

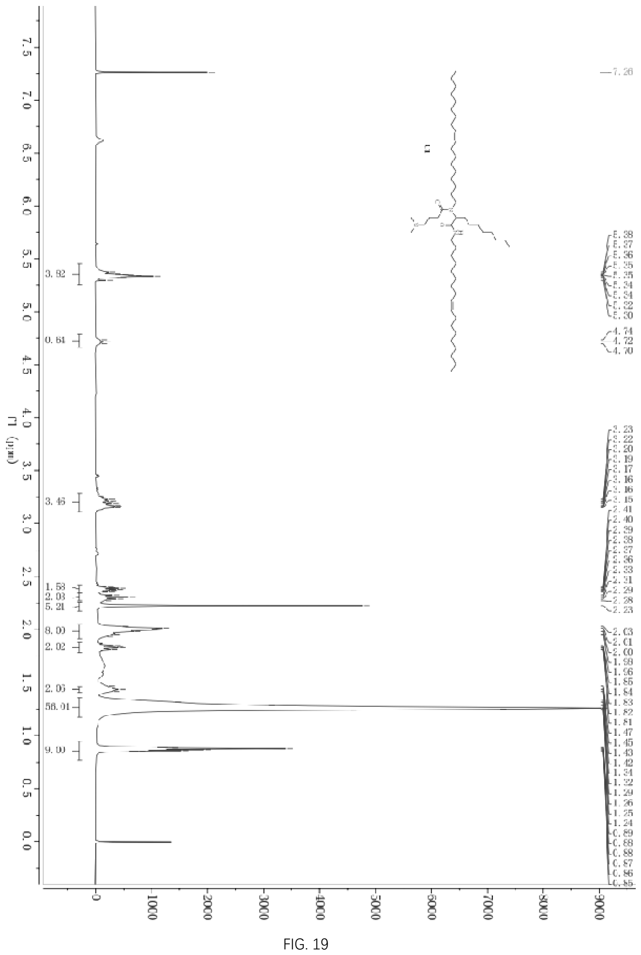

Figure 19: ¹H NMR spectrum of compound C1 (400 MHz, CDCl₃).

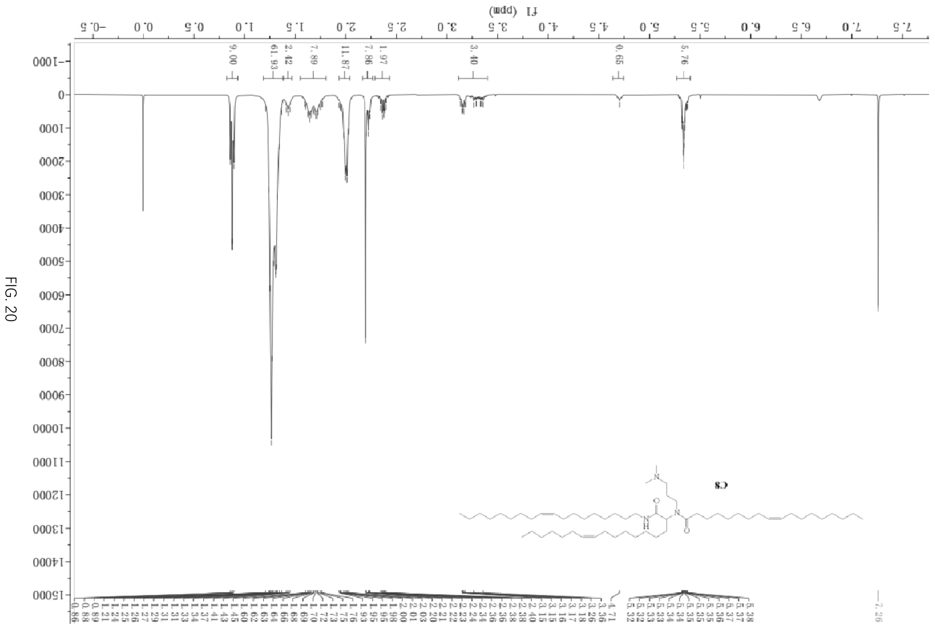

Figure 20: ¹H NMR spectrum of compound C8 (400 MHz, CDCl₃).

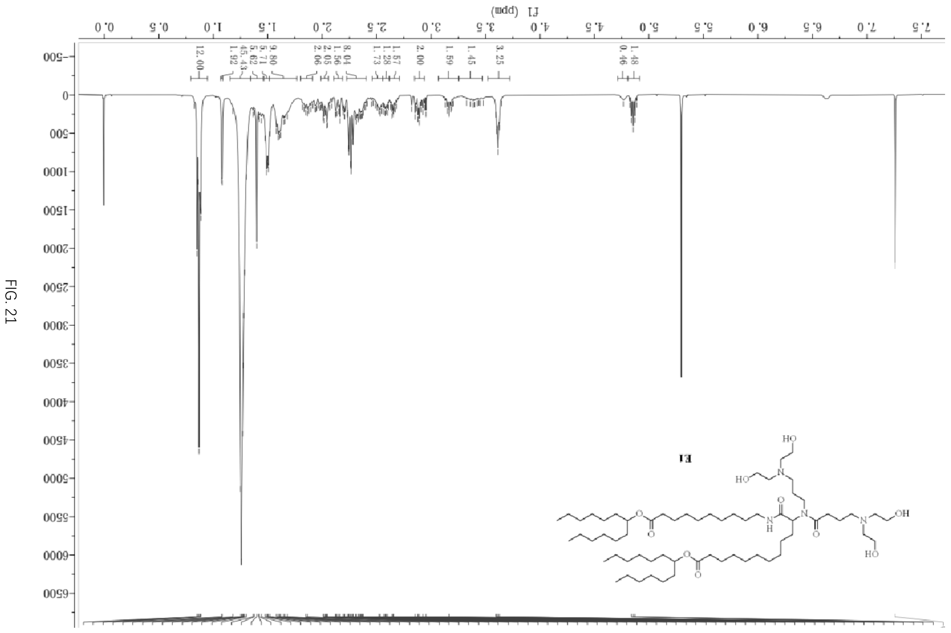

Figure 21: ¹H NMR spectrum of compound E1 (400 MHz, CDCl₃).
Figure 22: DLS particle size distribution of mRNA-loaded LNPs.
Figure 23: TEM characterization of mRNA-loaded LNPs.
Figure 24: Fluorescence expression data from in vitro administration of Luciferase mRNA-loaded LNPs.
Figure 25: Cell viability assay data from in vitro administration of Luciferase mRNA-loaded LNPs.
Figure 26: Fluorescence imaging results 6 hours post intramuscular injection of Luciferase mRNA-loaded LNPs in vivo.
Figure 27: Fluorescence imaging results 6 hours post tail vein injection of Luciferase mRNA-loaded LNPs in vivo, showing liver and spleen distribution.
Figure 28: Fluorescence imaging results 6 hours post intramuscular injection of LNPs prepared with compound A36 at different ratios.
Figure 29: Fluorescence imaging results 6 hours post intramuscular injection of LNPs prepared with compound B29 at different ratios.
Figure 30: Fluorescence imaging results 6 hours post intramuscular injection of LNPs prepared with compound C8 at different ratios.
Figure 31: Fluorescence imaging results 6 hours post intramuscular injection of LNPs prepared with compound D5 at different ratios.
Figure 32: Fluorescence imaging results 6 hours post intramuscular injection of LNPs prepared with compound E4 at different ratios.
Figure 33: Spleen distribution expression data from in vivo tail vein injection of Luciferase mRNA-loaded LNPs.
Figure 34: Fluorescence imaging results 6 hours post intramuscular injection of different doses of mRNA-loaded LNPs.
Figure 35: Serum hEPO protein content 24 hours post intramuscular injection of hEPO mRNA-loaded LNPs.
Figure 36: Binding antibody titer results from in vivo intramuscular injection of Covid19-S protein mRNA-loaded LNPs.
Figure 37: Neutralizing antibody titer results from in vivo intramuscular injection of Covid19-S protein mRNA-loaded LNPs.
Figure 38: Cell immune Elispot results from in vivo intramuscular injection of Covid19-S protein mRNA-loaded LNPs.
Figure 39: Body temperature changes post in vivo intramuscular injection of Covid19-S protein mRNA-loaded LNPs.
Figure 40: Body weight changes post in vivo intramuscular injection of Covid19-S protein mRNA-loaded LNPs.
Figure 41: Biochemical indicator changes in blood post in vivo intramuscular injection of Covid19-S protein mRNA-loaded LNPs.
Figure 42: Storage stability results of LNPs prepared with different compounds.
Figure 43: Knockdown results of overexpressed F-Luc cell lines using siRNA-LNPs prepared with different compounds.

### DETAILED DESCRIPTION OF THE PREFERRED EMBODIMENTS

### 1 General Techniques

The technologies and procedures described or referenced in this article are commonly understood and/or typically employed by those skilled in the field using conventional methods, such as those widely used methods described in Sambrook et al., Molecular Cloning: A Laboratory Manual (3rd Edition, 2001), and Current Protocols in Molecular Biology (edited by Ausubel et al., 2003).

### 2 Terminology

Unless otherwise specified, all technical and scientific terms used in this article have the same meanings as those commonly understood by those skilled in the field. For the purpose of interpreting this specification, the following terms will be defined as described, and terms used in the singular form will encompass the plural form and vice versa. All patents, applications, published applications, and other publications are incorporated herein by reference in their entirety. In the event of any conflict between the description of any term herein and that in any incorporated document, the description of the term herein shall prevail.

### 3 Compound Structure and Types

**Table 1: Structural Formulas of Compounds**

| Compound Type A | |
|---|---|
| 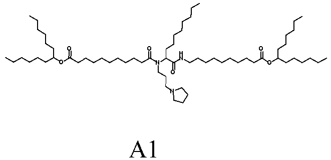 | 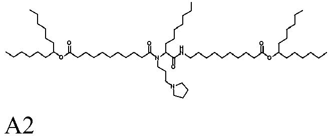 |
| 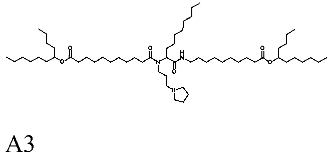 | 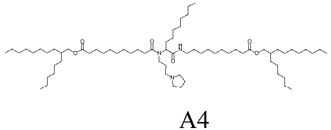 |
| 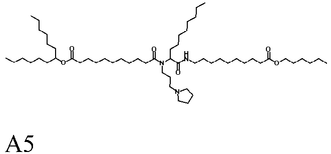 | 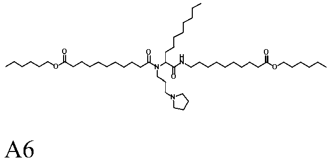 |
| 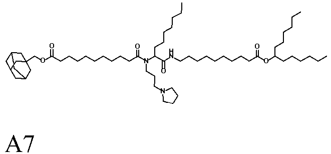 | 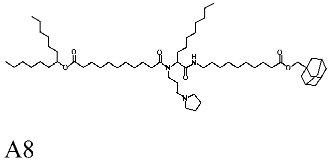 |
| 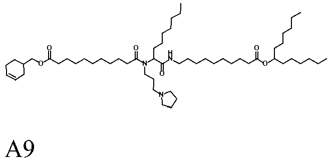 | 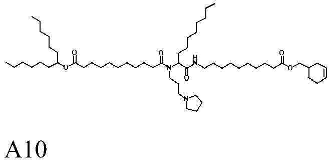 |
| 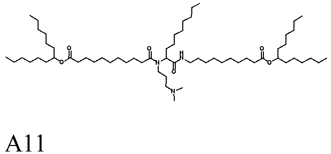 | 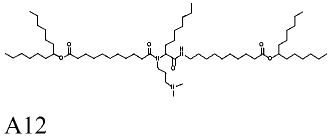 |
| 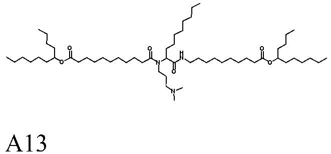 | 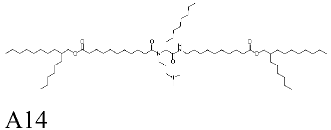 |
| 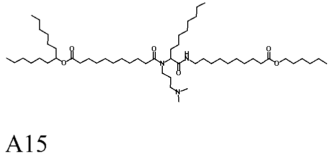 | 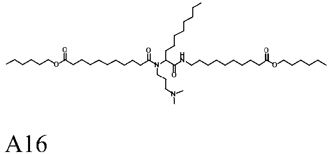 |
| 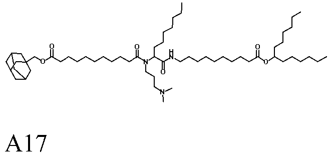 | 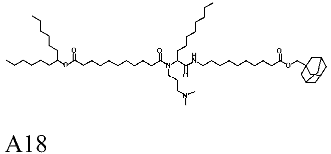 |
| 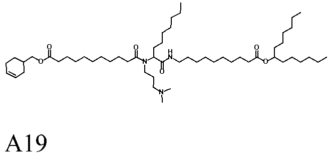 | 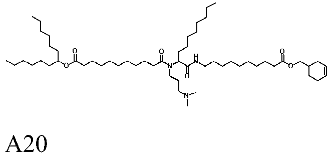 |
| 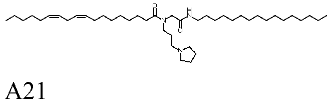 | 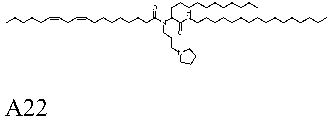 |
| 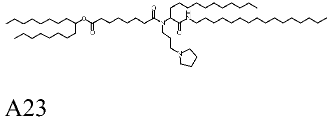 | 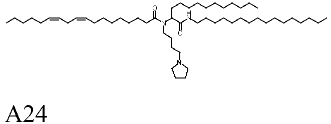 |
| 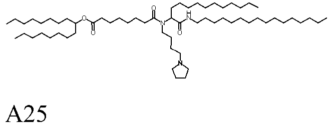 | 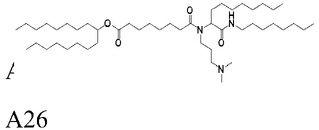 |
| 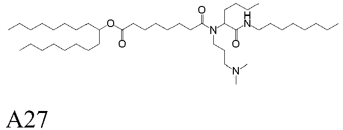 | 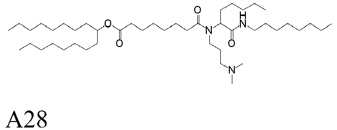 |
| 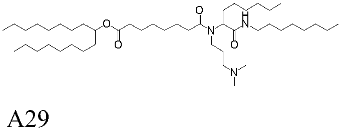 | 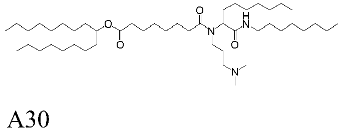 |
| 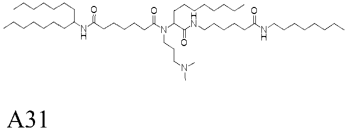 | 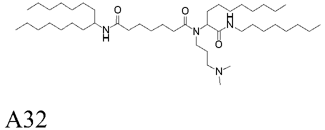 |
| 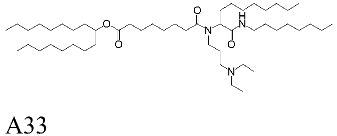 | 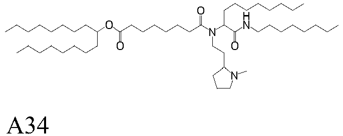 |
| 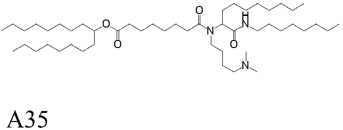 | 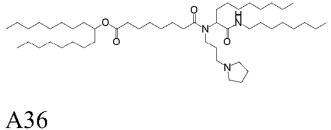 |
| 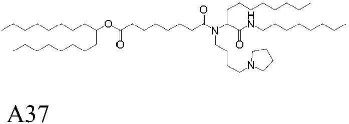 | 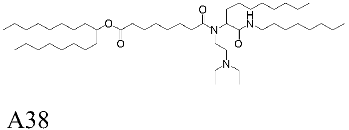 |
| 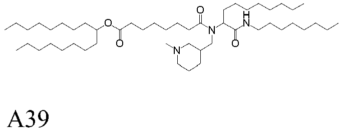 | 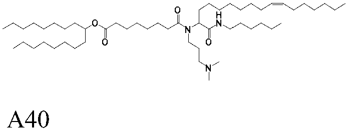 |
| 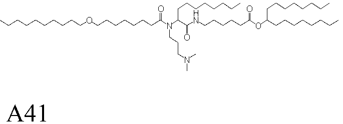 | 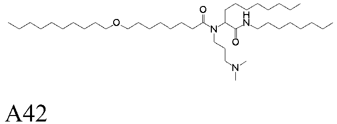 |
| 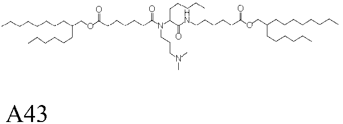 | 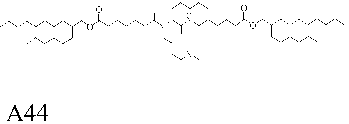 |
| 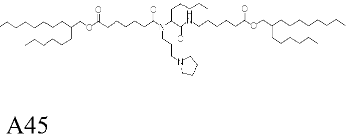 | 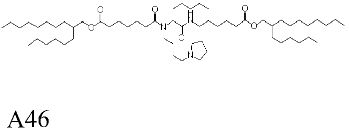 |
| 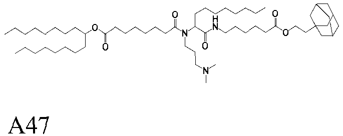 | 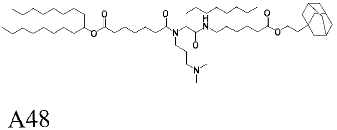 |

| Compound Type B | |
|---|---|
| 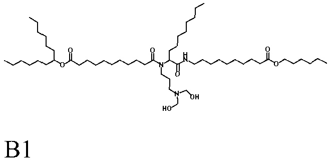 | 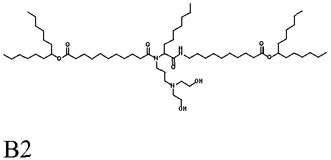 |
| 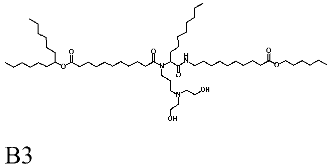 | 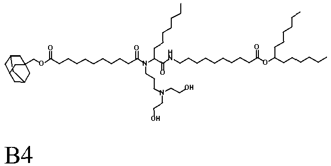 |
| 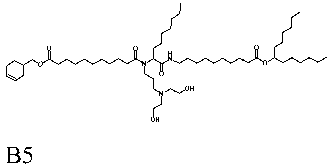 | 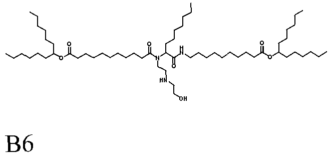 |
| 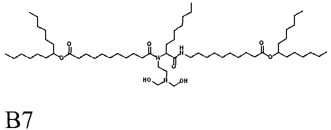 | 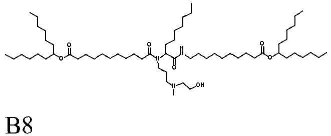 |
| 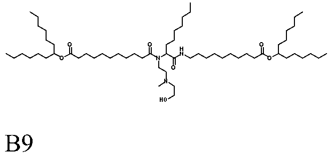 | 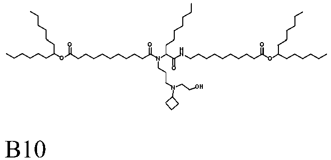 |
| 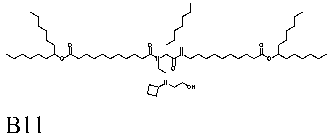 | 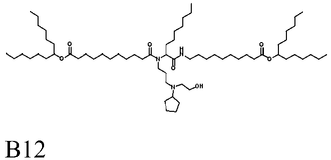 |
| 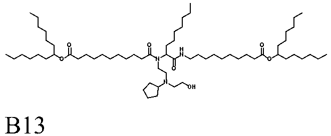 | 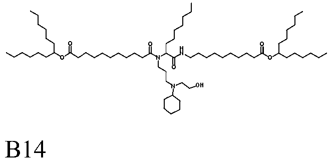 |
| 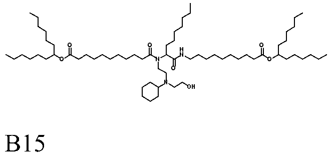 | 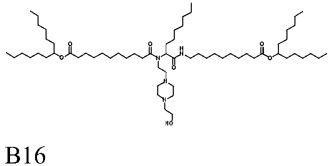 |
| 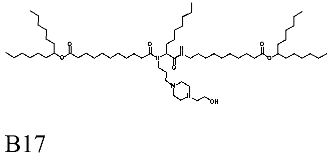 | 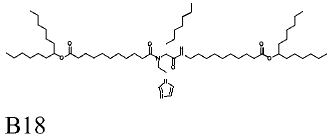 |
| 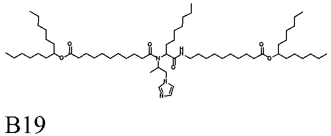 | 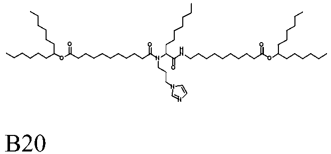 |
| 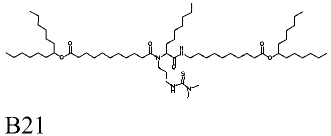 | 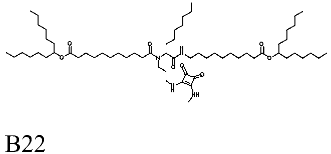 |
| 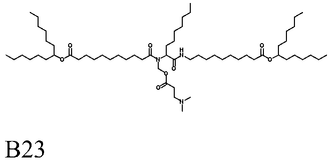 | 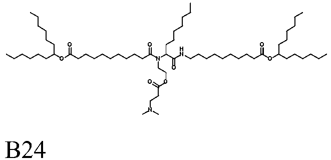 |
| 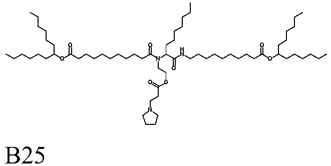 | 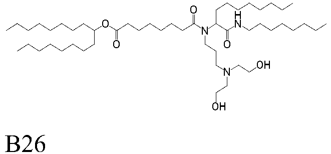 |
| 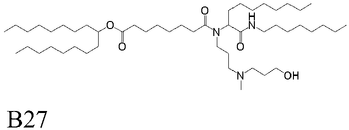 | 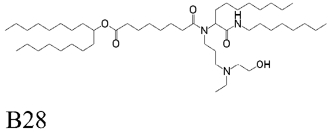 |
| 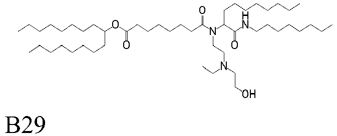 | 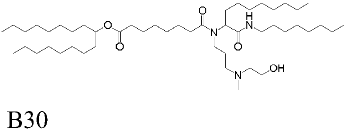 |
| 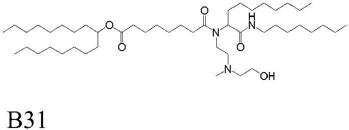 | 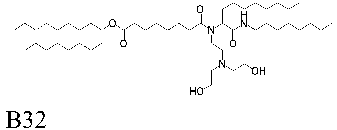 |
| 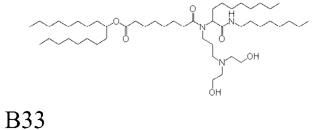 | 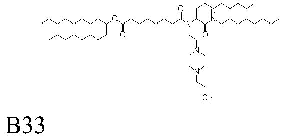 |
| 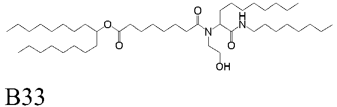 | 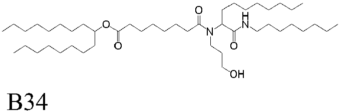 |
| 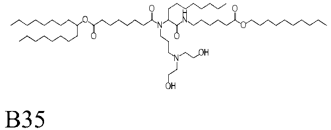 | 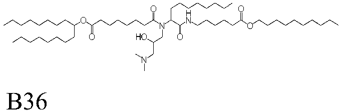 |
| 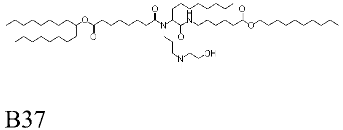 | 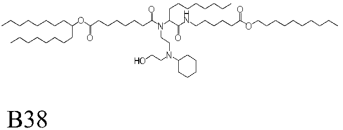 |
| 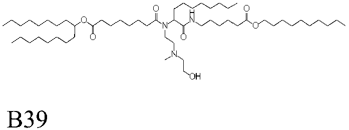 | 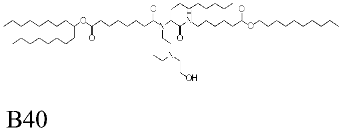 |
| 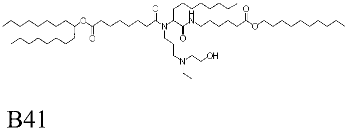 | 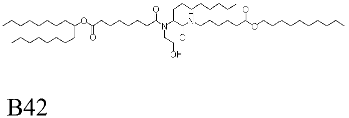 |
| 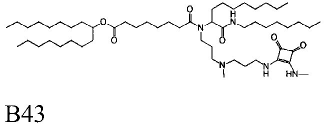 | |

| Compound Type C | |
|---|---|
| 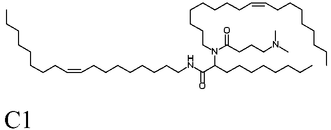 | 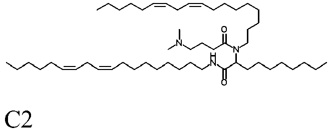 |
| 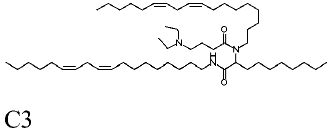 | 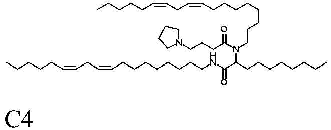 |
| 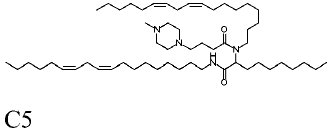 | 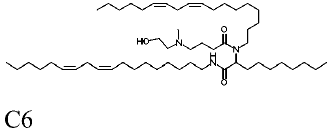 |
| 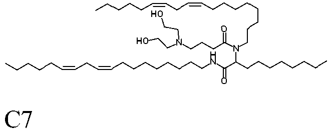 | 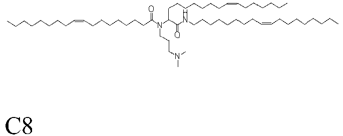 |

| Compound Type D | |
|---|---|
| 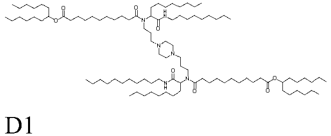 | 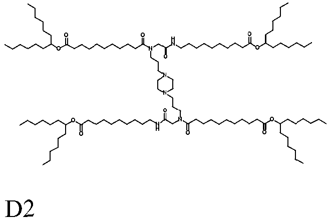 |
| 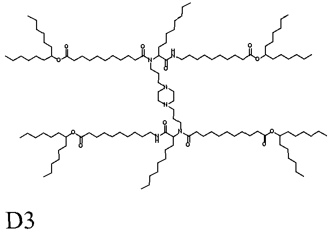 | 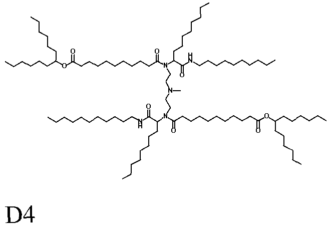 |
| 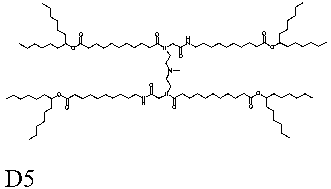 | 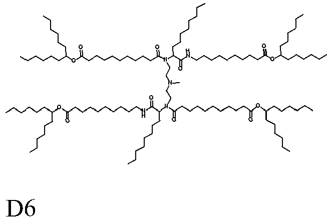 |

| Compound Type E | |
|---|---|
| 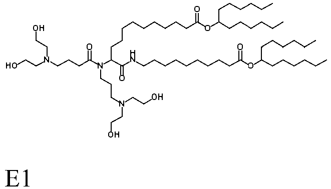 | 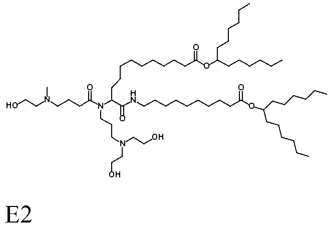 |
| 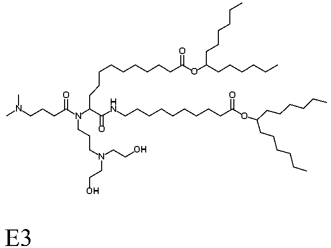 | 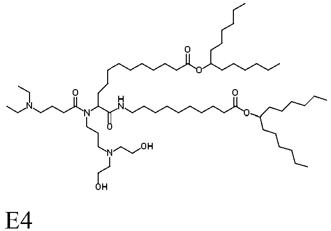 |
| 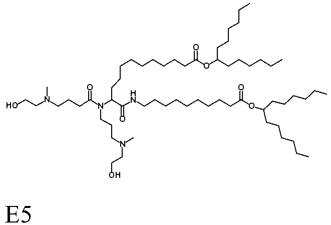 | 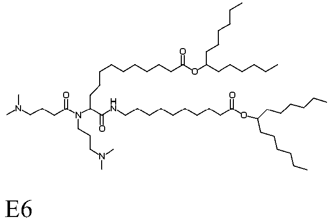 |
| 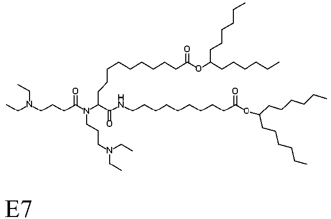 | 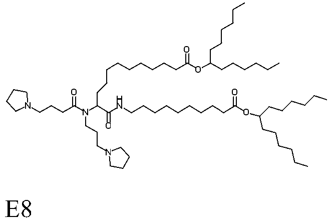 |
| | |
| 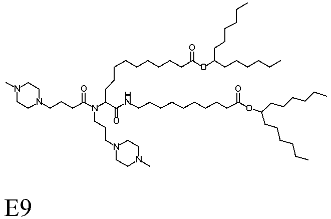 | 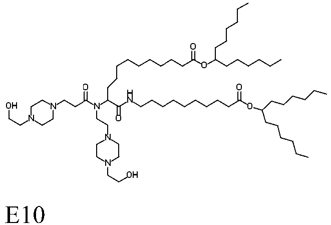 |
| 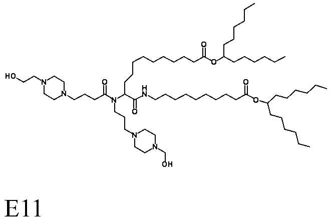 | |

It should be understood that any embodiment of the compounds provided herein, as shown above, and any specific substituent and/or variable of the compounds provided herein can be independently combined with other embodiments and/or substituents and/or variables of the compounds to form embodiments not specifically described above. In addition, where a list of substituents and/or variables for a particular group or variable is provided, it should be understood that each individual substituent and/or variable can be deleted from the list, and the remaining substituents and/or variables will be considered within the scope of the embodiments provided herein.

It should be understood that in this disclosure, the combination of substituents and/or variables depicted in various formulas is only permissible when such combinations result in stable compounds.

In one aspect, the compound has the structural formula (I-1): 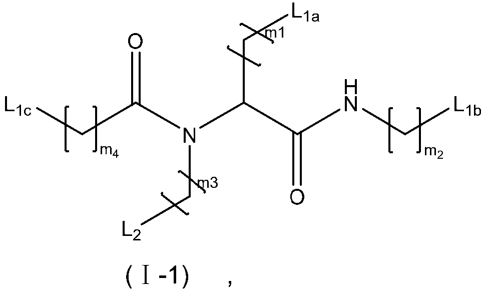 The compound provided herein may also be used as its pharmaceutically acceptable salts, prodrugs, or isomers.
In the compound:
m1, m2, m3, and m4 are each independently selected from integers between 1 and 18, inclusive.
L1a, L1b, and L1c are each independently selected from the group consisting of: -H, -S-SR1, -SR1, -C(=O)OR1, -OC(=O)R1, -N(R2)R1, -C(=O)N(R2)R1-, -N(R2)C(=O)R1-, -C(=O)NR1, and -OR1.
R1 and R2 are each independently selected from the group consisting of: -H, alicyclic hydrocarbons, 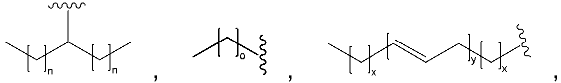 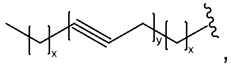 wherein n, x, and o are each independently selected from integers of 0 to 12, and y is independently selected from 1, 2, 3, or 4.
L2 is independently selected from the group consisting of: -R3XR4Y, -R3Y, -R3YR5, -Y, -R3XR4(Z)Y.
R3, R4, and R5 are each independently selected from the group consisting of: C1-C5 alkane and C2-C5 alkene.
X is independently selected from the group consisting of: -O-, -N(Rx)-, -S-, -S-S-, -OC(=O)-, -C(=O)O-, -OP(=O)O-, -OS(=O)O-, -C(=O)N-, -N(C(=O)) -. Rx is independently selected from the group consisting of: -H, -(CH2)m-OH, -(CH2)m-CN, -(CH2)m, and alicyclic hydrocarbons, wherein m is independently selected from 0, 1, 2, 3, 4, or 5.
Y is independently selected from the group consisting of: -(CH2)m-OH, -(CH2)m-CN, -(CH2)m-N(R**)R*, -(CH2)m-G-(CH2)n, wherein m and n are each independently selected from 0, 1, 2, 3, 4, or 5.
G is independently selected from aryl or heteroaryl.
R* and R** are each independently selected from the group consisting of: H, C1-C3 alkyl, -(CH2)mQ, wherein m is independently selected from 1, 2, 3, or 4. Q is independently selected from the group consisting of: -OH and -CN.
Z is independently selected from the group consisting of: -(CH2)m-OH, -(CH2)m-CN, -(CH2)m-N(R**)R*, -(CH2)m-G-(CH2)n, wherein m and n are each independently selected from 0, 1, 2, 3, 4, or 5.
G is independently selected from aryl or heteroaryl.
R* and R** are each independently selected from the group consisting of: H, C1-C3 alkyl, -(CH2)aQ, wherein a is independently selected from 1, 2, 3, or 4. Q is independently selected from the group consisting of: -OH and -CN.
R is independently selected from the group consisting of: -(CH2)m-, -N-, -S-, -C(=O)O-, -OC(=O)-, -N((CH2)m)-, -C(=O)N-, and -O-, wherein m is independently selected from 1, 2, 3, or 4.

Preferably, the positions of L1a, L1b, and L1c can be interchangeably or replaceably connected with L2, and/or the position of L2 can be interchangeably or replaceably connected with L1a, L1b, or L1c.

The number of L2 groups in the structural formula is at least one and no more than two.

In another aspect, the present invention also provides another compound which comprises one or more structural formula (I-2): 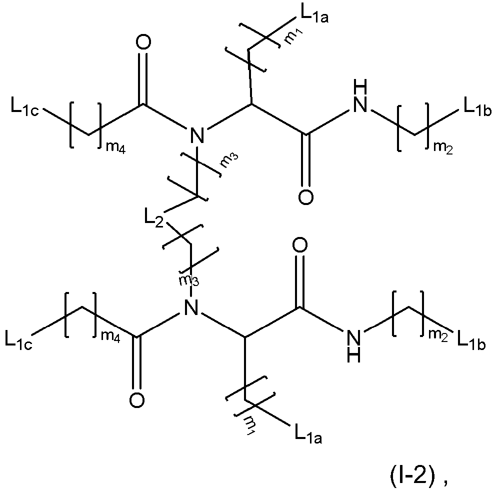 The compound provided herein may be used as its pharmaceutically acceptable salts, prodrugs, or isomers.
In the compound:
m1, m2, m3, and m4 are each independently selected from integers between 1 and 18, inclusive.
L1a, L1b, and L1c are each independently selected from the group consisting of: -H, -S-SR1, -SR1, -C(=O)OR1, -OC(=O)R1, -N(R2)R1, -C(=O)N(R2)R1-, -N(R2)C(=O)R1-, -C(=O)NR1, and -OR1.
R1 and R2 are each independently selected from the group consisting of: -H, alicyclic hydrocarbons, 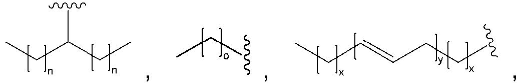 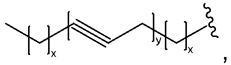 wherein n, x, and o are each independently selected from integers of 0 to 12, and y is independently selected from 1, 2, 3, or 4.

The advantage of formula (I-2) is its high nitrogen content, which allows for tighter encapsulation of therapeutic agents, potentially reducing the amount of lipid compound required.

As a preferred embodiment of the compounds described in this patent, the parent structure of the compound is prepared by the Ugi reaction (i.e., Ugi reaction) from amine, carboxylic acid, aldehyde, and isonitrile.

As a preferred embodiment of the compounds described in this patent, R1 is alicyclic, and R1 is a vitamin E succinate, adamantane, or its derivative;

R2 is alicyclic, and R2 is a vitamin E succinate, adamantane, or its derivative.

### 4 Nanoparticle Compositions

In one aspect, this disclosure describes nanoparticle compositions comprising the lipid compounds described herein. In specific embodiments, the nanoparticle compositions comprise compounds according to formulas (I-1) to (I-2) (and their sub-formulas) as described herein.

In some embodiments, when measured by methods such as Dynamic Light Scattering (DLS), transmission electron microscopy, scanning electron microscopy, or another method, the maximum size of the nanoparticle compositions provided herein is 1 µm or less (e.g., ≤1 µm, ≤900 nm, ≤ 800 nm, ≤700 nm, ≤600 nm, ≤500 nm, ≤400 nm, ≤300 nm, ≤200 nm, ≤175 nm, ≤150 nm, ≤125 nm, ≤100 nm, ≤75 nm, ≤50 nm, or less). In one embodiment, the lipid nanoparticles provided herein have at least one dimension in the range of approximately 40 nm to approximately 200 nm. In one embodiment, the at least one dimension is within the range of approximately 40 nm to approximately 100 nm.

Nanoparticle compositions that may be used in combination with the present disclosure include, for example, lipid nanoparticles (LNPs), nanolipoprotein particles, liposomes, lipid vesicles, and lipoplexes. In some embodiments, the nanoparticle composition is a vesicle comprising one or more lipid bilayers. In some embodiments, the nanoparticle composition comprises two or more concentric bilayers separated by an aqueous compartment. The lipid bilayers may be functionalized and/or crosslinked with each other. The lipid bilayer may comprise one or more ligands, proteins, or channels.

The characteristics of the nanoparticle composition may depend on its components. For example, a nanoparticle composition comprising cholesterol as a structural lipid may have different characteristics compared to a nanoparticle composition comprising a different structural lipid. Similarly, the characteristics of the nanoparticle composition may depend on the absolute or relative amounts of its components. For example, a nanoparticle composition comprising a higher molar percentage of phospholipid may have different characteristics compared to a nanoparticle composition comprising a lower molar percentage of phospholipid. The characteristics may also vary depending on the preparation method and conditions of the nanoparticle composition.

Nanoparticle compositions can be characterized by various methods. For example, the morphology and size distribution of nanoparticle compositions can be examined using microscopy (e.g., transmission electron microscopy or scanning electron microscopy). The zeta potential can be measured using dynamic light scattering or potentiometric titration. Dynamic light scattering can also be used to determine particle size. Instruments such as the Zetasizer Nano ZS (Malvern Instruments Ltd., Malvern, Worcestershire, UK) can also be used to measure multiple characteristics of nanoparticle compositions, such as particle size, polydispersity index, and zeta potential.

Dh (Size): The average size of the nanoparticle composition can range from tens of nanometers to hundreds of nanometers. For example, the average size may be about 40 nm to about 150 nm, such as about 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, or 150 nm. In some embodiments, the average size of the nanoparticle composition may be about 50 nm to about 100 nm, about 50 nm to about 90 nm, about 50 nm to about 80 nm, about 50 nm to about 70 nm, about 50 nm to about 60 nm, about 60 nm to about 100 nm, about 60 nm to about 90 nm, about 60 nm to about 80 nm, about 60 nm to about 70 nm, about 70 nm to about 100 nm, about 70 nm to about 90 nm, about 70 nm to about 80 nm, about 80 nm to about 100 nm, about 80 nm to about 90 nm, or about 90 nm to about 100 nm. In certain embodiments, the average size of the nanoparticle composition may be about 70 nm to about 100 nm. In some embodiments, the average size may be about 80 nm. In other embodiments, the average size may be about 100 nm.

PDI: The nanoparticle composition may be relatively homogeneous. The homogeneity of the nanoparticle composition, such as its particle size distribution, can be indicated by the polydispersity index. A smaller polydispersity index (e.g., less than 0.3) generally indicates a narrower particle size distribution. The polydispersity index of the nanoparticle composition may be about 0 to about 0.25, such as 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20, 0.21, 0.22, 0.23, 0.24, or 0.25. In some embodiments, the polydispersity index of the nanoparticle composition may be about 0.10 to about 0.20.

Encapsulation Efficiency: The encapsulation efficiency of a therapeutic and/or prophylactic agent refers to the amount of the therapeutic and/or prophylactic agent encapsulated within or otherwise associated with the nanoparticle composition relative to the initial amount provided during preparation. Encapsulation efficiency is desirably high (such as close to 100%). Encapsulation efficiency can be measured, for example, by comparing the amount of therapeutic and/or prophylactic agent in a solution containing the nanoparticle composition before and after disruption of the nanoparticle composition using one or more organic solvents or detergents. Fluorescence can be used to measure the amount of free therapeutic and/or prophylactic agent (e.g., RNA) in solution. For the nanoparticle compositions described herein, the encapsulation efficiency of the therapeutic and/or prophylactic agent may be at least 50%, such as 50%, 55%, 60%, 65%, 70%, 75%, 80%, 85%, 90%, 91%, 92%, 93%, 94%, 95%, 96%, 97%, 98%, 99%, or 100%. In some embodiments, the encapsulation efficiency may be at least 80%. In certain embodiments, the encapsulation efficiency may be at least 90%.

Apparent pKa: The zeta potential of the nanoparticle composition can indicate the electrophoretic potential of the composition. For example, zeta potential may describe the surface charge of the nanoparticle composition. Nanoparticle compositions with relatively low positive or negative charges are generally desirable, as materials with higher charges may interact undesirably with cells, tissues, and other components in the body. In some embodiments, the zeta potential of the nanoparticle composition may be about -10 mV to about +20 mV, about -10 mV to about +15 mV, about -10 mV to about +10 mV, about -10 mV to about +5 mV, about -10 mV to about 0 mV, about -10 mV to about -5 mV, about -5 mV to about +20 mV, about -5 mV to about +15 mV, about -5 mV to about +10 mV, about -5 mV to about +5 mV, about -5 mV to about 0 mV, about 0 mV to about +20 mV, about 0 mV to about +15 mV, about 0 mV to about +10 mV, about 0 mV to about +5 mV, about +5 mV to about +20 mV, about +5 mV to about +15 mV, or about +5 mV to about +10 mV.

In another embodiment, self-replicating RNA can be formulated in liposomes. As a non-limiting example, self-replicating RNA can be formulated in liposomes as described in International Publication No. WO20120067378, which is incorporated herein by reference in its entirety. In one aspect, the liposome may comprise a lipid with a pKa value favorable for mRNA delivery. In another aspect, the liposome may have a substantially neutral surface charge at physiological pH and thus can be effective for immunization (see, for example, the liposomes described in International Publication No. WO20120067378, which is incorporated herein by reference in its entirety).

In some embodiments, the nanoparticle composition comprises a lipid component, which includes at least one lipid, such as a compound according to one of formulas (I) to (IV) (and their sub-formulas) as described herein. For example, in some embodiments, the nanoparticle composition may comprise a lipid component that includes one of the compounds provided herein. The nanoparticle composition may also comprise one or more additional lipids or non-lipid components as described below.

In some embodiments, the nanoparticle composition comprises a lipid component, which includes at least one lipid, such as a compound according to one of formulas (I) to (IV) (and their sub-formulas) as described herein. For example, in some embodiments, the nanoparticle composition may comprise a lipid component that includes one of the compounds provided herein. The nanoparticle composition may also comprise one or more additional lipids or non-lipid components as described below.

### 5 Formulations

In accordance with this disclosure, the nanoparticle compositions described herein may comprise at least one lipid component and one or more additional components, such as therapeutic and/or prophylactic agents. The nanoparticle compositions may be designed for use in one or more specific applications or targets. The components of the nanoparticle composition may be selected based on specific applications or targets, and/or based on the efficacy, toxicity, cost, ease of use, availability, or other characteristics of one or more components. Similarly, the specific formulation of the nanoparticle composition may be selected for specific applications or targets based on, for example, the efficacy and toxicity of specific combinations of each component.

In some embodiments, the lipid component of the nanoparticle composition may comprise a lipid as described herein according to one of formulas (I-1) to (I-2) (and their sub-formulas), a phospholipid (e.g., an unsaturated lipid, such as DOPE or DSPC), a PEG-lipid, and/or a structural lipid. The components of the lipid component may be provided in specific ratios.

In one embodiment, the present disclosure provides a nanoparticle composition comprising a cationic or ionizable lipid compound provided herein, a therapeutic agent, and one or more excipients. In one embodiment, the cationic or ionizable lipid compound comprises a compound according to one of formulas (I-1) to (I-2) (and their sub-formulas) as described herein, and optionally one or more additional ionizable lipid compounds. In one embodiment, the one or more excipients are selected from neutral lipids, sterols, and polymer-bound lipids. In one embodiment, the therapeutic agent is encapsulated within or associated with the lipid nanoparticles.

In one embodiment, the present disclosure provides a lipid nanoparticle composition, which comprises:
i) 40 to 50 mole percent of a cationic lipid;
ii) a neutral lipid;
iii) a sterol;
iv) a polymer-bound lipid;
v) a therapeutic agent.

### 6 Examples

The examples provided in this section are illustrative only and not limiting.

### General Methods

Preparative HPLC: Purification was performed on a SepaBean^{®} machine U100-Test equipped with an evaporative light scattering detector (ELSD), using SepaFlash^{®} rapid separation columns. Solvents included dichloromethane (A), methanol (B), methanol with 7M ammonia (C), petroleum ether (D), and ethyl acetate (E).

LCMS Analysis: Conducted on a Thermo (LC-MS) system. Chromatography was performed on a SunFire C18 column, using water with 0.1% trifluoroacetic acid (A) and acetonitrile with 0.1% formic acid (B) as mobile phases.

### 6.1 Example 1

### Preparation of Class A Compounds

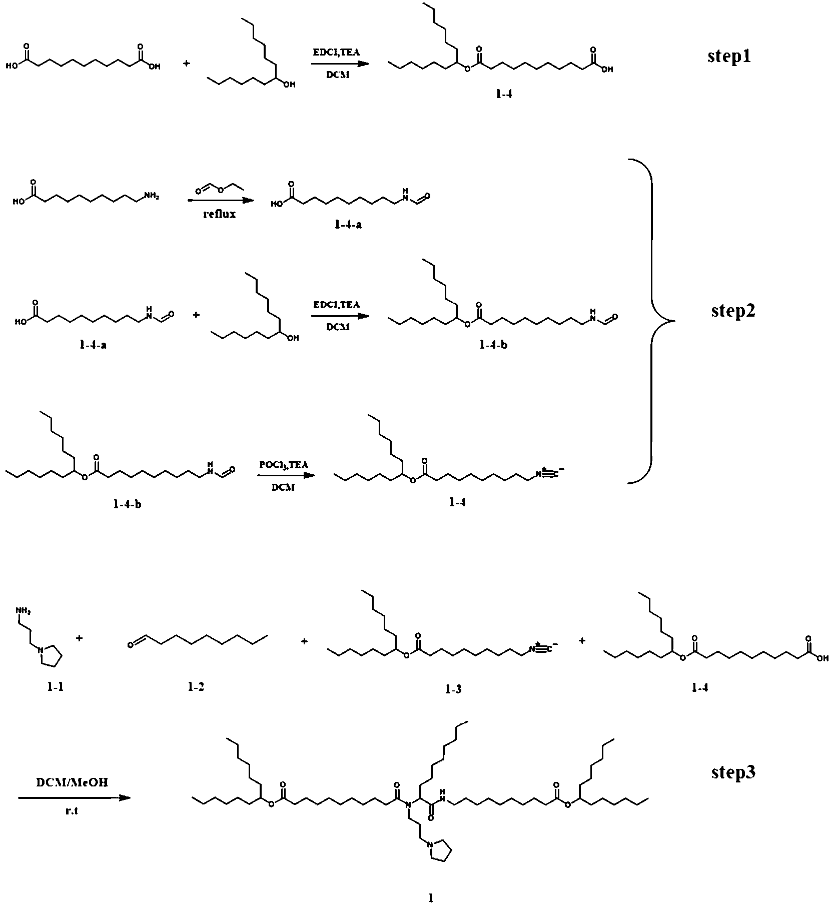

### Preparation of Compound A1

### Step 1: Preparation of Intermediate A1-3

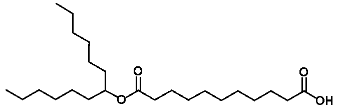

To a solution of 7-tridecanol (1 g, 5 mmol), undecanedioic acid (1.5 g, 7 mmol), and triethylamine (0.2 g, 1.6 mmol) in dichloromethane, EDCI (1.5 g, 7.7 mmol) was added and stirred overnight at room temperature. TLC indicated the complete consumption of 7-tridecanol. The crude product was purified by preparative chromatography under a gradient program of solvent D:solvent E = 100:0 to 80:20, yielding a colorless oily compound (500 mg, 25% yield).

### Step 2: Preparation of Intermediate A1-4

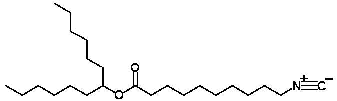

### Preparation of Compound A1-4-a

In a 100 mL round-bottom flask, (10-amino capric acid) (1.87 g, 100 mmol) and ethyl formate (18 mL) were added and refluxed overnight. TLC indicated the completion of the reaction. The mixture was evaporated under reduced pressure, and the crude product was purified by preparative chromatography under a gradient program of solvent D:solvent E = 100:0 to 80:20, yielding a colorless oil (1.75 g, 81.3% yield).

### Preparation of Compound A1-4-b

At 0°C, to a solution of 1-4-a (1 g, 4.6 mmol), 7-tridecanol (1.11 g, 5.52 mmol), and triethylamine (0.47 g, 4.6 mmol) in dichloromethane, EDCI was added and stirred overnight at room temperature. TLC indicated the completion of the reaction. The reaction mixture was adjusted to pH 8 with 1N HCI, extracted with dichloromethane (3×50 mL), and the organic phase was washed with saturated brine, dried over anhydrous sodium sulfate, and evaporated under reduced pressure. The crude product was purified by preparative chromatography under a gradient program of solvent D:solvent E = 100:0 to 80:20, yielding a colorless oil (1.59 g, 86.8% yield).

### Preparation of Compound A1-4

To a round-bottom flask containing 1-4-b (1 g, 2.5 mmol) and triethylamine (0.758 g, 7.5 mmol), dissolved in DCM, the solution was cooled to 0°C. Under nitrogen protection, POCI3 (0.421 g, 2.75 mmol) was slowly added dropwise. After completion of addition, the reaction was allowed to proceed at room temperature for 4 hours. TLC indicated the completion of the reaction. The reaction was quenched with ice water, extracted with ethyl acetate (2×20 mL), and the organic phase was washed three times with brine, dried over anhydrous sodium sulfate, and evaporated under reduced pressure. The crude product was purified by preparative chromatography under a gradient program of solvent D:solvent E = 100:0 to 80:20, yielding a colorless oil (0.8 g, 84.2% yield).

### Step 3: Preparation of Compound A1

At room temperature, A1-1 (1-(3-aminopropyl)pyrrolidine) (170 mg, 1.33 mmol) was dissolved in dichloromethane/methanol (VDCM:VMeOH = 3:1). A1-2 (nonanal) (190 mg, 1.33 mmol) was added, and after stirring for 1 hour, compound 1-3 (500 mg, 1.33 mmol) was added. After stirring for an additional 0.5 hours, compound 1-4 (530 mg, 1.33 mmol) was added. The mixture was stirred at room temperature for 16 hours. TLC indicated the completion of the reaction. The mixture was evaporated under reduced pressure and purified by preparative HPLC under a gradient program of solvent A:solvent B:solvent C = 100:0:0 to 70:24:6, yielding a colorless oily compound 1 (100.0 mg, 30% yield). MS(ESI): [M+H]+ = 1030.70.

This preparation procedure serves as the standard compound preparation procedure A.

### Preparation of Compound A3

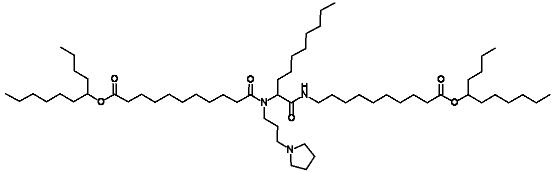

### Step 1: Preparation of Intermediate A3-3

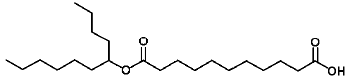

To a solution of 5-undecanol (1 g, 6 mmol), undecanedioic acid (1.5 g, 7 mmol), and DMAP (0.15 g, 1.1 mmol) in dichloromethane, EDCI (1.7 g, 9 mmol) was added and stirred overnight at room temperature. TLC indicated the complete consumption of 5-undecanol. The crude product was purified by preparative chromatography under a gradient program of solvent D:solvent E = 100:0 to 80:20, yielding a colorless oily compound (635 mg, 30% yield).

### Step 2: Preparation of Intermediate A3-4

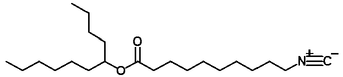

### Preparation of Compound A3-4-b

At 0°C, to a solution of A1-4-a (1 g, 4.6 mmol), 5-undecanol (1.05 g, 6.1 mmol), and triethylamine (0.47 g, 4.6 mmol) in dichloromethane, EDCI was added and stirred overnight at room temperature. TLC indicated the completion of the reaction. The reaction mixture was adjusted to pH 8 with 1N HCI, extracted with dichloromethane (3×50 mL), and the organic phase was washed with saturated brine, dried over anhydrous sodium sulfate, and evaporated under reduced pressure. The crude product was purified by preparative chromatography under a gradient program of solvent D:solvent E = 100:0 to 80:20, yielding a colorless oil (1.13 g, 67.0% yield).

### Preparation of Compound A3-4

To a round-bottom flask containing A3-4-b (0.55 g, 1.5 mmol) and triethylamine (0.45 g, 4.5 mmol), dissolved in DCM, the solution was cooled to 0°C. Under nitrogen protection, POCI3 (0.421 g, 2.75 mmol) was slowly added dropwise. After completion of addition, the reaction was allowed to proceed at room temperature for 4 hours. TLC indicated the completion of the reaction. The reaction was quenched with ice water, extracted with ethyl acetate (2×10 mL), and the organic phase was washed three times with brine, dried over anhydrous sodium sulfate, and evaporated under reduced pressure. The crude product was purified by preparative chromatography under a gradient program of solvent D:solvent E = 100:0 to 80:20, yielding a colorless oil (0.40 g, 77.0% yield).

### Step 3: Preparation of Compound A3

The compound was synthesized according to preparation procedure A, with intermediate A3-1 being the same as A1-1 and intermediate A3-2 being the same as 1-2. The crude product was purified by preparative HPLC under a gradient program of solvent A:solvent B:solvent C = 100:0:0 to 70:24:6, yielding a colorless oily compound A3 (130 mg, 25% yield). MS(ESI): [M+H]+ = 970.60.

### Preparation of Compound A4

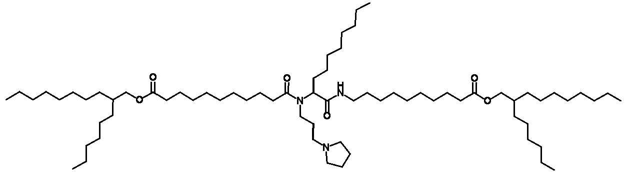

### Step 1: Preparation of Intermediate A4-3

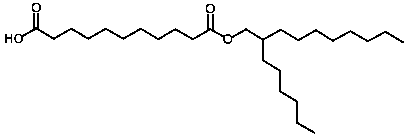

To a solution of 2-hexyl-1-decanol (1 g, 4 mmol), undecanedioic acid (1.07 g, 5 mmol), and DMAP (0.1 g, 0.8 mmol) in dichloromethane, EDCI (1.2 g, 6 mmol) was added and stirred overnight at room temperature. TLC indicated the complete consumption of 2-hexyl-1-decanol. The crude product was purified by preparative chromatography under a gradient program of solvent D:solvent E = 100:0 to 80:20, yielding a colorless oily compound (525 mg, 34% yield).

### Step 2: Preparation of Intermediate A4-4

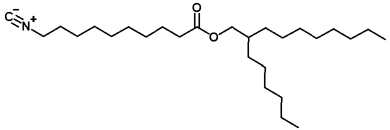

### Preparation of Compound A4-4-b

At 0°C, to a solution of A1-4-a (0.5 g, 2.3 mmol), 7-pentadecanol (0.72 g, 3.22 mmol), and triethylamine (0.24 g, 2.3 mmol) in dichloromethane, EDCI was added and stirred overnight at room temperature. TLC indicated the completion of the reaction. The reaction mixture was adjusted to pH 8 with 1N HCI, extracted with dichloromethane (3×50 mL), and the organic phase was washed with saturated brine, dried over anhydrous sodium sulfate, and evaporated under reduced pressure. The crude product was purified by preparative chromatography under a gradient program of solvent D:solvent E = 100:0 to 80:20, yielding a colorless oil (0.71 g, 72.5% yield).

### Preparation of Compound A4-4

To a round-bottom flask containing A4-4-b (0.42 g, 1 mmol) and triethylamine (0.3 g, 3 mmol), dissolved in DCM, the solution was cooled to 0°C. Under nitrogen protection, POCI3 (0.167 g, 1.09 mmol) was slowly added dropwise. After completion of addition, the reaction was allowed to proceed at room temperature for 4 hours. TLC indicated the completion of the reaction. The reaction was quenched with ice water, extracted with ethyl acetate (2×10 mL), and the organic phase was washed three times with brine, dried over anhydrous sodium sulfate, and evaporated under reduced pressure. The crude product was purified by preparative chromatography under a gradient program of solvent D:solvent E = 100:0 to 80:20, yielding a colorless oil (0.31 g, 76.0% yield).

### Step 3: Preparation of Compound A4

The compound was synthesized according to preparation procedure A, with intermediate A4-1 being the same as A1-1 and intermediate A4-2 being the same as A1-2. The crude product was purified by preparative HPLC under a gradient program of solvent A:solvent B:solvent C = 100:0:0 to 70:24:6, yielding a colorless oily compound A4 (156 mg, 35% yield). MS(ESI): [M+H]+ = 1114.87.

### Preparation of Compound A5

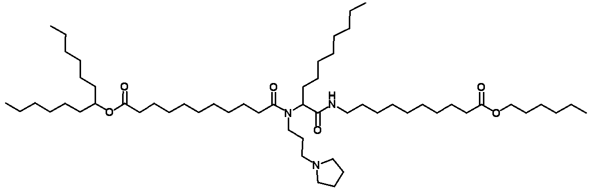

### Step 1: Preparation of Intermediate A5-3 (Same as Intermediate A1-3)

### Step 2: Preparation of Intermediate A5-4

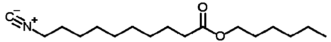

### Preparation of Compound A5-4-b

At 0°C, to a solution of A1-4-a (2.15 g, 10 mmol), n-hexanol (1.27 g, 12.5 mmol), and triethylamine (3.1 g, 30 mmol) in dichloromethane, EDCI was added and stirred overnight at room temperature. TLC indicated the completion of the reaction. The reaction mixture was adjusted to pH 8 with 1N HCI, extracted with dichloromethane (3×50 mL), and the organic phase was washed with saturated brine, dried over anhydrous sodium sulfate, and evaporated under reduced pressure. The crude product was purified by preparative chromatography under a gradient program of solvent D:solvent E = 100:0 to 80:20, yielding a colorless oil (2.66 g, 89.0% yield).

### Preparation of Compound A5-4

To a round-bottom flask containing A5-4-b (1 g, 3.3 mmol) and triethylamine (1 g, 9.9 mmol), dissolved in DCM, the solution was cooled to 0°C. Under nitrogen protection, POCI3 (0.551 g, 3.6 mmol) was slowly added dropwise. After completion of addition, the reaction was allowed to proceed at room temperature for 4 hours. TLC indicated the completion of the reaction. The reaction was quenched with ice water, extracted with ethyl acetate (2×20 mL), and the organic phase was washed three times with brine, dried over anhydrous sodium sulfate, and evaporated under reduced pressure. The crude product was purified by preparative chromatography under a gradient program of solvent D:solvent E = 100:0 to 80:20, yielding a colorless oil (0.80 g, 86.9% yield).

### Step 3: Preparation of Compound A5

The compound was synthesized according to preparation procedure A, with intermediate A5-1 being the same as A1-1 and intermediate A5-2 being the same as A1-2. The crude product was purified by preparative HPLC under a gradient program of solvent A:solvent B:solvent C = 100:0:0 to 70:24:6, yielding a colorless oily compound A5 (88 mg, 24% yield). MS(ESI): [M+H]+ = 932.51.

### Preparation of Compound A6

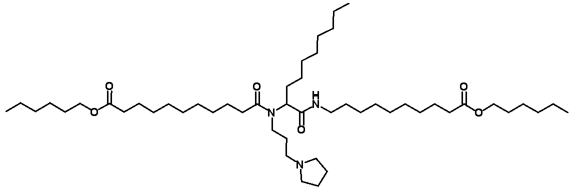

### Step 1: Preparation of Intermediate A6-3

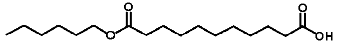

To a solution of n-hexanol (1 g, 10 mmol), undecanedioic acid (2.54 g, 12 mmol), and DMAP (0.24 g, 2 mmol) in dichloromethane, EDCI (2.82 g, 14.7 mmol) was added and stirred overnight at room temperature. TLC indicated the complete consumption of n-hexanol. The crude product was purified by preparative chromatography under a gradient program of solvent D:solvent E = 100:0 to 80:20, yielding a colorless oily compound (1.3 g, 44% yield).

### Step 2: Preparation of Intermediate A6-4 (Same as Intermediate A5-4)

### Step 3: Preparation of Compound A6

The compound was synthesized according to preparation procedure A, with intermediate A6-1 being the same as A1-1 and intermediate A6-2 being the same as A1-2. The crude product was purified by preparative HPLC under a gradient program of solvent A:solvent B:solvent C = 100:0:0 to 70:24:6, yielding a colorless oily compound A6 (155 mg, 32% yield). MS(ESI): [M+H]+ = 834.33.

### Preparation of Compound A7

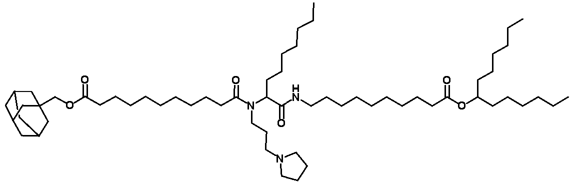

### Step 1: Preparation of Intermediate A7-3

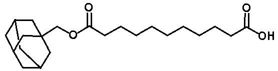

To a solution of adamantanemethanol (1 g, 6 mmol), undecanedioic acid (1.56 g, 7.2 mmol), and DMAP (0.15 g, 1.2 mmol) in tetrahydrofuran, EDCI (1.73 g, 9 mmol) was added and stirred overnight at 50°C. TLC indicated the complete consumption of adamantanemethanol. The crude product was purified by preparative chromatography under a gradient program of solvent D:solvent E = 100:0 to 80:20, yielding a colorless oily compound (1.2 g, 44% yield).

### Step 2: Preparation of Intermediate A7-4 (Same as Intermediate A1-4)

### Step 3: Preparation of Compound A7

The compound was synthesized according to preparation procedure A, with intermediate A7-1 being the same as A1-1 and intermediate A7-2 being the same as A2-2. The crude product was purified by preparative HPLC under a gradient program of solvent A:solvent B:solvent C = 100:0:0 to 70:24:6, yielding a colorless oily compound A7 (198 mg, 38% yield). MS(ESI): [M+H]+ = 982.57.

### Preparation of Compound A8

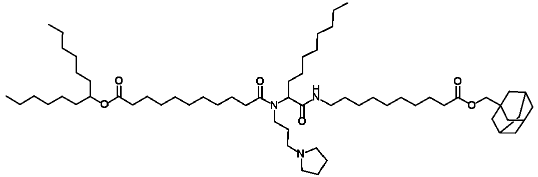

### Step 1: Preparation of Intermediate A8-3 (Same as Intermediate A1-3)

### Step 2: Preparation of Intermediate A8-4

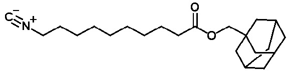

### Preparation of Compound A8-4-b

At 0°C, to a solution of A1-4-a (1 g, 4.6 mmol), 1-adamantanol (0.92 g, 5.52 mmol), and triethylamine (0.47 g, 4.6 mmol) in dichloromethane, EDCI was added and stirred overnight at room temperature. TLC indicated the completion of the reaction. The reaction mixture was adjusted to pH 8 with 1N HCI, extracted with dichloromethane (3×50 mL), and the organic phase was washed with saturated brine, dried over anhydrous sodium sulfate, and evaporated under reduced pressure. The crude product was purified by preparative chromatography under a gradient program of solvent D:solvent E = 100:0 to 80:20, yielding a colorless oil (0.89 g, 56.0% yield).

### Preparation of Compound A8-4

To a round-bottom flask containing A8-4-b (0.54 g, 1.5 mmol) and triethylamine (0.45 g, 4.5 mmol), dissolved in DCM, the solution was cooled to 0°C. Under nitrogen protection, POCI3 (0.421 g, 2.75 mmol) was slowly added dropwise. After completion of addition, the reaction was allowed to proceed at room temperature for 4 hours. TLC indicated the completion of the reaction. The reaction was quenched with ice water, extracted with ethyl acetate (2×10 mL), and the organic phase was washed three times with brine, dried over anhydrous sodium sulfate, and evaporated under reduced pressure. The crude product was purified by preparative chromatography under a gradient program of solvent D:solvent E = 100:0 to 80:20, yielding a colorless oil (0.37 g, 71.4% yield).

### Step 3: Preparation of Compound A8

The compound was synthesized according to preparation procedure A, with intermediate A8-1 being the same as A1-1 and intermediate A8-2 being the same as A1-2. The crude product was purified by preparative HPLC under a gradient program of solvent A:solvent B:solvent C = 100:0:0 to 70:24:6, yielding a colorless oily compound A8 (98 mg, 27% yield). MS(ESI): [M+H]+ = 996.60.

### Preparation of Compound A9

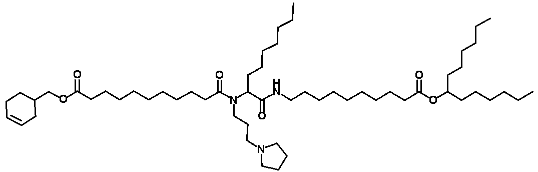

### Step 1: Preparation of Intermediate A9-3

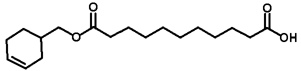

To a solution of 3-cyclohexene-1-methanol (1 g, 9 mmol), undecanedioic acid (2.31 g, 10.7 mmol), and DMAP (0.22 g, 1.8 mmol) in tetrahydrofuran, EDCI (2.56 g, 13.4 mmol) was added and stirred overnight at 50°C. TLC indicated the complete consumption of 3-cyclohexene-1-methanol. The crude product was purified by preparative chromatography under a gradient program of solvent D:solvent E = 100:0 to 80:20, yielding a colorless oily compound (1.15 g, 42% yield).

### Step 2: Preparation of Intermediate A9-4 (Same as Intermediate A1-4)

### Step 3: Preparation of Compound A9

The compound was synthesized according to preparation procedure A, with intermediate A9-1 being the same as A1-1 and intermediate A9-2 being the same as A1-2. The crude product was purified by preparative HPLC under a gradient program of solvent A:solvent B:solvent C = 100:0:0 to 70:24:6, yielding a colorless oily compound A9 (108.6 mg, 29% yield). MS(ESI): [M+H]+ = 928.48.

### Preparation of Compound A10

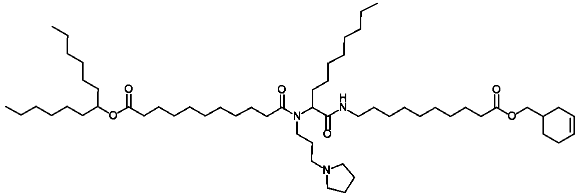

### Step 1: Preparation of Intermediate A10-3 (Same as Intermediate A1-3)

### Step 2: Preparation of Intermediate A10-4

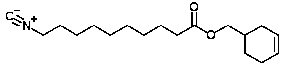

### Preparation of Compound A10-4-b

At 0°C, to a solution of A1-4-a (0.5 g, 2.3 mmol), cyclohex-3-ene-1-methanol (0.31 g, 2.76 mmol), and triethylamine (0.23 g, 2.3 mmol) in dichloromethane, EDCI was added and stirred overnight at room temperature. TLC indicated the completion of the reaction. The reaction mixture was adjusted to pH 8 with 1N HCI, extracted with dichloromethane (3×50 mL), and the organic phase was washed with saturated brine, dried over anhydrous sodium sulfate, and evaporated under reduced pressure. The crude product was purified by preparative chromatography under a gradient program of solvent D:solvent E = 100:0 to 80:20, yielding a colorless oil (0.56 g, 79.5% yield).

### Preparation of Compound A10-4

To a round-bottom flask containing A10-4-b (0.46 g, 1.5 mmol) and triethylamine (0.45 g, 4.5 mmol), dissolved in DCM, the solution was cooled to 0°C. Under nitrogen protection, POCI3 (0.421 g, 2.75 mmol) was slowly added dropwise. After completion of addition, the reaction was allowed to proceed at room temperature for 4 hours. TLC indicated the completion of the reaction. The reaction was quenched with ice water, extracted with ethyl acetate (2×10 mL), and the organic phase was washed three times with brine, dried over anhydrous sodium sulfate, and evaporated under reduced pressure. The crude product was purified by preparative chromatography under a gradient program of solvent D:solvent E = 100:0 to 80:20, yielding a colorless oil (0.38 g, 88.0% yield).

### Step 3: Preparation of Compound A10

The compound was synthesized according to preparation procedure A, with intermediate A10-1 being the same as A1-1 and intermediate A10-2 being the same as A1-2. The crude product was purified by preparative HPLC under a gradient program of solvent A:solvent B:solvent C = 100:0:0 to 70:24:6, yielding a colorless oily compound A10 (118.7 mg, 32% yield). MS(ESI): [M+H]+ = 942.51.

### Preparation of Compound A11

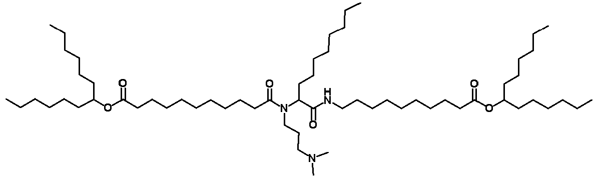

### Step 1: Preparation of Intermediate A11-3 (Same as Intermediate 1-3)

### Step 2: Preparation of Intermediate A11-4 (Same as Intermediate 1-4)

### Step 3: Preparation of Compound A11

The compound was synthesized according to preparation procedure A, with intermediate A11-1 being N,N-dimethyl-1,3-diaminopropane and intermediate A11-2 being the same as A1-2. The crude product was purified by preparative HPLC under a gradient program of solvent A:solvent B:solvent C = 100:0:0 to 70:24:6, yielding a colorless oily compound A11 (202 mg, 48% yield). MS(ESI): [M+H]+ = 1004.67.

### Preparation of Compound A12

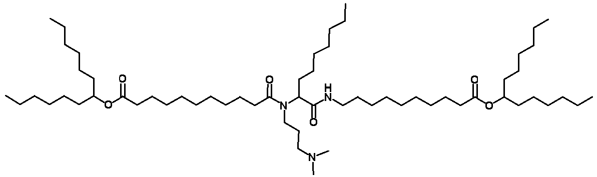

### Step 1: Preparation of Intermediate A12-3 (Same as Intermediate A1-3)

### Step 2: Preparation of Intermediate A12-4 (Same as Intermediate A1-4)

### Step 3: Preparation of Compound A12

The compound was synthesized according to preparation procedure A, with intermediate A12-1 being the same as A11-1 and intermediate A12-2 being A2-2. The crude product was purified by preparative HPLC under a gradient program of solvent A:solvent B:solvent C = 100:0:0 to 70:24:6, yielding a colorless oily compound A12 (168.0 mg, 36% yield). MS(ESI): [M+H]+ = 990.64.

### Preparation of Compound A13

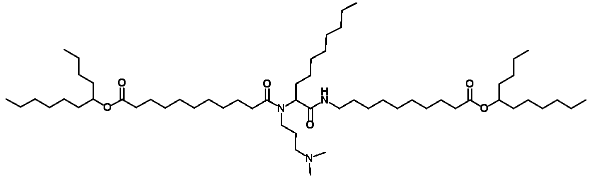

### Step 1: Preparation of Intermediate A13-3 (Same as Intermediate 3-3)

### Step 2: Preparation of Intermediate A13-4 (Same as Intermediate 3-4)

### Step 3: Preparation of Compound A13

The compound was synthesized according to preparation procedure A, with intermediate A13-1 being the same as A11-1 and intermediate A13-2 being the same as A1-2. The crude product was purified by preparative HPLC under a gradient program of solvent A:solvent B:solvent C = 100:0:0 to 70:24:6, yielding a colorless oily compound A13 (67 mg, 20% yield). MS(ESI): [M+H]+ = 948.56.

### Preparation of Compound A14

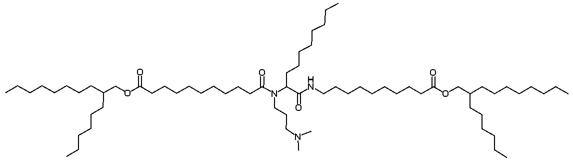

### Step 1: Preparation of Intermediate A14-3 (Same as Intermediate A4-3)

### Step 2: Preparation of Intermediate A14-4 (Same as Intermediate A4-4)

### Step 3: Preparation of Compound A14

The compound was synthesized according to preparation procedure A, with intermediate A14-1 being the same as A11-1 and intermediate A14-2 being the same as A1-2. The crude product was purified by preparative HPLC under a gradient program of solvent A:solvent B:solvent C = 100:0:0 to 70:24:6, yielding a colorless oily compound A14 (87.6 mg, 26% yield). MS(ESI): [M+H]+ = 1088.83.

### Preparation of Compound A15

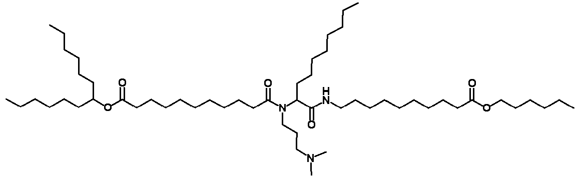

### Step 1: Preparation of Intermediate A15-3 (Same as Intermediate A1-3)

### Step 2: Preparation of Intermediate A15-4 (Same as Intermediate A5-4)

### Step 3: Preparation of Compound A15

The compound was synthesized according to preparation procedure A, with intermediate A15-1 being the same as A11-1 and intermediate A15-2 being the same as A1-2. The crude product was purified by preparative HPLC under a gradient program of solvent A:solvent B:solvent C = 100:0:0 to 70:24:6, yielding a colorless oily compound A15 (102.9 mg, 31% yield). MS(ESI): [M+H]+ = 906.48.

### Preparation of Compound A16

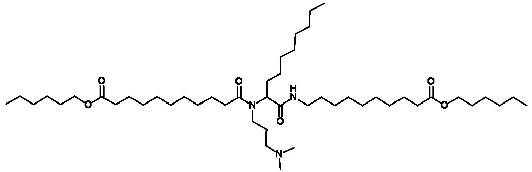

### Step 1: Preparation of Intermediate A16-3 (Same as Intermediate A6-3)

### Step 2: Preparation of Intermediate A16-4 (Same as Intermediate A5-4)

### Step 3: Preparation of Compound A16

The compound was synthesized according to preparation procedure A, with intermediate A16-1 being the same as A11-1 and intermediate A16-2 being the same as A1-2. The crude product was purified by preparative HPLC under a gradient program of solvent A:solvent B:solvent C = 100:0:0 to 70:24:6, yielding a colorless oily compound A16 (126.7 mg, 32% yield). MS(ESI): [M+H]+ = 808.29.

### Preparation of Compound A17

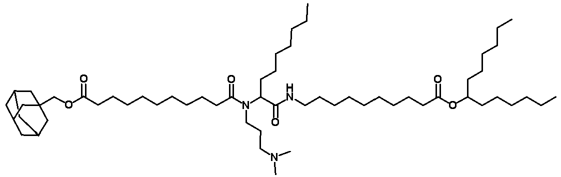

### Step 1: Preparation of Intermediate A17-3 (Same as Intermediate A7-3)

### Step 2: Preparation of Intermediate A17-4 (Same as Intermediate A1-4)

### Step 3: Preparation of Compound A17

The compound was synthesized according to preparation procedure A, with intermediate A17-1 being the same as A11-1 and intermediate A17-2 being the same as 2-2. The crude product was purified by preparative HPLC under a gradient program of solvent A:solvent B:solvent C = 100:0:0 to 70:24:6, yielding a colorless oily compound A17 (173.5 mg, 33% yield). MS(ESI): [M+H]+ = 956.54.

### Preparation of Compound A18

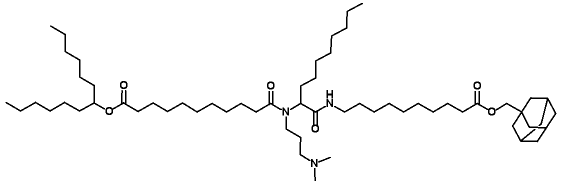

### Step 1: Preparation of Intermediate A18-3 (Same as Intermediate A1-3)

### Step 2: Preparation of Intermediate A18-4 (Same as Intermediate A8-4)

### Step 3: Preparation of Compound A18

The compound was synthesized according to preparation procedure A, with intermediate A18-1 being the same as A11-1 and intermediate A18-2 being the same as A1-2. The crude product was purified by preparative HPLC under a gradient program of solvent A:solvent B:solvent C = 100:0:0 to 70:24:6, yielding a colorless oily compound A18 (138.6 mg, 36% yield). MS(ESI): [M+H]+ = 970.56.

### Preparation of Compound A19

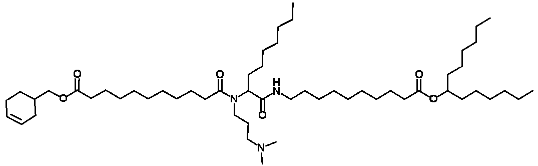

### Step 1: Preparation of Intermediate A19-3 (Same as Intermediate A8-3)

### Step 2: Preparation of Intermediate A19-4 (Same as Intermediate A1-4)

### Step 3: Preparation of Compound A19

The compound was synthesized according to preparation procedure A, with intermediate A19-1 being the same as A11-1 and intermediate A19-2 being the same as A1-2. The crude product was purified by preparative HPLC under a gradient program of solvent A:solvent B:solvent C = 100:0:0 to 70:24:6, yielding a colorless oily compound A19 (58.6 mg, 20% yield). MS(ESI): [M+H]+ = 902.44.

### Preparation of Compound A20

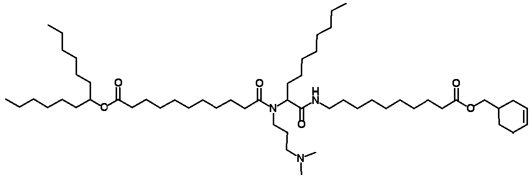

### Step 1: Preparation of Intermediate A20-3 (Same as Intermediate A1-3)

### Step 2: Preparation of Intermediate A20-4 (Same as Intermediate A10-4)

### Step 3: Preparation of Compound A20

The compound was synthesized according to preparation procedure A, with intermediate A20-1 being the same as A11-1 and intermediate A20-2 being the same as A1-2. The crude product was purified by preparative HPLC under a gradient program of solvent A:solvent B:solvent C = 100:0:0 to 70:24:6, yielding a colorless oily compound A20 (118.7 mg, 32% yield). MS(ESI): [M+H]+ = 916.47.

### Preparation of Compound A21

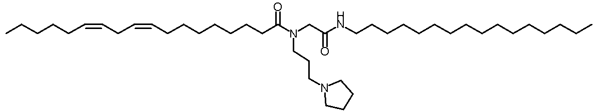

### Step 1: Preparation of Intermediate A21-4 (Same as Intermediate A1-4)

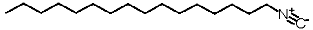

### Preparation of Compound A21-4-A

In a round-bottom flask, hexadecylamine (2.41 g, 10 mmol) was added to ethyl formate (25 mL), and the mixture was heated to 55°C and stirred overnight. TLC indicated the completion of the reaction. The mixture was concentrated under reduced pressure to yield a colorless oil (2.69 g, 100%), which was used without further purification or analysis.

### Preparation of Compound A21-4-b

In a round-bottom flask, hexadecylformamide (2.69 g, 10 mmol) and triethylamine (3.03 g, 30 mmol) were dissolved in DCM. The solution was cooled to 0°C, and under nitrogen protection, POCI3 (1.67 g, 10.9 mmol) was slowly added dropwise. After completion of addition, the reaction was allowed to proceed at room temperature for 4 hours. TLC indicated the completion of the reaction. The reaction was quenched with ice water, extracted with ethyl acetate (2×50 mL), and the organic phase was washed three times with brine, dried over anhydrous sodium sulfate, and concentrated under reduced pressure. The crude product was purified by preparative chromatography under a gradient program of solvent D:solvent E = 100:0 to 80:20, yielding a colorless oil (2.21 g, 88.0% yield).

### Step 2: Preparation of Compound A21

The compound was synthesized according to preparation procedure A, with intermediate A21-1 being the same as A1-1, intermediate A21-2 being formaldehyde, and intermediate A21-3 being linoleic acid. The crude product was purified by preparative HPLC under a gradient program of solvent A:solvent B:solvent C = 100:0:0 to 70:24:6, yielding a colorless oily compound A21 (102.8 mg, 35% yield). MS(ESI): [M+H]+ = [Mass value not provided].

### Preparation of Compound A22

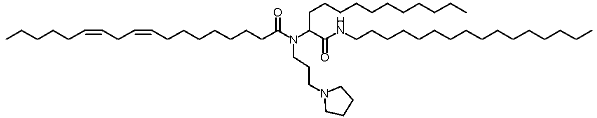

### Step 1: Preparation of Intermediate A22-4 (Same as Intermediate A21-4)

### Step 3: Preparation of Compound A22

The compound was synthesized according to preparation procedure A, with intermediate A22-1 being the same as A1-1, intermediate A20-2 being dodecanal, and intermediate A22-3 being the same as A21-3. The crude product was purified by preparative HPLC under a gradient program of solvent A:solvent B:solvent C = 100:0:0 to 70:24:6, yielding a colorless oily compound A22 (89.6 mg, 29% yield). MS(ESI): [M+H]+ = 827.44.

### Preparation of Compound A23

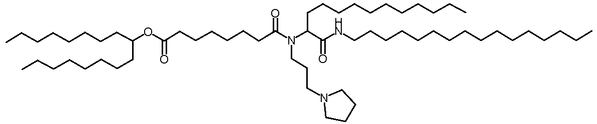

### Step 1: Preparation of Intermediate A23-3

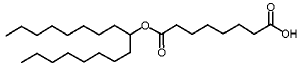

To a solution of 9-heptadecanol (1 g, 3.9 mmol), suberic acid (0.82 g, 4.7 mmol), and DMAP (0.1 g, 0.8 mmol) in dichloromethane, EDCI (1.12 g, 5.8 mmol) was added and stirred overnight at room temperature. TLC indicated the complete consumption of 9-heptadecanol. The crude product was purified by preparative chromatography under a gradient program of solvent D:solvent E = 100:0 to 80:20, yielding a colorless oily intermediate A23-3 (775 mg, 48% yield).

### Step 2: Preparation of Intermediate A23-4 (Same as Intermediate A21-4)

### Step 3: Preparation of Compound A23

The compound was synthesized according to preparation procedure A, with intermediate A23-1 being the same as A1-1 and intermediate A23-2 being the same as 22-2. The crude product was purified by preparative HPLC under a gradient program of solvent A:solvent B:solvent C = 100:0:0 to 70:24:6, yielding a colorless oily compound A23 (89.6 mg, 29% yield). MS(ESI): [M+H]+ = 959.64.

### Preparation of Compound A24

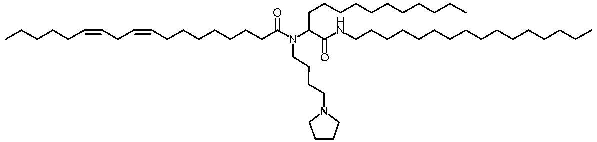

### Step 1: Preparation of Intermediate A24-3 (Same as Intermediate A23-3)

### Step 2: Preparation of Intermediate A24-4 (Same as Intermediate A21-4)

### Step 3: Preparation of Compound A24

The compound was synthesized according to preparation procedure A, with intermediate A24-1 being the same as A1-1 and intermediate A24-2 being the same as 22-2. The crude product was purified by preparative HPLC under a gradient program of solvent A:solvent B:solvent C = 100:0:0 to 70:24:6, yielding a colorless oily compound A24 (135.7 mg, 40% yield). MS(ESI): [M+H]+ = 841.46.

### Preparation of Compound A25

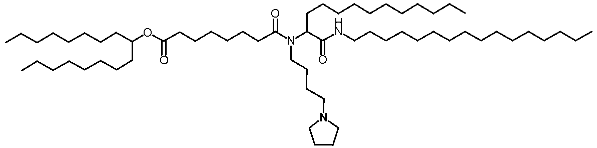

### Step 1: Preparation of Intermediate A25-3 (Same as Intermediate A23-3)

### Step 2: Preparation of Intermediate A25-4 (Same as Intermediate A21-4)

### Step 3: Preparation of Compound A25

The compound was synthesized according to preparation procedure A, with intermediate A25-1 being the same as A1-1 and intermediate A25-2 being the same as 22-2. The crude product was purified by preparative HPLC under a gradient program of solvent A:solvent B:solvent C = 100:0:0 to 70:24:6, yielding a colorless oily compound (189.2 mg, 47% yield). MS(ESI): [M+H]+ = 973.67.

### Preparation of Compound A26

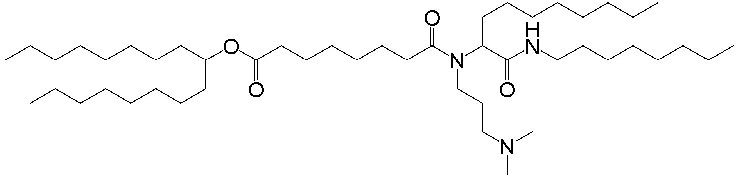

### Step 1: Preparation of Intermediate A26-3 (Same as Intermediate A23-3)

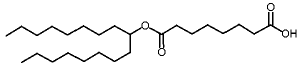

### Step 2: Preparation of Intermediate A26-4 (Same as Intermediate A21-4)

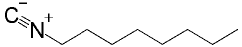

### Step 3: Preparation of Compound A26

The compound was synthesized according to preparation procedure A, with intermediate A26-1 being N,N-dimethyl-1,3-diaminopropane, intermediate A26-2 being nonanal, intermediate A26-3 being the same as A23-3, and intermediate A26-4 being n-octyl isonitrile. The crude product was purified by preparative HPLC under a gradient program of solvent A:solvent B:solvent C = 100:0:0 to 70:24:6, yielding a colorless oily compound (201 mg, 48% yield). MS(ESI): [M+H]+ = 778.7.

### Preparation of Compound A27

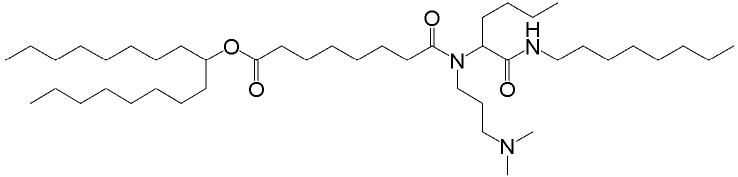

The compound was synthesized according to preparation procedure A, with intermediate A27-1 being N,N-dimethyl-1,3-diaminopropane, intermediate A27-2 being pentanal, intermediate A27-3 being the same as A23-3, and intermediate A27-4 being n-octyl isonitrile. The crude product was purified by preparative HPLC under a gradient program of solvent A:solvent B:solvent C = 100:0:0 to 70:24:6, yielding a colorless oily compound (155 mg, 41% yield). MS(ESI): [M+H]+ = 722.6.

### Preparation of Compound A28

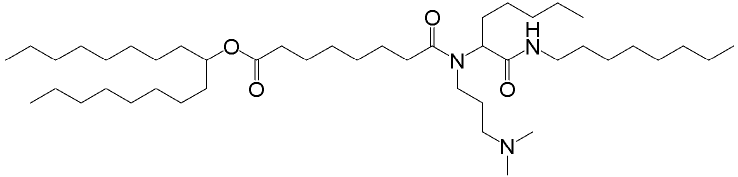

The compound was synthesized according to preparation procedure A, with intermediate A28-1 being N,N-dimethyl-1,3-diaminopropane, intermediate A28-2 being hexanal, intermediate A28-3 being the same as A23-3, and intermediate A28-4 being n-octyl isonitrile. The crude product was purified by preparative HPLC under a gradient program of solvent A:solvent B:solvent C = 100:0:0 to 70:24:6, yielding a colorless oily compound (224 mg, 53% yield). MS(ESI): [M+H]+ = 737.0.

### Preparation of Compound A29

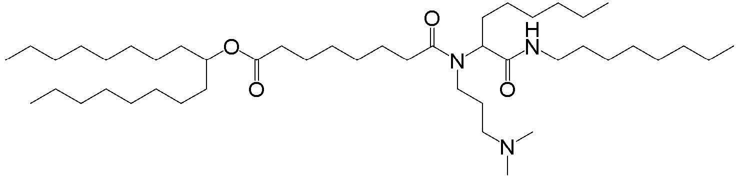

The compound was synthesized according to preparation procedure A, with intermediate A29-1 being N,N-dimethyl-1,3-diaminopropane, intermediate A29-2 being heptanal, intermediate A29-3 being the same as A23-3, and intermediate A29-4 being n-octyl isonitrile. The crude product was purified by preparative HPLC under a gradient program of solvent A:solvent B:solvent C = 100:0:0 to 70:24:6, yielding a colorless oily compound (164 mg, 38% yield). MS(ESI): [M+H]+ = 750.7.

### Preparation of Compound A30

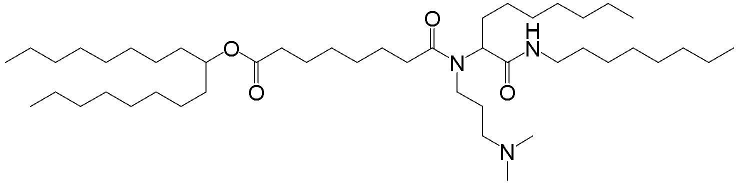

The compound was synthesized according to preparation procedure A, with intermediate A30-1 being N,N-dimethyl-1,3-diaminopropane, intermediate A30-2 being octanal, intermediate A30-3 being the same as A23-3, and intermediate A30-4 being n-octyl isonitrile. The crude product was purified by preparative HPLC under a gradient program of solvent A:solvent B:solvent C = 100:0:0 to 70:24:6, yielding a colorless oily compound (314 mg, 45% yield). MS(ESI): [M+H]+ = 764.7.

### Preparation of Compound A31

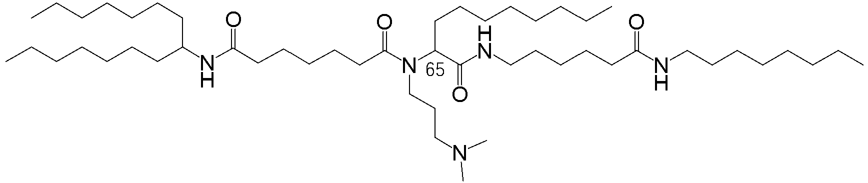

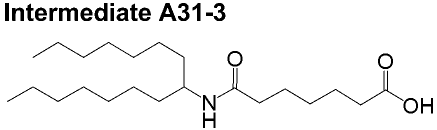

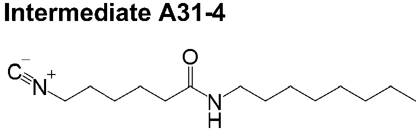

### Step 1: Preparation of Compound A41

The compound was synthesized according to preparation procedure A, with intermediate A31-1 being N,N-dimethyl-1,3-diaminopropane, intermediate A31-2 being nonanal, and intermediates A31-3 and A31-4 as described above. The crude product was purified by preparative HPLC under a gradient program of solvent A:solvent B:solvent C = 100:0:0 to 70:24:6, yielding a colorless oily compound (124 mg, 28% yield). MS(ESI): [M+H]+ = 848.8.

### Preparation of Compound A32

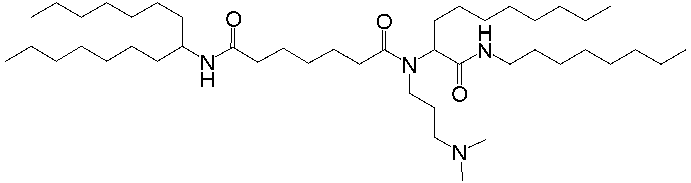

The compound was synthesized according to preparation procedure A, with intermediate A32-1 being N,N-dimethyl-1,3-diaminopropane, intermediate A32-2 being nonanal, intermediate A32-3 being the same as A31-3, and intermediate A32-4 being n-octyl isonitrile. The crude product was purified by preparative HPLC under a gradient program of solvent A:solvent B:solvent C = 100:0:0 to 70:24:6, yielding a colorless oily compound (311 mg, 56% yield). MS(ESI): [M+H]+ = 735.7.

### Preparation of Compound A33

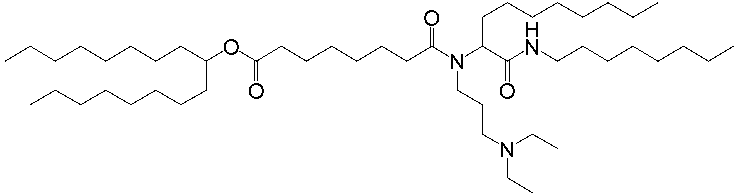

### Step 1: Preparation of Intermediate A33-3 (Same experimental procedure as Intermediate A23-3)

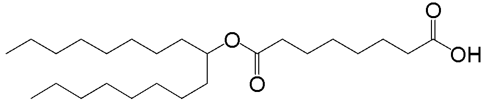

The compound was synthesized according to preparation procedure A, with intermediate A33-1 being 3-diethylaminopropylamine, intermediate A33-2 being nonanal, intermediate A33-3 as described above, and intermediate A33-4 being n-octyl isonitrile. The crude product was purified by preparative HPLC under a gradient program of solvent A:solvent B:solvent C = 100:0:0 to 70:24:6, yielding a colorless oily compound (136 mg, 59% yield). MS(ESI): [M+H]+ = 806.7.

### Preparation of Compound A34

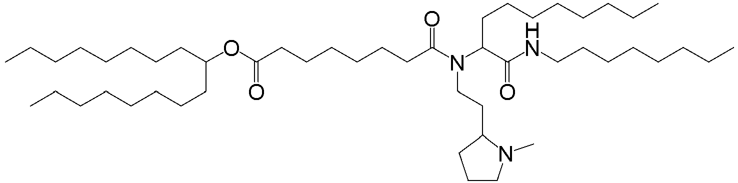

The compound was synthesized according to preparation procedure A, with intermediate A34-1 being N-methyl-2-(2-aminoethyl)pyrrolidine, intermediate A34-2 being nonanal, intermediate A34-3 being the same as A33-3, and intermediate A34-4 being n-octyl isonitrile. The crude product was purified by preparative HPLC under a gradient program of solvent A:solvent B:solvent C = 100:0:0 to 70:24:6, yielding a colorless oily compound (112 mg, 47% yield). MS(ESI): [M+H]+ = 804.7.

### Preparation of Compound A35

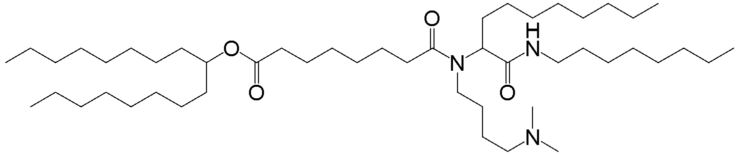

The compound was synthesized according to preparation procedure A, with intermediate A35-1 being N,N-dimethyl-1,3-diaminopropane, intermediate A35-2 being nonanal, intermediate A35-3 being the same as A33-3, and intermediate A35-4 being n-octyl isonitrile. The crude product was purified by preparative HPLC under a gradient program of solvent A:solvent B:solvent C = 100:0:0 to 70:24:6, yielding a colorless oily compound (161 mg, 44% yield). MS(ESI): [M+H]+ = 792.7.

### Preparation of Compound A36

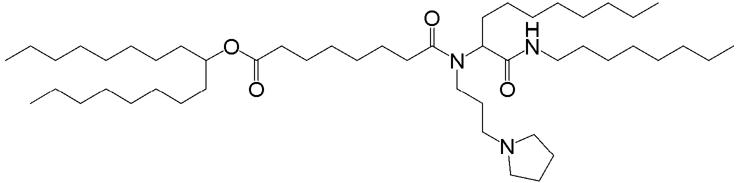

The compound was synthesized according to preparation procedure A, with intermediate A36-1 being 1-(3-aminopropyl)pyrrolidine, intermediate A36-2 being nonanal, intermediate A36-3 being the same as A33-3, and intermediate A36-4 being n-octyl isonitrile. The crude product was purified by preparative HPLC under a gradient program of solvent A:solvent B:solvent C = 100:0:0 to 70:24:6, yielding a colorless oily compound (190 mg, 50% yield). MS(ESI): [M+H]+ = 804.7.

### Preparation of Compound A37

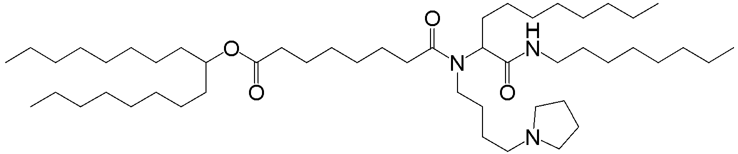

The compound was synthesized according to preparation procedure A, with intermediate A37-1 being 4-pyrrolidinobutylamine, intermediate A37-2 being nonanal, intermediate A37-3 being the same as A33-3, and intermediate A37-4 being n-octyl isonitrile. The crude product was purified by preparative HPLC under a gradient program of solvent A:solvent B:solvent C = 100:0:0 to 70:24:6, yielding a colorless oily compound (352 mg, 37% yield). MS(ESI): [M+H]+ = 818.

### Preparation of Compound A38

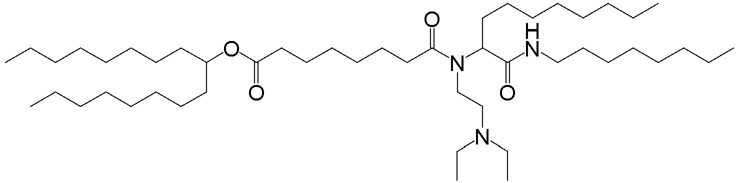

The compound was synthesized according to preparation procedure A, with intermediate A38-1 being N,N-diethyl ethylenediamine, intermediate A37-2 being nonanal, intermediate A38-3 being the same as A33-3, and intermediate A38-4 being n-octyl isonitrile. The crude product was purified by preparative HPLC under a gradient program of solvent A:solvent B:solvent C = 100:0:0 to 70:24:6, yielding a colorless oily compound (496 mg, 44% yield). MS(ESI): [M+H]+ = 792.

### Preparation of Compound A39

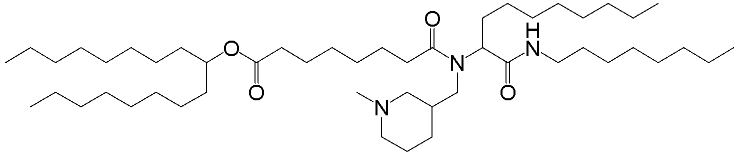

The compound was synthesized according to preparation procedure A, with intermediate A39-1 being 1-methyl-3-aminoethylpiperidine, intermediate A39-2 being nonanal, intermediate A39-3 being the same as A33-3, and intermediate A39-4 being n-octyl isonitrile. The crude product was purified by preparative HPLC under a gradient program of solvent A:solvent B:solvent C = 100:0:0 to 70:24:6, yielding a colorless oily compound (232 mg, 52% yield). MS(ESI): [M+H]+ = 804.

### Preparation of Compound A40

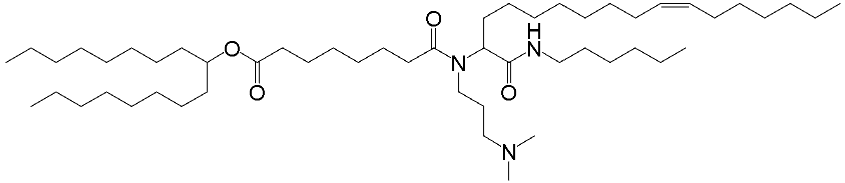

The compound was synthesized according to preparation procedure A, with intermediate A40-1 being N,N-dimethyl-1,3-diaminopropane, intermediate A40-2 being (Z)-7-hexadecenal, intermediate A40-3 being the same as A33-3, and intermediate A40-4 being n-hexyl isonitrile. The crude product was purified by preparative HPLC under a gradient program of solvent A:solvent B:solvent C = 100:0:0 to 70:24:6, yielding a colorless oily compound (216 mg, 43% yield). MS(ESI): [M+H]+ = 846.7.

### Preparation of Compound A41

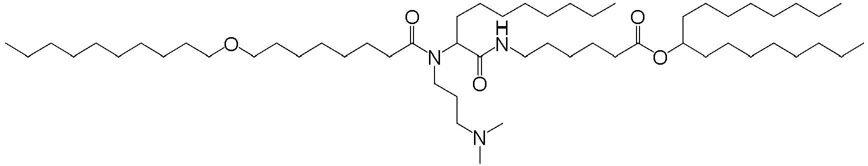

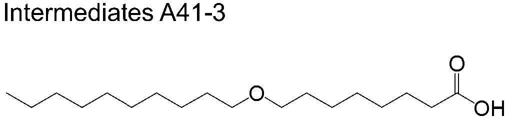

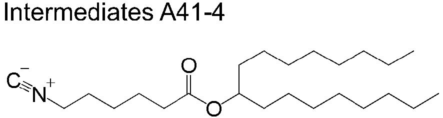

### Step 1: Preparation of Compound A41

The compound was synthesized according to preparation procedure A, with intermediate A41-1 being N,N-dimethyl-1,3-diaminopropane, intermediate A41-2 being nonanal, and intermediates A41-3 and A41-4 as described above. The crude product was purified by preparative HPLC under a gradient program of solvent A:solvent B:solvent C = 100:0:0 to 70:24:6, yielding a colorless oily compound (332 mg, 33% yield). MS(ESI): [M+H]+ = 907.

### Preparation of Compound A42

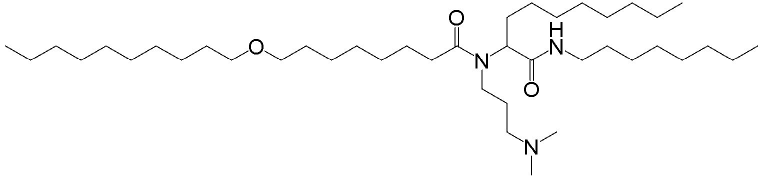

The compound was synthesized according to preparation procedure A, with intermediate A42-1 being N,N-dimethyl-1,3-diaminopropane, intermediate A42-2 being nonanal, intermediate A42-3 being the same as A41-3, and intermediate A42-4 being the same as A41-4. The crude product was purified by preparative HPLC under a gradient program of solvent A:solvent B:solvent C = 100:0:0 to 70:24:6, yielding a colorless oily compound (156 mg, 37% yield). MS(ESI): [M+H]+ = 666.6.

### Preparation of Compound A43

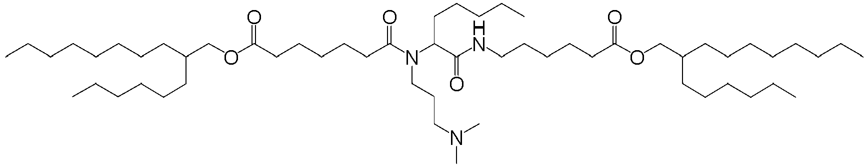

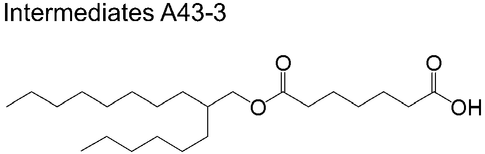

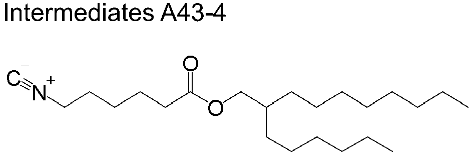

### Step 1: Preparation of Compound A43

The compound was synthesized according to preparation procedure A, with intermediate A43-1 being N,N-dimethyl-1,3-diaminopropane, intermediate A42-2 being hexanal, and intermediates A43-3 and A43-4 as described above. The crude product was purified by preparative HPLC under a gradient program of solvent A:solvent B:solvent C = 100:0:0 to 70:24:6, yielding a colorless oily compound (199 mg, 43% yield). MS(ESI): [M+H]+ = 934.8.

### Preparation of Compound A44

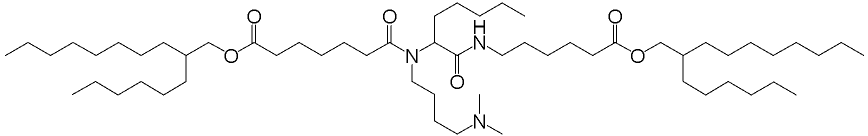

The compound was synthesized according to preparation procedure A, with intermediate A44-1 being 4-dimethylaminobutylamine, intermediate A44-2 being hexanal, intermediate A44-3 being the same as A43-3, and intermediate A44-4 being the same as A43-4. The crude product was purified by preparative HPLC under a gradient program of solvent A:solvent B:solvent C = 100:0:0 to 70:24:6, yielding a colorless oily compound (271 mg, 36% yield). MS(ESI): [M+H]+ = 948.

### Preparation of Compound A45

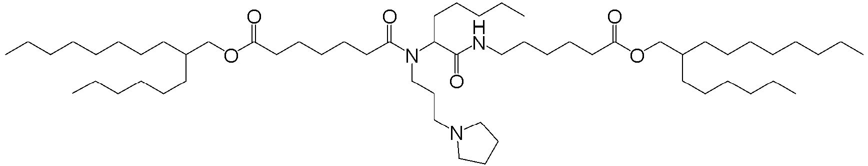

The compound was synthesized according to preparation procedure A, with intermediate A44-1 being 1-(3-aminopropyl)pyrrolidine, intermediate A44-2 being hexanal, intermediate A45-3 being the same as A43-3, and intermediate A45-4 being the same as A43-4. The crude product was purified by preparative HPLC under a gradient program of solvent A:solvent B:solvent C = 100:0:0 to 70:24:6, yielding a colorless oily compound (228 mg, 53% yield). MS(ESI): [M+H]+ = 960.8.

### Preparation of Compound A46

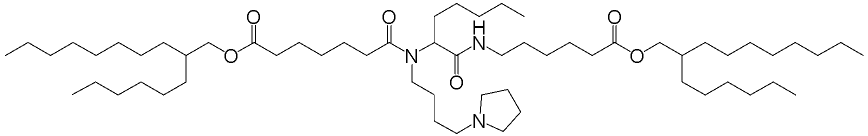

The compound was synthesized according to preparation procedure A, with intermediate A44-1 being 4-pyrrolidinobutylamine, intermediate A46-2 being hexanal, intermediate A46-3 being the same as A43-3, and intermediate A46-4 being the same as A43-4. The crude product was purified by preparative HPLC under a gradient program of solvent A:solvent B:solvent C = 100:0:0 to 70:24:6, yielding a colorless oily compound (119 mg, 42% yield). MS(ESI): [M+H]+ = 974.8.

### Preparation of Compound A47

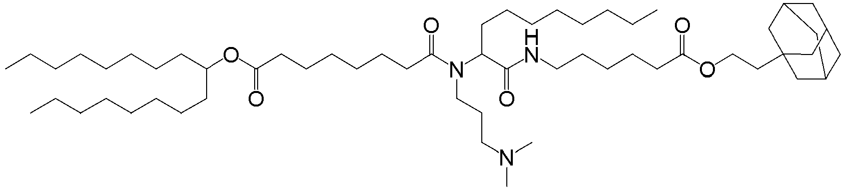

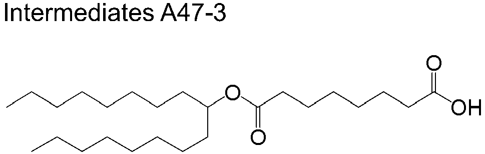

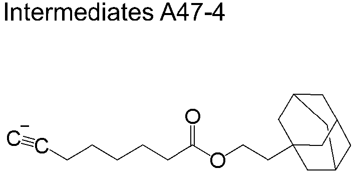

### Step 1: Preparation of Compound A47

The compound was synthesized according to preparation procedure A, with intermediate A47-1 being 4-dimethylaminobutylamine, intermediate A46-2 being nonanal, and intermediates A47-3 and A47-4 as described above. The crude product was purified by preparative HPLC under a gradient program of solvent A:solvent B:solvent C = 100:0:0 to 70:24:6, yielding a colorless oily compound (568 mg, 44% yield). MS(ESI): [M+H]+ = 941.8.

### Preparation of Compound A48

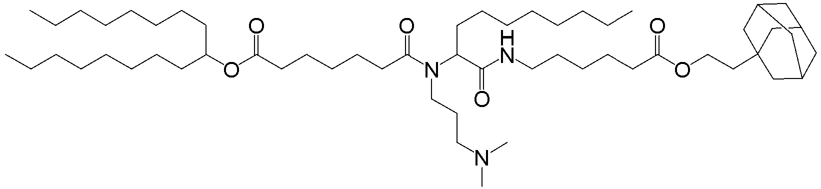

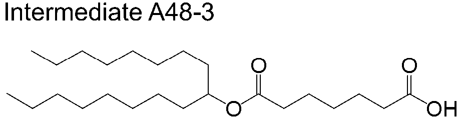

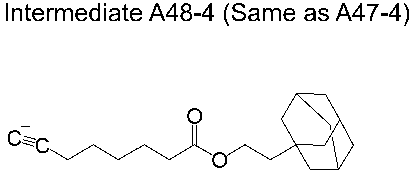

### Step 1: Preparation of Compound A48

The compound was synthesized according to preparation procedure A, with intermediate A48-1 being 4-dimethylaminobutylamine, intermediate A48-2 being nonanal, and intermediates A48-3 and A48-4 as described above. The crude product was purified by preparative HPLC under a gradient program of solvent A:solvent B:solvent C = 100:0:0 to 70:24:6, yielding a colorless oily compound (356 mg, 46% yield). MS(ESI): [M+H]+ = 928.8.

### 6.2 Example 2

### Preparation of Type B Compounds

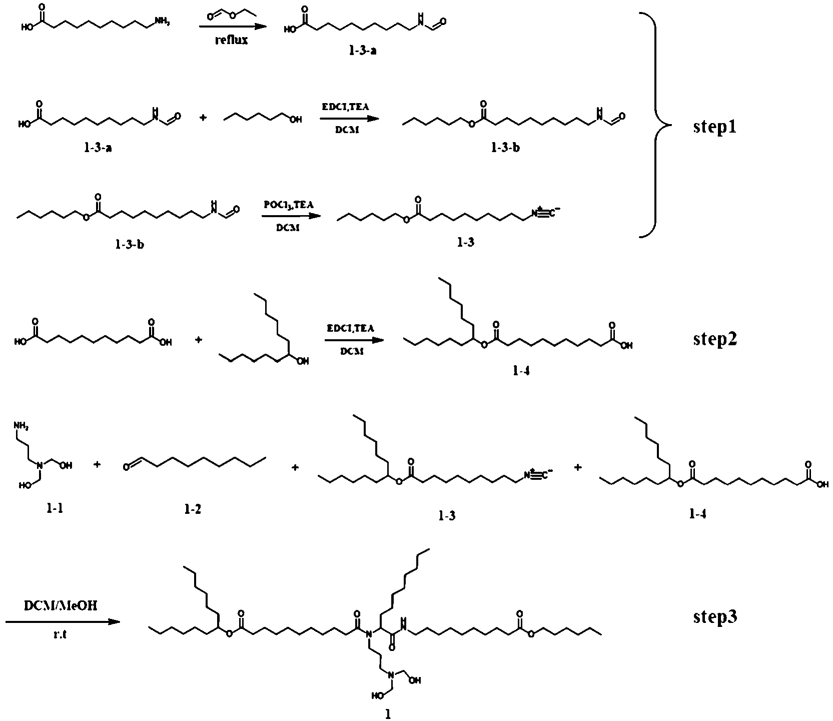

### Preparation of Compound B1

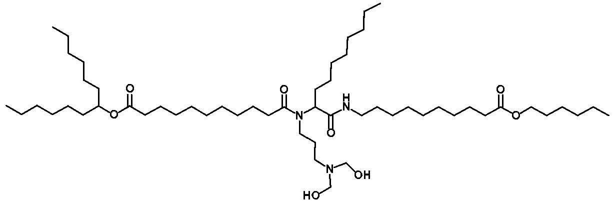

### Step 1: Preparation of Intermediate B1-3 (Same as A1-3)

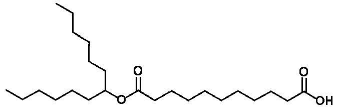

### Prepared using the same method as A1-3.

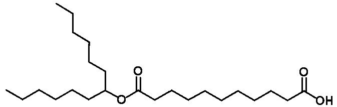

### Step 2: Preparation of Intermediate B1-4 (Same as A5-4)

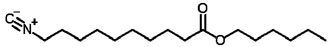

Prepared using the same method as A5-4.

### Step 3: Preparation of Compound B1

At room temperature, dissolve compound B1-1 (N-(3-aminopropyl)diethanolamine) (100 mg, 0.745 mmol) in ethanol. Add compound B1-2 (nonanal) (106.01 mg, 0.745 mmol) and stir for 1 hour. Then, add compound B1-3 (297.09 mg, 0.745 mmol) and continue stirring for 0.5 hours. Add compound B1-4 (282.93 mg, 0.745 mmol) and heat the mixture to 40°C for 16 hours. After TLC confirms the reaction completion, evaporate the mixture under reduced pressure and purify via preparative HPLC using a gradient program of solvent A:solvent B:solvent C = 100:0:0 to 70:24:6 to obtain colorless oily compound B1 (156.8 mg, 22% yield). MS(ESI): [M+H]+ = 937.81.

This preparation procedure is referred to as Standard Procedure B.

### Preparation of Compound B2

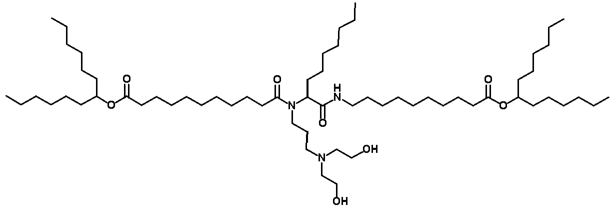

### Step 1: Preparation of Intermediate B2-3 (Same as Intermediate A1-3)

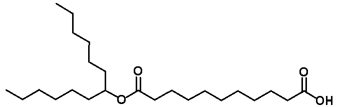

### Prepared using the same method as A1-3.

### Step 2: Preparation of Intermediate B2-4 (Same as Intermediate A1-4)

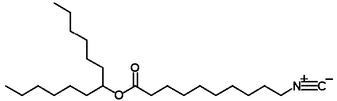

### Prepared using the same method as A1-4.

### Step 3: Preparation of Compound B2

The compound was synthesized according to preparation procedure B, with intermediate B2-1 being N-(3-aminopropyl)diethanolamine, intermediate B2-2 being octanal, and intermediates B2-3 and B2-4 as described above. The crude product was purified by preparative HPLC under a gradient program of solvent A:solvent B:solvent C = 100:0:0 to 70:24:6, yielding a colorless oily compound (128.8 mg, 30% yield). MS(ESI): [M+H]+ = 1051.69.

### Preparation of Compound B3

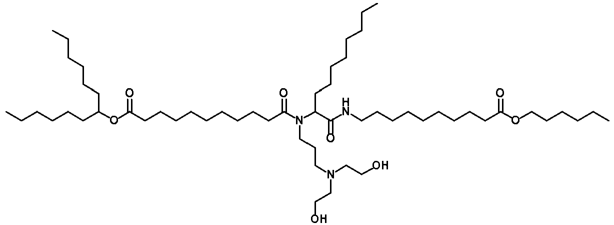

### Step 1: Preparation of Intermediate B3-3 (Same as Intermediate A1-3)

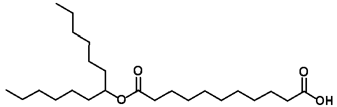

### Step 2: Preparation of Intermediate B3-4 (Same as Intermediate A1-4)

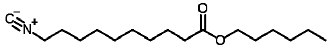

### Step 3: Preparation of Compound B3

The compound was synthesized according to preparation procedure B, with intermediate B3-1 being N-(3-aminopropyl)diethanolamine, intermediate B3-2 being nonanal, and intermediates B3-3 and B3-4 as described above. The crude product was purified by preparative HPLC under a gradient program of solvent A:solvent B:solvent C = 100:0:0 to 70:24:6, yielding a colorless oily compound (158.3 mg, 34% yield). MS(ESI): [M+H]+ = 967.53.

### Preparation of Compound B4

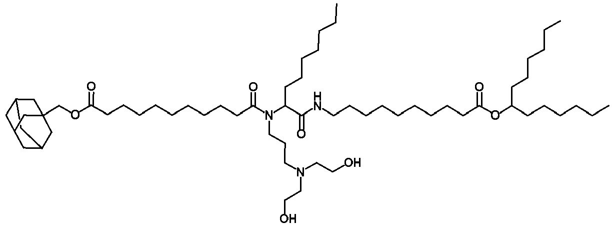

### Step 1: Preparation of Intermediate B4-3 (Same as Intermediate A7-3)

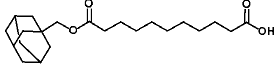

### Step 2: Preparation of Intermediate B4-4 (Same as Intermediate 1-4)

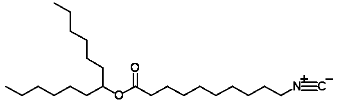

### Step 3: Preparation of Compound B4

The compound was synthesized according to preparation procedure B, with intermediate B4-1 being N-(3-aminopropyl)diethanolamine, intermediate B4-2 being octanal, and intermediates B4-3 and B4-4 as described above. The crude product was purified by preparative HPLC under a gradient program of solvent A:solvent B:solvent C = 100:0:0 to 70:24:6, yielding a colorless oily compound (205.6 mg, 42% yield). MS(ESI): [M+H]+ = 1017.59.

### Preparation of Compound B5

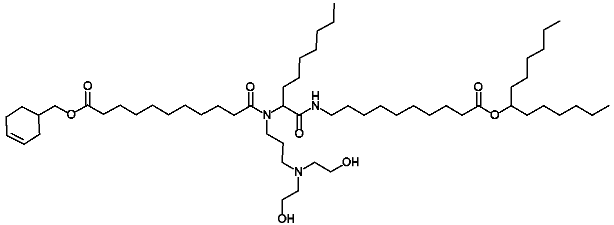

### Step 1: Preparation of Intermediate B5-3 (Same as Intermediate A9-3)

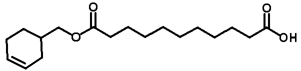

Prepared using the same method as intermediate A9-3.

### Step 2: Preparation of Intermediate B5-4 (Same as Intermediate A1-4)

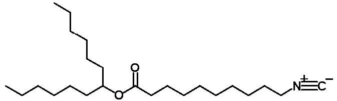

Prepared using the same method as A1-4.

### Step 3: Preparation of Compound B5

The compound was synthesized according to preparation procedure B, with intermediate B5-1 being N-(3-aminopropyl)diethanolamine, intermediate B5-2 being octanal, and intermediates B5-3 and B5-4 as described above. The crude product was purified by preparative HPLC under a gradient program of solvent A:solvent B:solvent C = 100:0:0 to 70:24:6, yielding a colorless oily compound (225.8 mg, 47% yield). MS(ESI): [M+H]+ = 963.50.

### Preparation of Compound B6

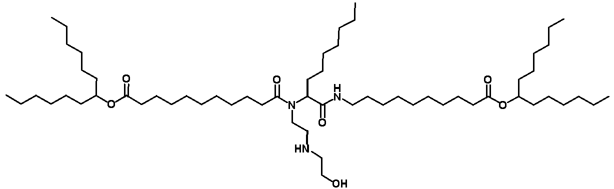

### Step 1: Preparation of Intermediate B6-3 (Same as Intermediate A1-3)

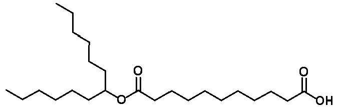

Prepared using the same method as A1-3.

### Step 2: Preparation of Intermediate B6-4 (Same as Intermediate A1-4)

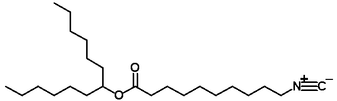

Prepared using the same method as A1-4.

### Step 3: Preparation of Compound B6

The compound was synthesized according to preparation procedure B, with intermediate B6-1 being hydroxyethyl ethylenediamine, intermediate B6-2 being octanal, and intermediates B6-3 and B6-4 as described above. The crude product was purified by preparative HPLC under a gradient program of solvent A:solvent B:solvent C = 100:0:0 to 70:24:6, yielding a colorless oily compound (125.8 mg, 27% yield). MS(ESI): [M+H]+ = 993.61.

### Preparation of Compound B7

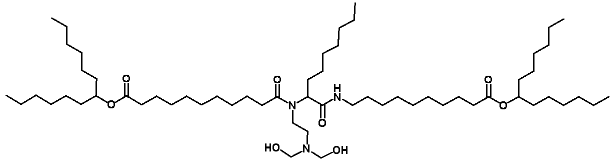

### Step 1: Preparation of Intermediate B7-3 (Same as Intermediate A1-3)

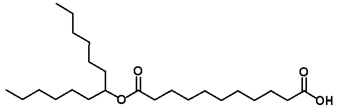

Prepared using the same method as A1-3.

### Step 2: Preparation of Intermediate B7-4 (Same as Intermediate A1-4)

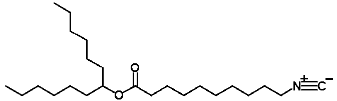

Prepared using the same method as A1-4.

### Step 3: Preparation of Compound B7

The compound was synthesized according to preparation procedure B, with intermediate B7-1 being N-(3-aminopropyl)diethanolamine, intermediate B7-2 being octanal, and intermediates B7-3 and B7-4 as described above. The crude product was purified by preparative HPLC under a gradient program of solvent A:solvent B:solvent C = 100:0:0 to 70:24:6, yielding a colorless oily compound (125.8 mg, 27% yield). MS(ESI): [M+H]+ = 1009.61.

### Preparation of Compound B8

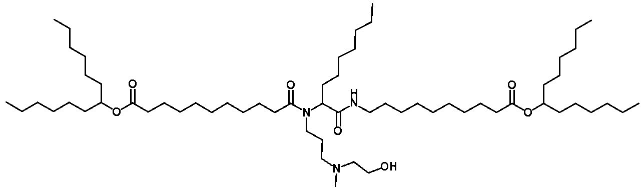

### Step 1: Preparation of Intermediate B8-3 (Same as Intermediate A1-3)

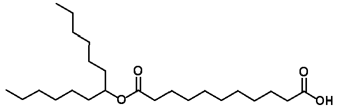

Prepared using the same method as A1-3.

### Step 2: Preparation of Intermediate B8-4 (Same as Intermediate A1-4)

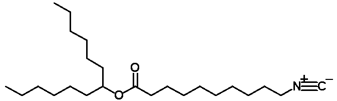

Prepared using the same method as A1-4.

### Step 3: Preparation of Compound B8

The compound was synthesized according to preparation procedure B, with intermediate B8-1 being N-methyl-N-(2-hydroxyethyl)-1,3-propanediamine, intermediate B8-2 being octanal, and intermediates B8-3 and B8-4 as described above. The crude product was purified by preparative HPLC under a gradient program of solvent A:solvent B:solvent C = 100:0:0 to 70:24:6, yielding a colorless oily compound (106.1 mg, 25% yield). MS(ESI): [M+H]+ = 1021.66.

### Preparation of Compound B9

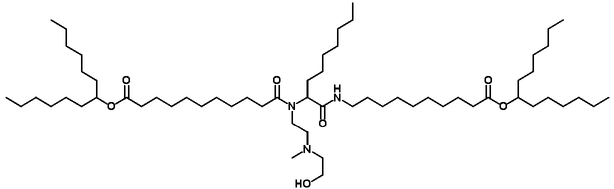

### Step 1: Preparation of Intermediate B9-3 (Same as Intermediate A1-3)

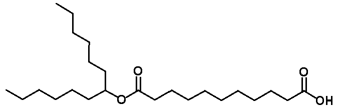

Prepared using the same method as A1-3.

### Step 2: Preparation of Intermediate B9-4 (Same as Intermediate A1-4)

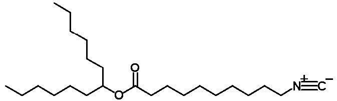

Prepared using the same method as A1-4.

### Step 3: Preparation of Compound B9

The compound was synthesized according to preparation procedure B, with intermediate B9-1 being 2-(2-aminoethyl-methyl-amino)ethanol, intermediate B9-2 being octanal, and intermediates B9-3 and B9-4 as described above. The crude product was purified by preparative HPLC under a gradient program of solvent A:solvent B:solvent C = 100:0:0 to 70:24:6, yielding a colorless oily compound (106.1 mg, 25% yield). MS(ESI): [M+H]+ = 1008.64.

### Preparation of Compound B10

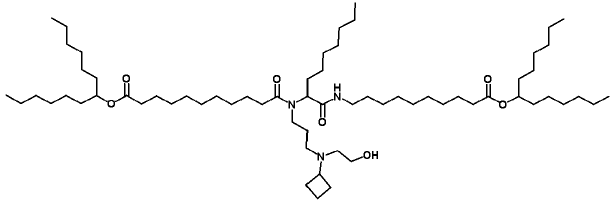

### Step 1: Preparation of Intermediate B10-1

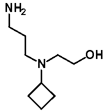

### Preparation of Compound B10-1-a

At 0°C, add DIEA (778.58 mg, 6.02 mmol) to a solution of N-BOC-3-chloropropylamine (350 mg, 1.81 mmol) and 2-(cyclobutylamino)ethanol (173.45 mg, 1.5 mmol) in THF. Stir the mixture at 70°C for 16 hours. Purify via preparative chromatography to obtain compound B10-1-a.

### Preparation of Compound B10-1

Dissolve compound B10-1-a in 10 volumes of THF, add an equal volume of trifluoroacetic acid, and stir at room temperature for 4 hours. After TLC confirms the reaction completion, adjust the pH to 8 with 1N sodium bicarbonate solution, extract with DCM three times, and evaporate the combined organic phases to dryness to obtain compound B10-1.

### Step 2: Preparation of Intermediate B10-3 (Same as Intermediate A1-3)

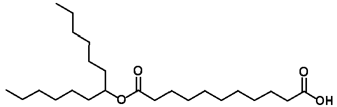

### Step 3: Preparation of Intermediate B10-4 (Same as Intermediate A1-4)

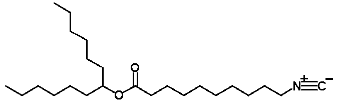

### Step 4: Preparation of Compound B10

The compound was synthesized according to preparation procedure B, with intermediate B10-2 being octanal, and intermediates B10-1, B10-3, and B10-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 1061.73.

### Preparation of Compound B11

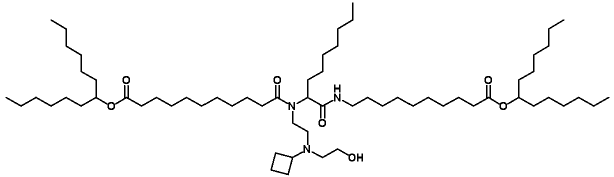

### Step 1: Preparation of Intermediate B11-1

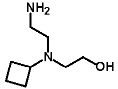

Prepared using the same method as compound B10-1.

### Step 2: Preparation of Intermediate B11-3 (Same as Intermediate A1-3)

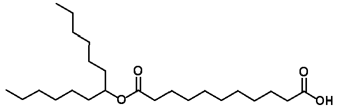

### Step 3: Preparation of Intermediate B11-4 (Same as Intermediate A1-4)

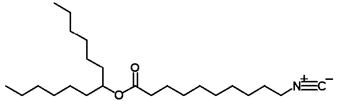

### Step 4: Preparation of Compound B11

The compound was synthesized according to preparation procedure B, with intermediate B11-2 being octanal, and intermediates B11-1, B11-3, and B11-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 1047.70.

### Preparation of Compound B12

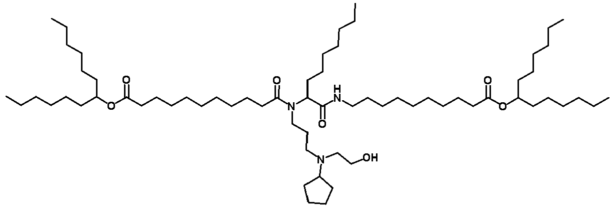

### Step 1: Preparation of Intermediate B12-1

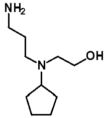

Prepared using the same method as compound B10-1.

### Step 2: Preparation of Intermediate B12-3 (Same as Intermediate A1-3)

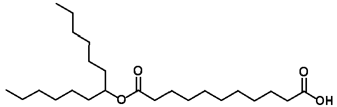

### Step 3: Preparation of Intermediate B12-4 (Same as Intermediate A1-4)

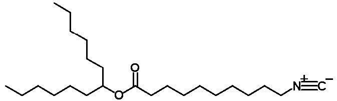

### Step 4: Preparation of Compound B12

The compound was synthesized according to preparation procedure B, with intermediate B12-2 being octanal, and intermediates B12-1, B12-3, and B12-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 1074.76.

### Preparation of Compound B13

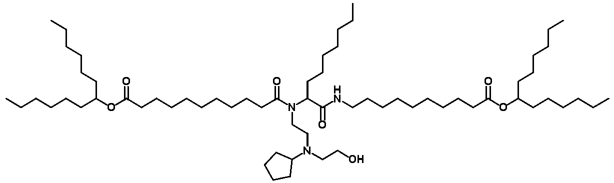

### Step 1: Preparation of Intermediate B13-1

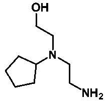

Prepared using the same method as compound B10-1.

### Step 2: Preparation of Intermediate B13-3 (Same as Intermediate A1-3)

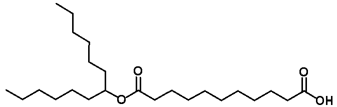

### Step 3: Preparation of Intermediate B13-4 (Same as Intermediate A1-4)

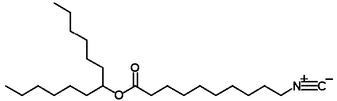

### Step 4: Preparation of Compound B13

The compound was synthesized according to preparation procedure B, with intermediate B13-2 being octanal, and intermediates B13-1, B13-3, and B13-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 1061.73.

### Preparation of Compound B14

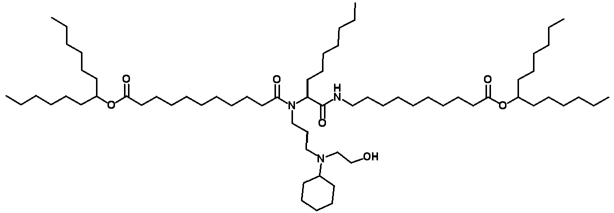

### Step 1: Preparation of Intermediate B14-1

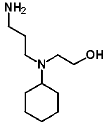

Prepared using the same method as compound B10-1.

### Step 2: Preparation of Intermediate B14-3 (Same as Intermediate A1-3)

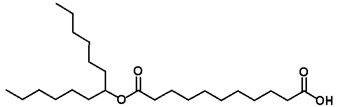

### Step 3: Preparation of Intermediate B14-4 (Same as Intermediate A1-4)

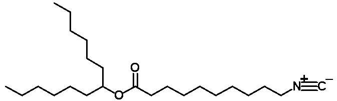

### Step 4: Preparation of Compound B14

The compound was synthesized according to preparation procedure B, with intermediate B14-2 being octanal, and intermediates B14-1, B14-3, and B14-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 1089.78.

### Preparation of Compound B15

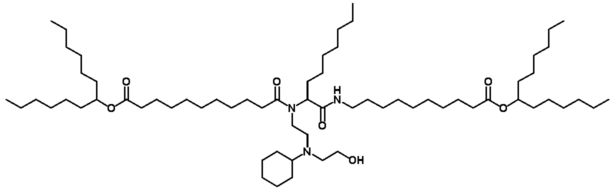

### Step 1: Preparation of Intermediate B15-1

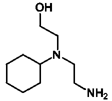

Prepared using the same method as compound B10-1.

### Step 2: Preparation of Intermediate B15-3 (Same as Intermediate A1-3)

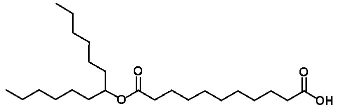

### Step 3: Preparation of Intermediate B15-4 (Same as Intermediate A1-4)

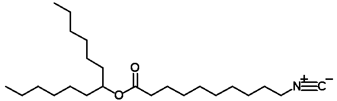

### Step 4: Preparation of Compound B15

The compound was synthesized according to preparation procedure B, with intermediate B15-2 being octanal, and intermediates B15-1, B15-3, and B15-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 1075.76.

### Preparation of Compound B16

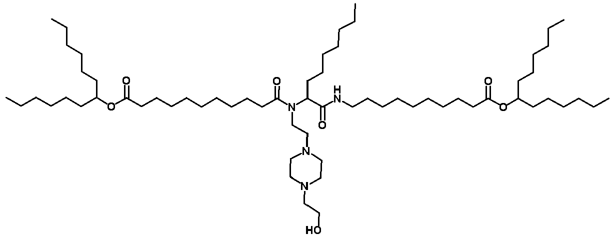

### Step 2: Preparation of Intermediate B16-3 (Same as Intermediate A1-3)

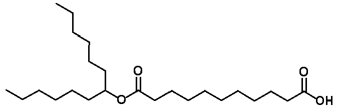

### Step 3: Preparation of Intermediate B16-4 (Same as Intermediate A1-4)

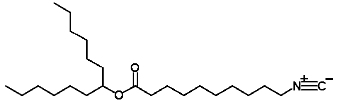

### Step 4: Preparation of Compound B16

The compound was synthesized according to preparation procedure B, with intermediate B16-1 being 2-(4-(2-aminoethyl)piperazin-1-yl)ethanol, intermediate B16-2 being octanal, and intermediates B12-3 and B12-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 1062.72.

Compounds B17-B25 were synthesized and purified according to Standard Procedure B as described above.

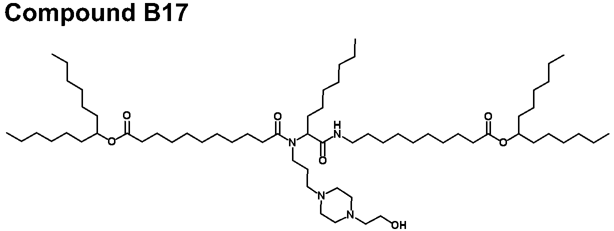

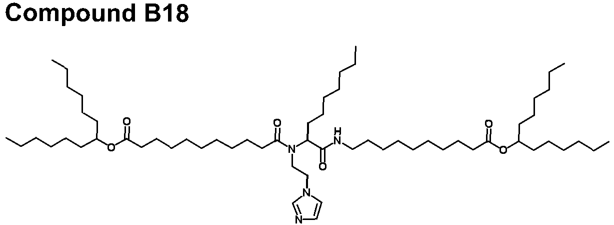

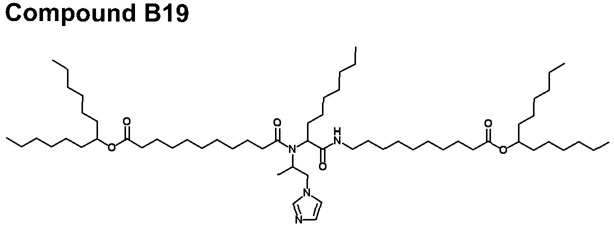

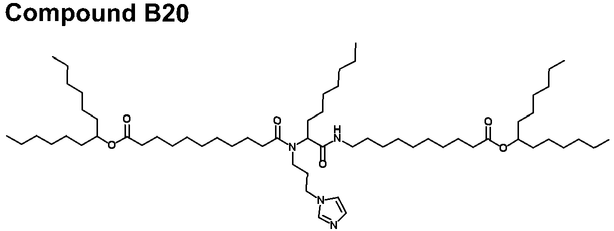

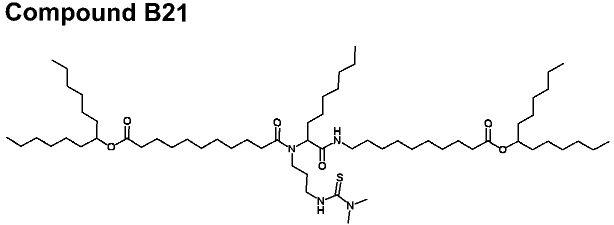

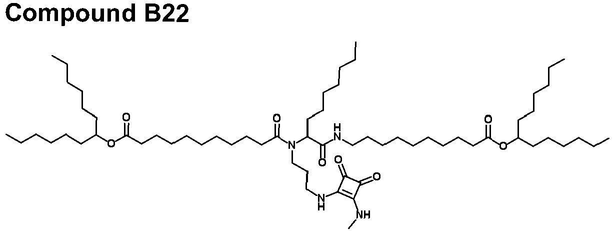

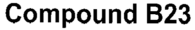

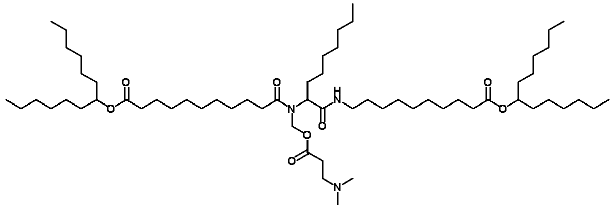

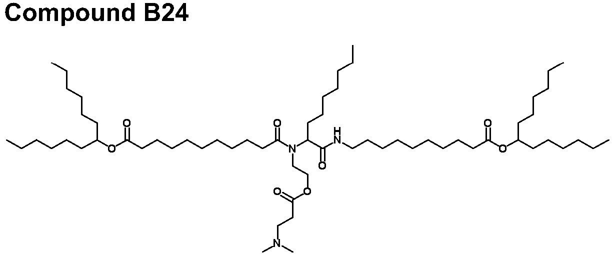

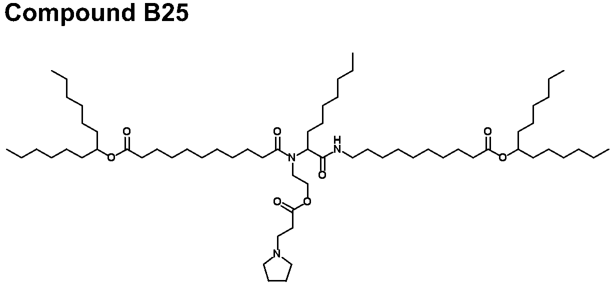

### Preparation of Compound B26

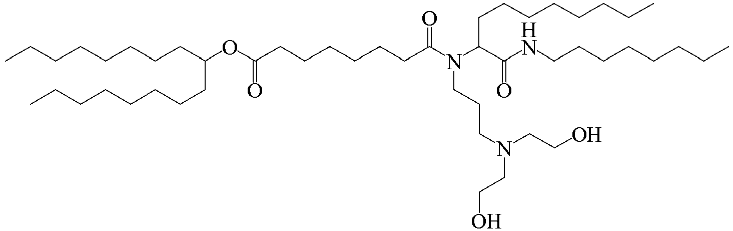

### Step 1: Preparation of Intermediate B26-3 (Same as Intermediate A1-3)

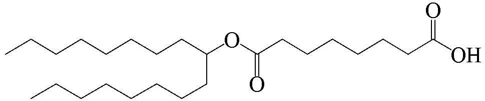

### Step 2: Preparation of Intermediate B26-4 (Same as Intermediate A1-4)

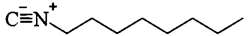

### Step 3: Preparation of Compound B26

The compound was synthesized according to preparation procedure B, with intermediate B26-1 being N-(3-aminopropyl)diethanolamine, intermediate B26-2 being nonanal, and intermediates B26-3 and B26-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 838.7.

### Preparation of Compound B27

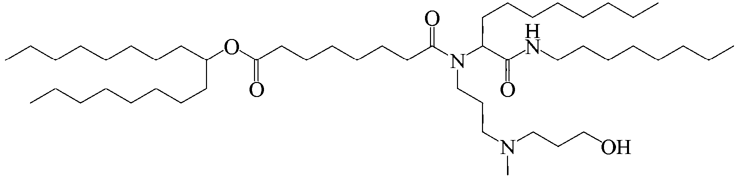

### Step 1: Preparation of Compound B27

The compound was synthesized according to preparation procedure B, with intermediate B27-1 being N-(3-hydroxypropyl)-N-methyl-1,3-propanediamine, intermediate B26-2 being nonanal, and intermediates B26-3 and B26-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 828.

### Preparation of Compound B28

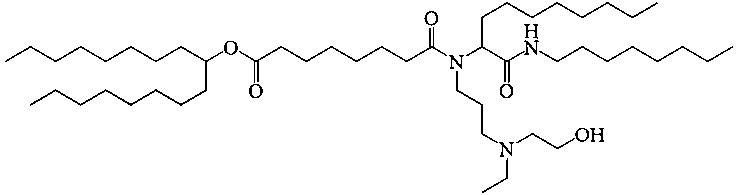

### Step 1: Preparation of Compound B28

The compound was synthesized according to preparation procedure B, with intermediate B28-1 being (3-aminopropyl)-(2-hydroxyethyl)ethylamine, intermediate B26-2 being nonanal, and intermediates B26-3 and B26-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 822.

### Preparation of Compound B29

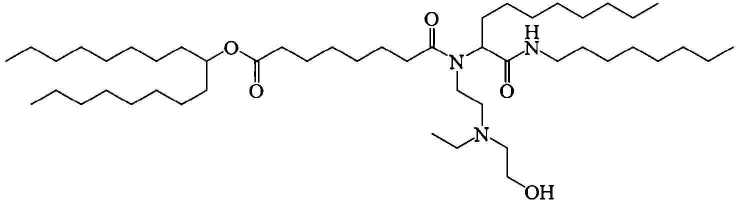

### Step 1: Preparation of Compound B29

The compound was synthesized according to preparation procedure B, with intermediate B29-1 being N-ethyl-N-ethanol-ethylenediamine, intermediate B26-2 being nonanal, and intermediates B26-3 and B26-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 808.8.

### Preparation of Compound B30

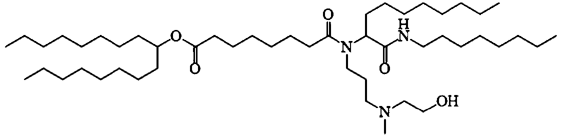

### Step 1: Preparation of Compound B30

The compound was synthesized according to preparation procedure B, with intermediate B30-1 being (3-aminopropyl)-(2-hydroxyethyl)ethylamine, intermediate B26-2 being nonanal, and intermediates B26-3 and B26-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 808.8.

### Preparation of Compound B31

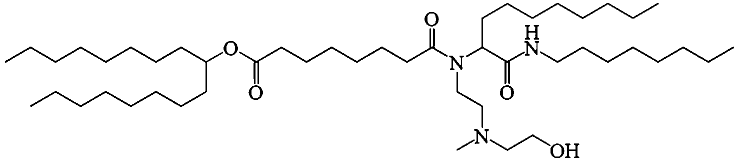

### Step 1: Preparation of Compound B31

The compound was synthesized according to preparation procedure B, with intermediate B31-1 being 2-(2-aminoethyl-methyl-amino)ethanol, intermediate B26-2 being nonanal, and intermediates B26-3 and B26-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 794.4.

### Preparation of Compound B32

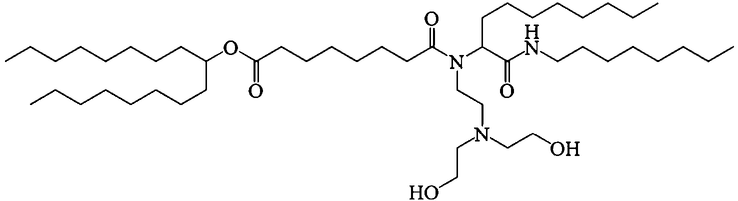

### Step 1: Preparation of Compound B32

The compound was synthesized according to preparation procedure B, with intermediate B32-1 being N-(3-aminoethyl)diethanolamine, intermediate B26-2 being nonanal, and intermediates B26-3 and B26-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 824.7.

### Preparation of Compound B33

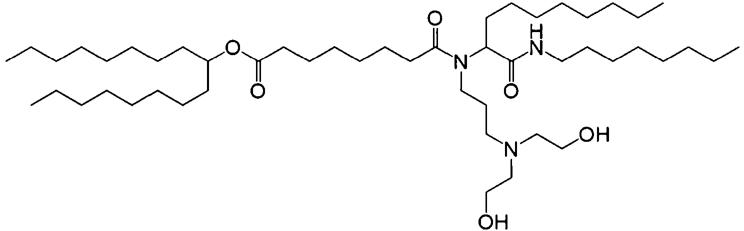

### Step 1: Preparation of Compound B33

The compound was synthesized according to preparation procedure B, with intermediate B33-1 being N-(3-aminopropyl)diethanolamine, intermediate B26-2 being nonanal, and intermediates B26-3 and B26-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 838.7.

### Preparation of Compound B34

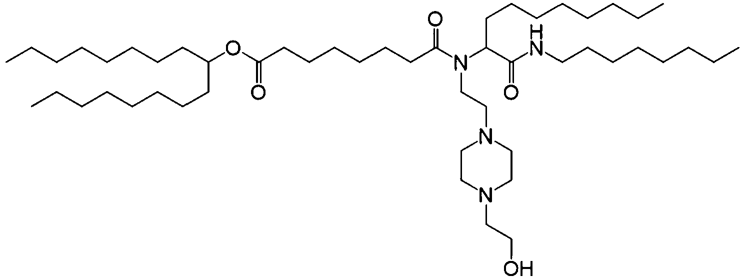

### Step 1: Preparation of Compound B34

The compound was synthesized according to preparation procedure B, with intermediate B34-1 being 2-(4-(2-aminoethyl)piperazin-1-yl)ethanol, intermediate B26-2 being nonanal, and intermediates B26-3 and B26-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 849.7.

### Preparation of Compound B35

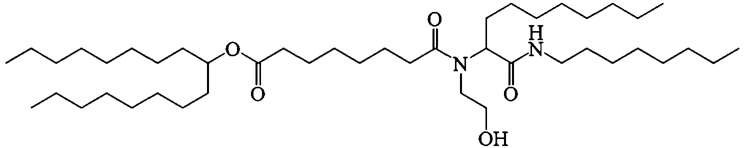

### Step 1: Preparation of Compound B35

The compound was synthesized according to preparation procedure B, with intermediate B35-1 being ethanolamine, intermediate B26-2 being nonanal, and intermediates B26-3 and B26-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 737.

### Preparation of Compound B36

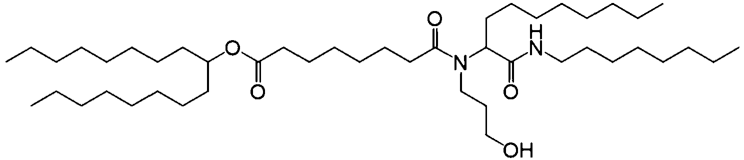

### Step 1: Preparation of Compound B36

The compound was synthesized according to preparation procedure B, with intermediate B36-1 being 3-amino-1-propanol, intermediate B26-2 being nonanal, and intermediates B26-3 and B26-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 751.

### Preparation of Compound B37

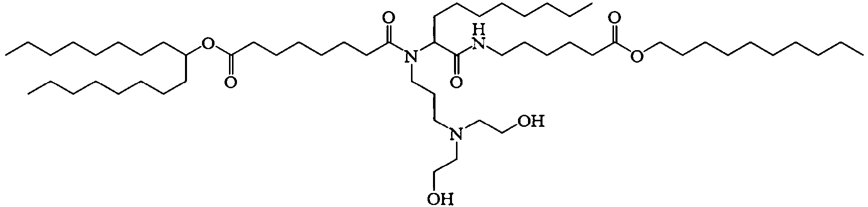

### Step 1: Preparation of Intermediate B37-4 (Same as Intermediate A1-4)

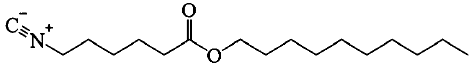

### Step 2: Preparation of Compound B37

The compound was synthesized according to preparation procedure B, with intermediate B37-1 being N-(3-aminopropyl)diethanolamine, intermediate B26-2 being nonanal, and intermediates B26-3 and B37-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 980.

### Preparation of Compound B38

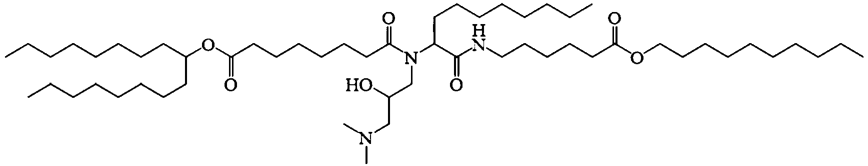

### Step 1: Preparation of Compound B38

The compound was synthesized according to preparation procedure B, with intermediate B38-1 being 1-amino-3-dimethylamino-2-propanol, intermediate B26-2 being nonanal, and intermediates B26-3 and B37-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 936.8.

### Preparation of Compound B39

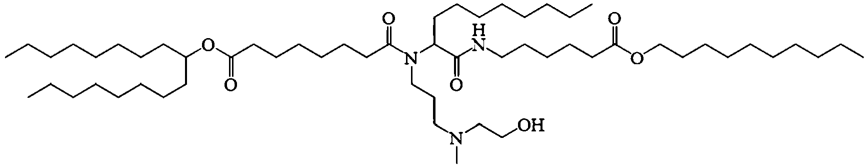

### Step 1: Preparation of Compound B39

The compound was synthesized according to preparation procedure B, with intermediate B39-1 being (3-aminopropyl)-(2-hydroxyethyl)ethylamine, intermediate B26-2 being nonanal, and intermediates B26-3 and B37-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 950.8.

### Preparation of Compound B40

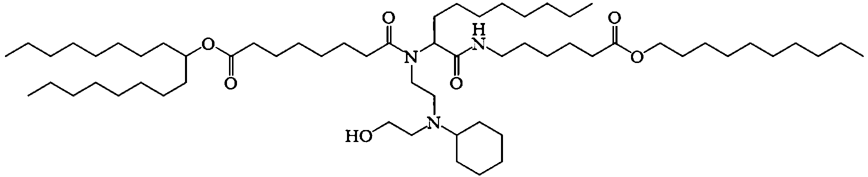

### Step 1: Preparation of Compound B40

The compound was synthesized according to preparation procedure B, with intermediate B40-1 being 2-[(2-aminoethyl)cyclohexylamino]ethanol, intermediate B26-2 being nonanal, and intermediates B26-3 and B37-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 1004.8.

### Preparation of Compound B41

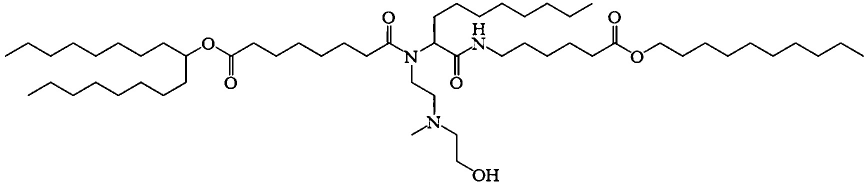

### Step 1: Preparation of Compound B41

The compound was synthesized according to preparation procedure B, with intermediate B41-1 being 2-[(2-aminoethyl)cyclohexylamino]ethanol, intermediate B26-2 being nonanal, and intermediates B26-3 and B37-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 936.8.

### Preparation of Compound B42

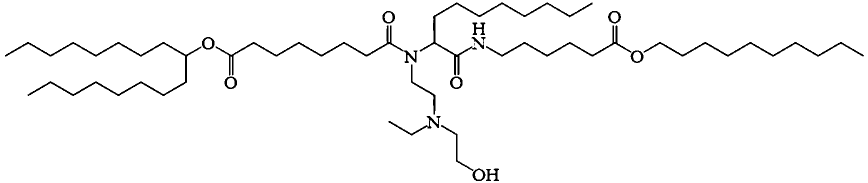

### Step 1: Preparation of Compound B42

The compound was synthesized according to preparation procedure B, with intermediate B42-1 being N-ethyl-N-ethanol-ethylenediamine, intermediate B26-2 being nonanal, and intermediates B26-3 and B37-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 950.8.

### Preparation of Compound B43

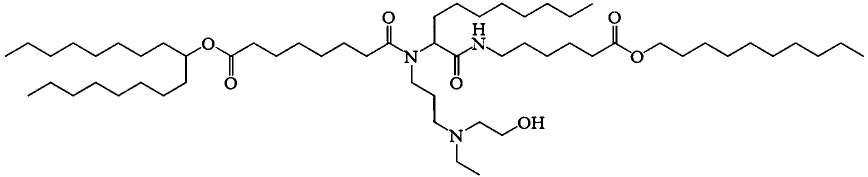

### Step 1: Preparation of Compound B43

The compound was synthesized according to preparation procedure B, with intermediate B43-1 being (3-aminopropyl)-(2-hydroxyethyl)ethylamine, intermediate B26-2 being nonanal, and intermediates B26-3 and B37-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 964.8.

### Preparation of Compound B44

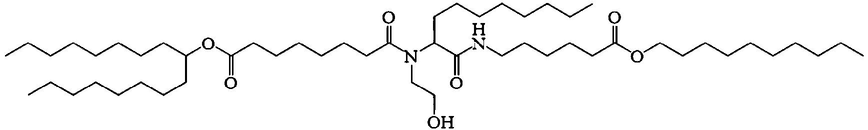

### Step 1: Preparation of Compound B44

The compound was synthesized according to preparation procedure B, with intermediate B44-1 being ethanolamine, intermediate B26-2 being nonanal, and intermediates B26-3 and B37-4 as described above. The crude product was purified by preparative HPLC under a gradient program of solvent A:solvent B:solvent C = 100:0:0 to 70:24:6, yielding a colorless oily compound (96.7 mg, 23% yield). MS(ESI): [M+H]+ = 878.7.

### 6.3 Example 3: Preparation of Type C Compounds Preparation of Compound C1

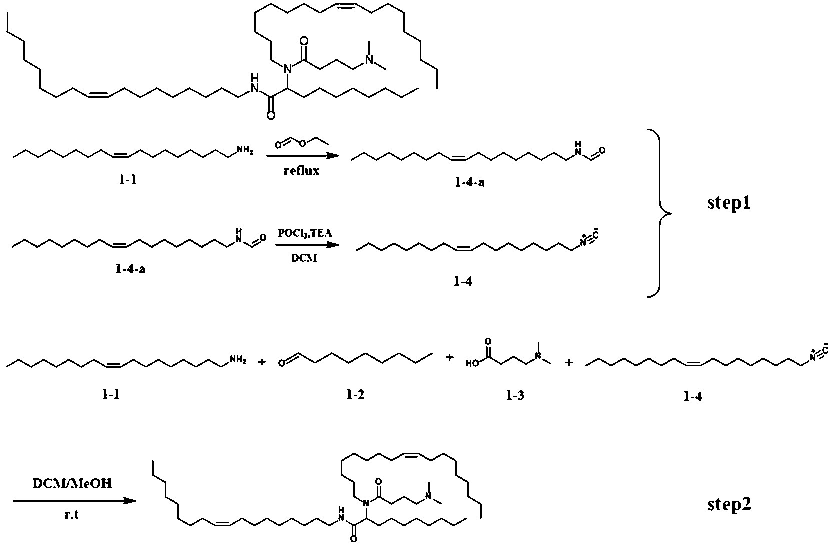

### Step 1: Preparation of Intermediate C1-4

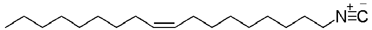

### Preparation of Compound C1-4-a

In a round-bottom flask, add oleylamine (1 g, 3.7 mmol) and ethyl formate (10 mL), and heat the mixture to 55°C for an overnight reaction. TLC shows the reaction completion. Evaporate the mixture under reduced pressure to obtain a colorless oily substance (1.09 g, 100%), which is used without further purification or analysis.

### Preparation of Compound C1-4

In a round-bottom flask, add C1-4-a (1.09 g, 3.7 mmol) and triethylamine (1.12 g, 11.1 mmol), dissolve in DCM, cool the solution to 0°C, and slowly add POCI3 (0.68 g, 4.44 mmol) under nitrogen protection. After the addition is complete, stir the reaction at room temperature for 4 hours. TLC shows the reaction completion. Quench the reaction with ice water, extract with ethyl acetate (2×20 mL), combine the organic phases, wash three times with brine, dry over anhydrous sodium sulfate, and evaporate the mixture under reduced pressure. Purify the crude product by preparative chromatography under a gradient program of solvent D:solvent E = 100:0 to 80:20 to obtain a light yellow oily substance (0.34 g, 84.0% yield).

### Step 2: Preparation of Compound C1

At room temperature, dissolve compound C1-1 (oleylamine) (100 mg, 0.374 mmol) in dichloromethane/methanol (VDCM:VMeOH = 3:1). Add compound D1-2 (nonanal) (53.17 mg, 0.374 mmol) and stir for 1 hour. Then, add compound 1-3 (4-dimethylaminobutyric acid) (49.04 mg, 0.374 mmol) and continue stirring for 0.5 hours. Add compound 1-4 (103.74 mg, 0.374 mmol) and stir the mixture at room temperature for 16 hours. TLC shows the reaction completion. Evaporate the mixture under reduced pressure and purify via preparative HPLC under a gradient program of solvent A:solvent B:solvent C = 100:0:0 to 70:24:6 to obtain colorless oily compound C1 (125.7 mg, 42% yield). MS(ESI): [M+H]+ = 800.80.

This preparation procedure is referred to as Standard Procedure C.

### Preparation of Compound C2

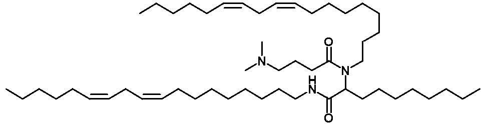

### Step 1: Preparation of Intermediate C2-4

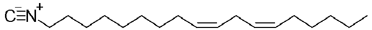

### Preparation of Compound C2-4-a

In a round-bottom flask, add linoleylamine (0.5 g, 1.8 mmol) and ethyl formate (5 mL), and heat the mixture to 55°C for an overnight reaction. TLC shows the reaction completion. Evaporate the mixture under reduced pressure to obtain a colorless oily substance (0.52 g, 100%), which is used without further purification or analysis.

### Preparation of Compound C2-4

In a round-bottom flask, add C2-4-a (0.52 g, 1.8 mmol) and triethylamine (0.54 g, 5.4 mmol), dissolve in DCM, cool the solution to 0°C, and slowly add POCI3 (0.30 g, 1.962 mmol) under nitrogen protection. After the addition is complete, stir the reaction at room temperature for 4 hours. TLC shows the reaction completion. Quench the reaction with ice water, extract with ethyl acetate (2×10 mL), combine the organic phases, wash three times with brine, dry over anhydrous sodium sulfate, and evaporate the mixture under reduced pressure. Purify the crude product by preparative chromatography under a gradient program of solvent D:solvent E = 100:0 to 80:20 to obtain a light yellow oily substance (0.33 g, 82.0% yield).

### Step 2: Preparation of Compound C2

The compound was synthesized according to preparation procedure D, with intermediate D2-1 being linoleylamine, intermediate C2-2 being the same as C1-2, intermediate C2-3 being the same as C1-3, and intermediate C2-4 being (Z)-1-isocyanoctadec-9-ene. The crude product was purified by preparative HPLC under a gradient program of solvent A:solvent B:solvent C = 100:0:0 to 70:24:6, yielding a colorless oily compound (137.1 mg, 30% yield). MS(ESI): [M+H]+ = 798.37.

### Preparation of Compound C3

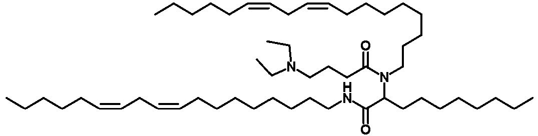

### Step 1: Preparation of Intermediate C3-4 (Same as Intermediate C2-4)

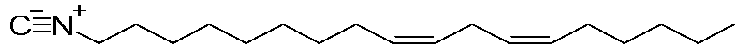

### Step 2: Preparation of Compound C3

The compound was synthesized according to preparation procedure D, with intermediate C3-1 being linoleylamine, intermediate C2-2 being the same as 1-2, intermediate C3-3 being 4-(diethylamino)butyric acid, and intermediate C3-4 being the same as C2-4. The crude product was purified by preparative HPLC under a gradient program of solvent A:solvent B:solvent C = 100:0:0 to 70:24:6, yielding a colorless oily compound (203.5 mg, 46% yield). MS(ESI): [M+H]+ = 826.42.

### Preparation of Compound C4

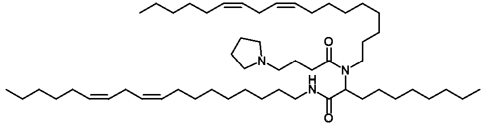

### Step 1: Preparation of Intermediate C4-4 (Same as Intermediate C2-4)

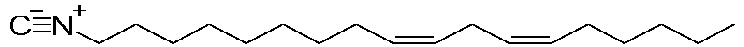

### Step 2: Preparation of Compound C4

The compound was synthesized according to preparation procedure C, with intermediate C4-1 being linoleylamine, intermediate C4-2 being the same as C1-2, intermediate C4-3 being pyrrolidine-1-butyric acid, and intermediate C4-4 being the same as C2-4. The crude product was purified by preparative HPLC under a gradient program of solvent A:solvent B:solvent C = 100:0:0 to 70:24:6, yielding a colorless oily compound (89.6 mg, 23% yield). MS(ESI): [M+H]+ = 824.41.

### Preparation of Compound C5

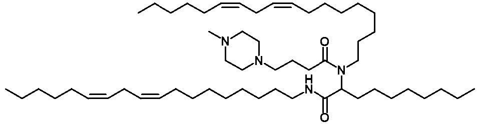

### Step 1: Preparation of Intermediate C5-4 (Same as Intermediate C2-4)

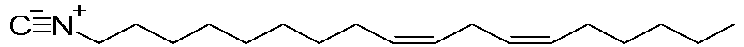

### Step 2: Preparation of Compound C5

The compound was synthesized according to preparation procedure C, with intermediate C5-1 being linoleylamine, intermediate C5-2 being the same as C1-2, intermediate C5-3 being 4-(4-methylpiperazin-1-yl)butyric acid, and intermediate C5-4 being the same as C2-4. The crude product was purified by preparative HPLC under a gradient program of solvent A:solvent B:solvent C = 100:0:0 to 70:24:6, yielding a colorless oily compound (198.5 mg, 38% yield). MS(ESI): [M+H]+ = 852.45.

### Preparation of Compound C6

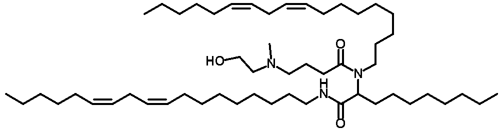

### Step 1: Preparation of Intermediate C6-4 (Same as Intermediate C2-4)

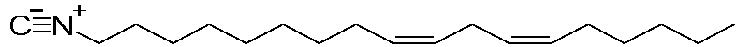

### Step 2: Preparation of Compound C6

The compound was synthesized according to preparation procedure C, with intermediate C6-1 being linoleylamine, intermediate C6-2 being the same as C1-2, intermediate C6-3 being 4-[2-hydroxyethyl(methyl)amino]butyric acid, and intermediate C6-4 being the same as C2-4. The crude product was purified by preparative HPLC under a gradient program of solvent A:solvent B:solvent C = 100:0:0 to 70:24:6, yielding a colorless oily compound (158.4 mg, 32% yield). MS(ESI): [M+H]+ = 828.39.

### Preparation of Compound C7

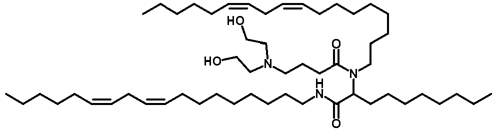

### Step 1: Preparation of Intermediate C7-4 (Same as Intermediate C2-4)

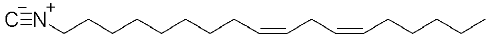

### Step 2: Preparation of Compound C7

The compound was synthesized according to preparation procedure C, with intermediate C7-1 being linoleylamine, intermediate C7-2 being the same as C1-2, intermediate C7-3 being N,N-dihydroxyethyl butyric acid, and intermediate C7-4 being the same as C2-4. The crude product was purified by preparative HPLC under a gradient program of solvent A:solvent B:solvent C = 100:0:0 to 70:24:6, yielding a colorless oily compound (216.8 mg, 48% yield). MS(ESI): [M+H]+ = 858.42.

### Preparation of Compound C8

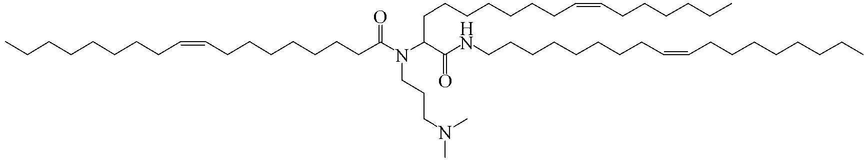

### Step 1: Preparation of Compound C8

The compound was synthesized according to preparation procedure C, with intermediate C8-1 being oleylamine, intermediate C8-2 being (Z)-7-hexadecenal, intermediate C8-3 being oleic acid, and intermediate C7-4 being the same as C1-4. The crude product was purified by preparative HPLC under a gradient program of solvent A:solvent B:solvent C = 100:0:0 to 70:24:6, yielding a colorless oily compound (158.4 mg, 32% yield). MS(ESI): [M+H]+ = 882.87.

### 6.4 Example

### Preparation of Type D Compounds

### Preparation of Compound D1

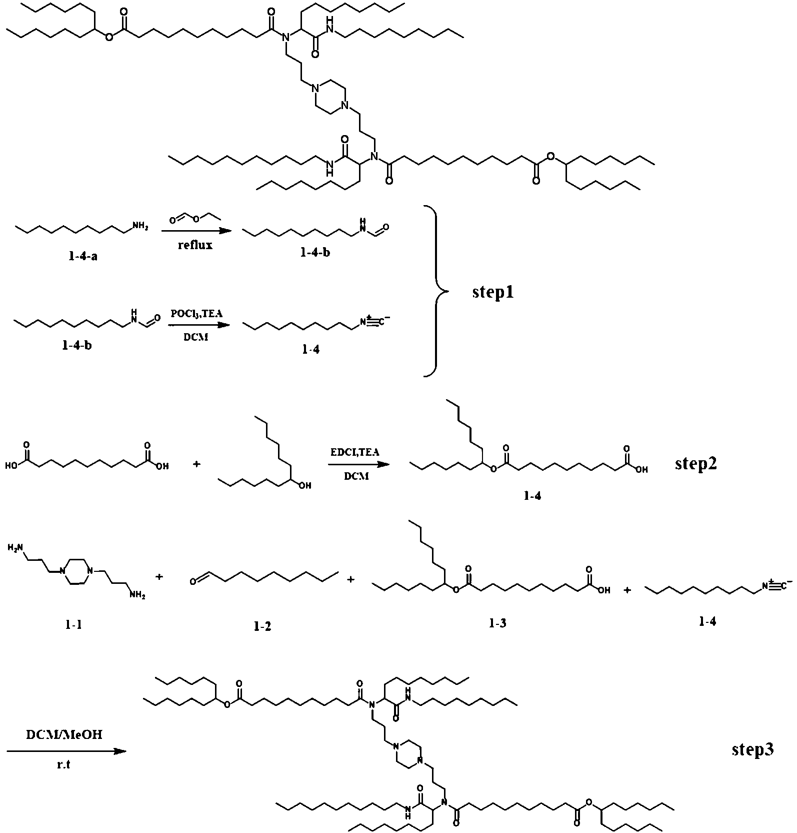

### Step 1: Preparation of Intermediate D1-3

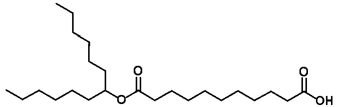

### Step 2: Preparation of Intermediate D1-4

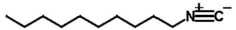

### Step 3: Preparation of Compound D1

At room temperature, dissolve compound D1-1 (1,4-bis(3-aminopropyl)piperazine) (100 mg, 0.5 mmol) in dichloromethane/methanol (VDCM:VMeOH = 3:1). Add compound D1-2 (nonanal) (142.01 mg, 1 mmol) and stir for 1 hour. Then, add compound D1-3 (397.97 mg, 1 mmol) and continue stirring for 0.5 hours. Add compound D1-4 (167.02 mg, 1 mmol) and stir the mixture at room temperature for 16 hours. TLC indicates the completion of the reaction. Evaporate the mixture under reduced pressure and purify via preparative HPLC using a gradient program of solvent A:solvent B:solvent C = 100:0:0 to 70:24:6 to obtain colorless oily compound D1 (156.8 mg, 22% yield). MS(ESI): [M+H]+ = 1595.66.
This preparation procedure is referred to as Standard Procedure D.

### Preparation of Compound D2

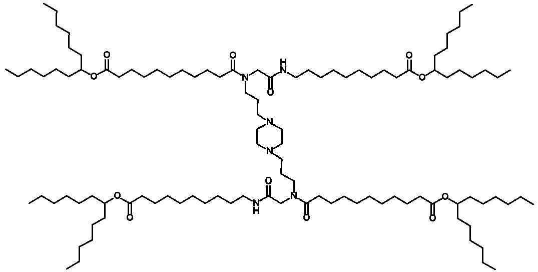

### Step 1: Preparation of Intermediate D2-3 (Same as Intermediate A1-3)

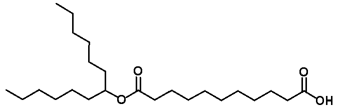

### Step 2: Preparation of Intermediate D2-4 (Same as Intermediate A1-4)

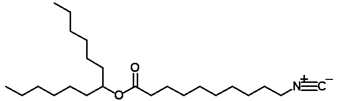

### Step 3: Preparation of Compound D2

The compound was synthesized according to preparation procedure D, with intermediate D2-1 being the same as D1-1 and intermediate D2-2 being formaldehyde. The crude product was purified by preparative HPLC under a gradient program of solvent A:solvent B:solvent C = 100:0:0 to 70:24:6, yielding a colorless oily compound (128.8 mg, 30% yield). MS(ESI): [M+H]+ = 1781.87.

### Preparation of Compound D3

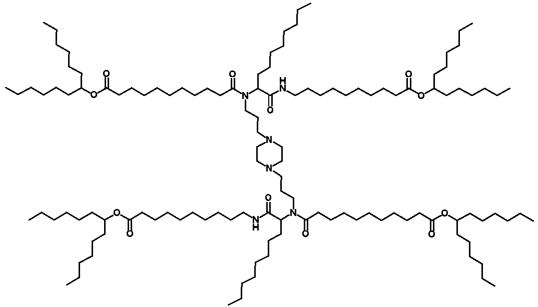

### Step 1: Preparation of Intermediate D3-3 (Same as Intermediate A1-3)

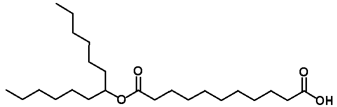

### Step 2: Preparation of Intermediate D3-4 (Same as Intermediate A1-4)

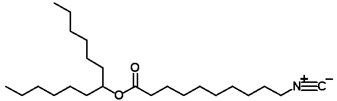

### Step 3: Preparation of Compound D3

The compound was synthesized according to preparation procedure D, with intermediate D3-1 being the same as E1-1 and intermediate D3-2 being the same as D1-2. The crude product was purified by preparative HPLC under a gradient program of solvent A:solvent B:solvent C = 100:0:0 to 70:24:6, yielding a colorless oily compound (201.9 mg, 45% yield). MS(ESI): [M+H]+ = 2006.30.

### Preparation of Compound D4

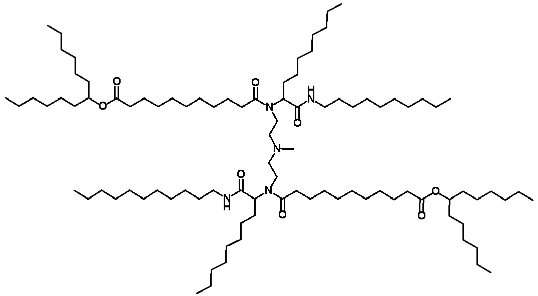

### Step 1: Preparation of Intermediate D4-3

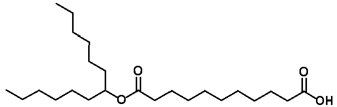

### Step 2: Preparation of Intermediate D4-4

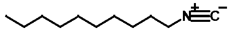

### Step 3: Preparation of Compound D4

The compound was synthesized according to preparation procedure D, with intermediate E4-1 being N-methyl-2,2'-diaminoethylamine and intermediate D4-2 being the same as D1-2. The crude product was purified by preparative HPLC under a gradient program of solvent A:solvent B:solvent C = 100:0:0 to 70:24:6, yielding a colorless oily compound (66.7 mg, 20% yield). MS(ESI): [M+H]+ = 1512.53.

### Preparation of Compound D5

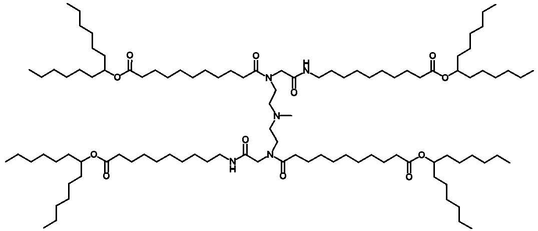

### Step 1: Preparation of Intermediate D5-3

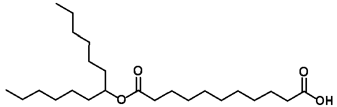

### Step 2: Preparation of Intermediate D5-4

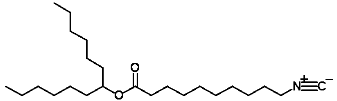

### Step 3: Preparation of Compound D5

The compound was synthesized according to preparation procedure D, with intermediate D5-1 being the same as D4-1 and intermediate D5-2 being formaldehyde. The crude product was purified by preparative HPLC under a gradient program of solvent A:solvent B:solvent C = 100:0:0 to 70:24:6, yielding a colorless oily compound (128.8 mg, 30% yield). MS(ESI): [M+H]+ = 1698.73.

### Preparation of Compound D6

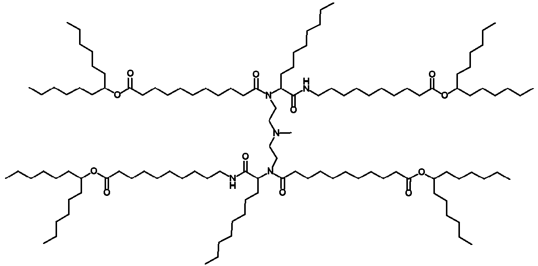

### Step 1: Preparation of Intermediate D6-3

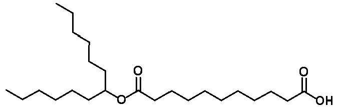

### Step 2: Preparation of Intermediate D6-4

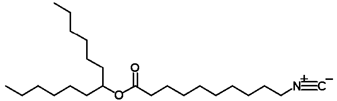

### Step 3: Preparation of Compound D6

The compound was synthesized according to preparation procedure D, with intermediate D6-1 being the same as D4-1 and intermediate D6-2 being nonanal. The crude product was purified by preparative HPLC under a gradient program of solvent A:solvent B:solvent C = 100:0:0 to 70:24:6, yielding a colorless oily compound (212.8 mg, 48% yield). MS(ESI): [M+H]+ = 1698.73.

### 6.5 Example

### Preparation of Type E Compounds

### Preparation of Compound E1

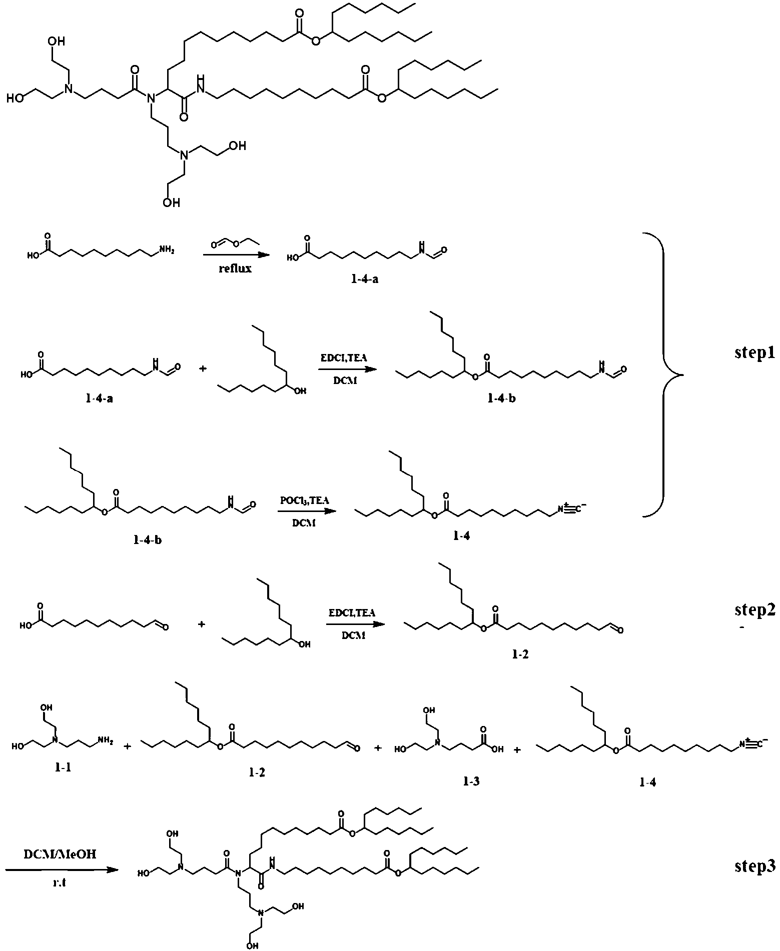

### Step 1: Preparation of Intermediate E1-2

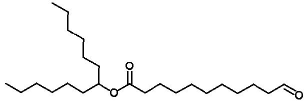

### Step 2: Preparation of Intermediate E1-4

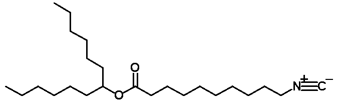

### Step 3: Preparation of Compound E1

At room temperature, dissolve compound E1-1 (N-(3-aminopropyl)diethanolamine) (100 mg, 0.616 mmol) in dichloromethane/methanol (VDCM:VMeOH = 3:1). Add intermediate E1-2 (235.85 mg, 0.616 mmol) and stir for 1 hour. Then, add compound E1-3 (N,N-dihydroxyethyl butyric acid) (117.87 mg, 0.616 mmol) and continue stirring for 0.5 hours. Add compound E1-4 (234 mg, 0.616 mmol) and stir the mixture at room temperature for 16 hours. TLC indicates the completion of the reaction. Evaporate the mixture under reduced pressure and purify via preparative HPLC using a gradient program of solvent A:solvent B:solvent C = 100:0:0 to 70:24:6 to obtain colorless oily compound E1 (205.7 mg, 48% yield). MS(ESI): [M+H]+ = 1098.0.

This preparation procedure is referred to as Standard Procedure E.

### Preparation of Compound E2

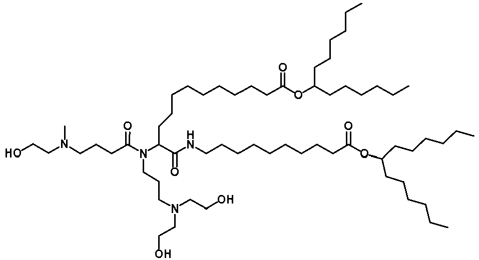

### Step 1: Preparation of Intermediate E2-2 (Same as Intermediate E1-2)

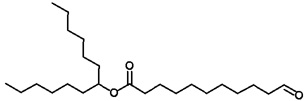

### Step 2: Preparation of Intermediate E2-4 (Same as Intermediate A1-4)

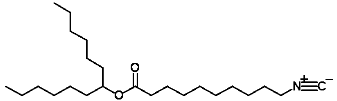

### Step 3: Preparation of Compound E2

The compound was synthesized according to preparation procedure E, with intermediate E2-1 being the same as F1-1 and intermediate E2-3 being 4-[2-hydroxyethyl(methyl)amino]butyric acid. The crude product was purified by preparative HPLC under a gradient program of solvent A:solvent B:solvent C = 100:0:0 to 70:24:6, yielding a colorless oily compound (132.2 mg, 31% yield). MS(ESI): [M+H]+ = 1068.68.

### Preparation of Compound E3

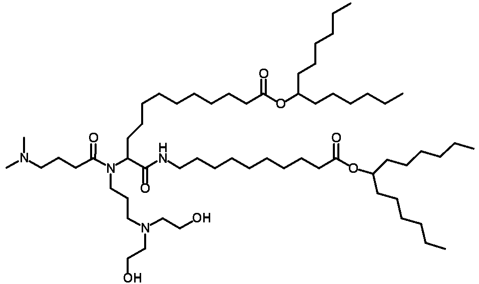

### Step 1: Preparation of Intermediate E3-2 (Same as Intermediate E1-2)

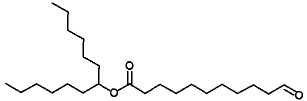

### Step 2: Preparation of Intermediate E3-4 (Same as Intermediate A1-4)

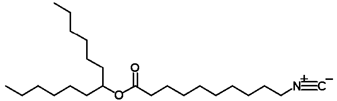

### Step 3: Preparation of Compound E3

The compound was synthesized according to preparation procedure F, with intermediate E3-1 being the same as E1-1 and intermediate E3-3 being 4-dimethylaminobutyric acid. The crude product was purified by preparative HPLC under a gradient program of solvent A:solvent B:solvent C = 100:0:0 to 70:24:6, yielding a colorless oily compound (107.8 mg, 29% yield). MS(ESI): [M+H]+ = 1038.65.

### Preparation of Compound E4

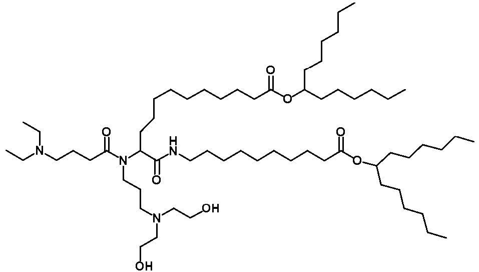

### Step 1: Preparation of Intermediate E4-2 (Same as Intermediate E1-2)

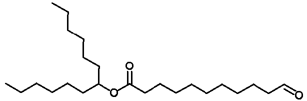

### Step 2: Preparation of Intermediate E4-4 (Same as Intermediate A1-4)

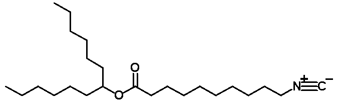

### Step 3: Preparation of Compound E4

The compound was synthesized according to preparation procedure E, with intermediate E4-1 being the same as E1-1 and intermediate E4-3 being 4-diethylaminobutyric acid. The crude product was purified by preparative HPLC under a gradient program of solvent A:solvent B:solvent C = 100:0:0 to 70:24:6, yielding a colorless oily compound (167.1 mg, 35% yield). MS(ESI): [M+H]+ = 1066.71.

### Preparation of Compound E5

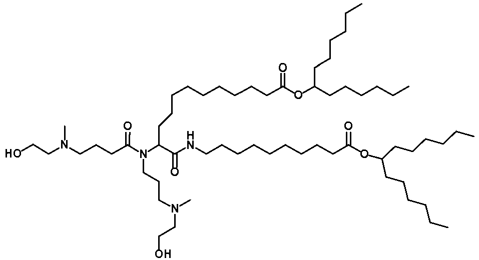

### Step 1: Preparation of Intermediate E5-2 (Same as Intermediate E1-2)

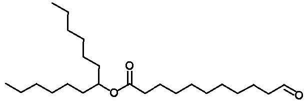

### Step 2: Preparation of Intermediate E5-4 (Same as Intermediate E1-4)

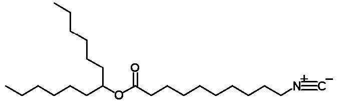

### Step 3: Preparation of Compound E5

The compound was synthesized according to preparation procedure E, with intermediate E5-1 being N-(2-hydroxyethyl)-N-methyl-1,3-diaminopropane and intermediate E5-3 being 4-[2-hydroxyethyl(methyl)amino]butyric acid. The crude product was purified by preparative HPLC under a gradient program of solvent A:solvent B:solvent C = 100:0:0 to 70:24:6, yielding a colorless oily compound (97.5 mg, 24% yield). MS(ESI): [M+H]+ = 1038.65.

### Preparation of Compound E6

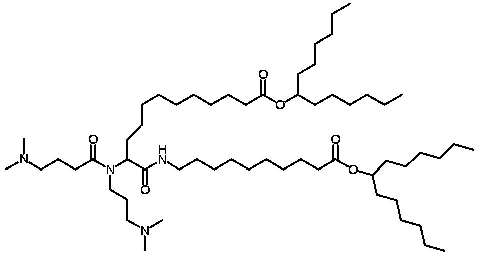

### Step 1: Preparation of Intermediate E6-2 (Same as Intermediate E1-2)

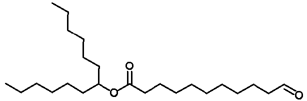

### Step 2: Preparation of Intermediate E6-4 (Same as Intermediate A1-4)

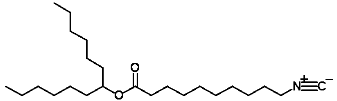

### Step 3: Preparation of Compound E6

The compound was synthesized according to preparation procedure E, with intermediate E6-1 being N,N-dimethyl-1,3-diaminopropane and intermediate E6-3 being 4-dimethylaminobutyric acid. The crude product was purified by preparative HPLC under a gradient program of solvent A:solvent B:solvent C = 100:0:0 to 70:24:6, yielding a colorless oily compound (208.2 mg, 47% yield). MS(ESI): [M+H]+ = 978.60.

### Preparation of Compound E7

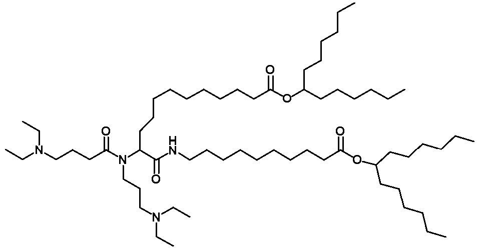

### Step 1: Preparation of Intermediate E7-2

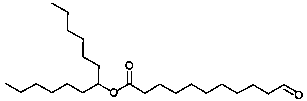

### Step 2: Preparation of Intermediate E7-4

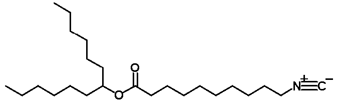

### Step 3: Preparation of Compound E7

The compound was synthesized according to preparation procedure E, with intermediate E7-1 being 3-diethylaminopropylamine and intermediate E7-3 being 4-diethylaminobutyric acid. The crude product was purified by preparative HPLC under a gradient program of solvent A:solvent B:solvent C = 100:0:0 to 70:24:6, yielding a colorless oily compound (58.6 mg, 20% yield). MS(ESI): [M+H]+ = 1034.71.

### Preparation of Compound E8

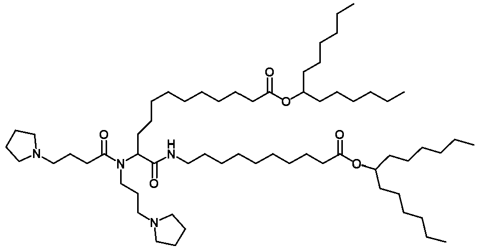

### Step 1: Preparation of Intermediate E8-2 (Same as Intermediate F1-2)

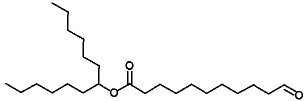

### Step 2: Preparation of Intermediate E8-4 (Same as Intermediate A1-4)

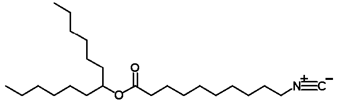

### Step 3: Preparation of Compound E8

The compound was synthesized according to preparation procedure E, with intermediate E8-1 being 1-(3-aminopropyl)pyrrolidine and intermediate E8-3 being pyrrolidine-1-butyric acid. The crude product was purified by preparative HPLC under a gradient program of solvent A:solvent B:solvent C = 100:0:0 to 70:24:6, yielding a colorless oily compound (76.6 mg, 22% yield). MS(ESI): [M+H]+ = 1030.68.

### Preparation of Compound E9

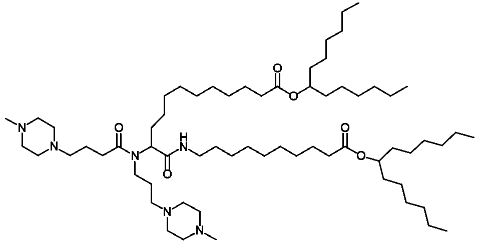

### Step 1: Preparation of Intermediate E9-2 (Same as Intermediate E1-2)

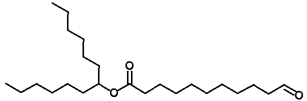

### Step 2: Preparation of Intermediate E9-4 (Same as Intermediate A1-4)

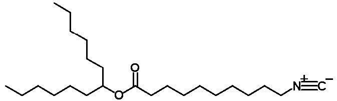

### Step 3: Preparation of Compound E9

The compound was synthesized according to preparation procedure E, with intermediate E9-1 being 1-(3-aminopropyl)-4-methylpiperazine and intermediate E9-3 being 4-(4-methylpiperazin-1-yl)butyric acid. The crude product was purified by preparative HPLC under a gradient program of solvent A:solvent B:solvent C = 100:0:0 to 70:24:6, yielding a colorless oily compound E9 (37.4 mg, 18% yield). MS(ESI): [M+H]+ = 1088.76.

### Preparation of Compound E10

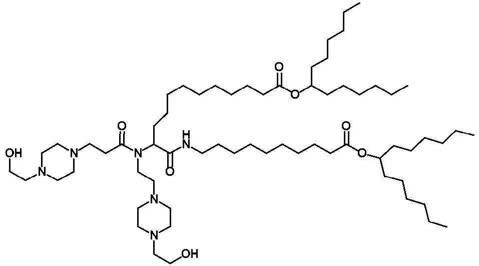

### Step 1: Preparation of Intermediate E10-2 (Same as Intermediate E1-2)

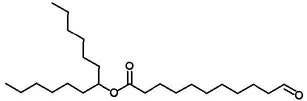

### Step 2: Preparation of Intermediate E10-4 (Same as Intermediate A1-2)

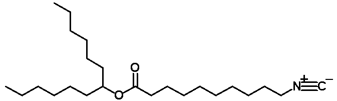

### Step 3: Preparation of Compound E10

The compound was synthesized according to preparation procedure E, with intermediate E10-1 being 2-(4-(2-aminoethyl)piperazin-1-yl)ethanol and intermediate E10-3 being 3-[4-(2-hydroxyethyl)piperazin-1-yl]propionic acid. The crude product was purified by preparative HPLC under a gradient program of solvent A:solvent B:solvent C = 100:0:0 to 70:24:6, yielding a colorless oily compound F10 (181.6 mg, 33% yield). MS(ESI): [M+H]+ = 1120.76.

### Preparation of Compound E11

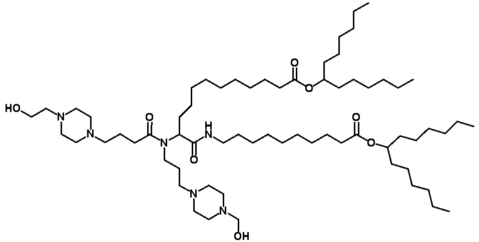

### Step 1: Preparation of Intermediate E11-2 (Same as Intermediate E1-2)

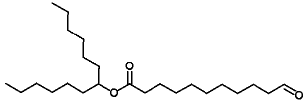

### Step 2: Preparation of Intermediate E11-4 (Same as Intermediate A1-4)

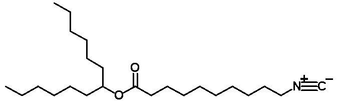

### Step 3: Preparation of Compound E11

The compound was synthesized according to preparation procedure E, with intermediate E11-1 being 4-[4-(2-hydroxyethyl)piperazin-1-yl]butyric acid and intermediate E11-3 being 4-(3-aminopropyl)piperazin-1-yl-methanol. The crude product was purified by preparative HPLC under a gradient program of solvent A:solvent B:solvent C = 100:0:0 to 70:24:6, yielding a colorless oily compound F11 (216.6 mg, 49% yield). MS(ESI): [M+H]+ = 1134.78.

### 6.6 Example: Preparation and Characterization of Lipid Nanoparticles

In summary, the cationic lipid described herein, DSPC, cholesterol, and PEG-lipid were dissolved in ethanol at a molar ratio of 50:10:38.5:1.5 and diluted in 10 mM to 50 mM citrate buffer at pH 4 with mRNA. Using a microfluidic device, the lipid ethanol solution was mixed with mRNA aqueous solution at a volume ratio of 1:3 and a total flow rate ranging from 6-30 mL/min, preparing LNPs with a total lipid to mRNA weight ratio of approximately 10:1 to 30:1. Ethanol was removed by dialysis and replaced with PBS. Finally, the lipid nanoparticles were filtered through a 0.22 µm sterile filter.

The size of lipid nanoparticles was measured by dynamic light scattering using a Malvern Zetasizer Nano ZS (Malvern, UK) with a 90° scattering detection mode. The encapsulation efficiency of lipid nanoparticles was determined using the Quant-it Ribogreen RNA Quantitation Kit (Thermo Fisher Scientific, UK) according to the manufacturer's instructions. The apparent pKa of LNP formulations correlates with the delivery efficiency of nucleic acids by LNPs in vivo. The apparent pKa ranges from approximately 5 to 7. An analysis based on fluorescence from 2-(p-tolylamino)-6-naphthalenesulfonic acid (TNS) was used to determine the apparent pKa of formulations. As described above, LNP formulations containing cationic lipid/DSPC/cholesterol/DMG-PEG (50/10/38.5/1.5 mol%) in PBS were prepared. A 300 µM stock solution of TNS in distilled water was prepared. LNP formulations were diluted to 0.1 mg/mL total lipid in 3 mL of buffer solution containing 50 mM sodium citrate, 50 mM sodium phosphate, 50 mM sodium borate, and 30 mM sodium chloride (pH ranged from 3 to 9). Aliquots of TNS solution were added to achieve a final concentration of 0.1 mg/mL, and after vortex mixing, fluorescence intensity was measured at room temperature using a Molecular Devices Spectramax iD3 spectrophotometer with an excitation wavelength of 325 nm and an emission wavelength of 435 nm. S-shaped curve best-fit analysis was applied to the fluorescence data, and the pKa value was measured as the pH value producing half-maximal fluorescence intensity.

### 6.7 Example: Animal Studies

The efficacy of nanoparticle compositions was evaluated using bioluminescent studies. A dose of 0.25 mg/kg (mpk) of the formulation was administered to mice (n=3) via intramuscular injection, and bioluminescence was measured at a 6-hour time point. The test lipid nanoparticles from the MC3 formulation were evaluated, and in some cases, controls (e.g., PBS controls) were assessed for comparison.

The characteristics of the test lipid nanoparticles measured from the test groups, including F-luc luminescence intensity, are listed in the table below.

**Table 2: Properties of Nanoparticles Formed by Compounds**

| **Serial Number** | **Size (nm)** | **PDI** | **Encapsulation efficiency (%)** | **Relative Total flux** | **Zeta potential (mV)** |
|---|---|---|---|---|---|
| A1 | 82.96 | 0.135 | 67.41 | B | -12.25 |
| A2 | 78.3 | 0.153 | 50.77 | C | -2.69 |
| A3 | 81.09 | 0.202 | 95.23 | C | -2.04 |
| A4 | 72.85 | 0.202 | 97.34 | D | 5.65 |
| A5 | 60.62 | 0.13 | 92.48 | D | -17.73 |
| A6 | 55.75 | 0.234 | 84.80 | A | -9.54 |
| A7 | 62.53 | 0.274 | 96.10 | C | 4.99 |
| A8 | 66.98 | 0.16 | 92.88 | D | -19.03 |
| A9 | 64.58 | 0.191 | 93.54 | D | -18.67 |
| A10 | 61.16 | 0.415 | 54.08 | A | -3.45 |
| A11 | 225.6 | 0.075 | 71.39 | D | -2.89 |
| A12 | 63.48 | 0.207 | 24.84 | C | -9.55 |
| A13 | 118.5 | 0.157 | 94.62 | B | 2.90 |
| A14 | 89.33 | 0.228 | 94.31 | C | 4.02 |
| A15 | 113.2 | 0.251 | 93.95 | A | 4.75 |
| A16 | 78.42 | 0.183 | 41.55 | C | 0.78 |
| A17 | 60.77 | 0.07 | 53.24 | D | -0.68 |
| A18 | 78.14 | 0.136 | 87.17 | D | -15.20 |
| A19 | 78.64 | 0.093 | 89.13 | D | -0.20 |
| A20 | 93.07 | 0.113 | 60.65 | D | 17.70 |
| A21 | 95.73 | 0.071 | 54.53 | C | 11.03 |
| A22 | 84.36 | 0.176 | 91.06 | C | 11.60 |
| A23 | 81.59 | 0.113 | 94.29 | C | 12.80 |
| A24 | 95.52 | 0.192 | 9.33 | C | 7.89 |
| A25 | 92.75 | 0.224 | 93.66 | D | 11.31 |
| A26 | 94.21 | 0.11 | 94.09 | A | 2.30 |
| A27 | 121.37 | 0.11 | 95.33 | A | -3.43 |
| A28 | 88.05 | 0.15 | 85.93 | A | -0.41 |
| A29 | 94.71 | 0.09 | 94.69 | A | -1.24 |
| A30 | 86.87 | 0.12 | 96.65 | B | 0.47 |
| A31 | 79.94 | 0.22 | 97.50 | B | -4.31 |
| A32 | 85.33 | 0.13 | 95.33 | B | 1.89 |
| A33 | 79.84 | 0.10 | 94.69 | A | 0.51 |
| A34 | 94.21 | 0.11 | 95.23 | A | 2.08 |
| A35 | 74.92 | 0.19 | 97.34 | A | -7.81 |
| A36 | 79.52 | 0.17 | 92.48 | A | -1.82 |
| A37 | 85.32 | 0.14 | 84.80 | B | -5.90 |
| A38 | 80.57 | 0.15 | 96.10 | A | 2.32 |
| A39 | 78.16 | 0.10 | 92.88 | A | -3.35 |
| A40 | 75.94 | 0.17 | 93.54 | C | -0.97 |
| A41 | 264.60 | 0.66 | 54.08 | C | -10.28 |
| A42 | 90.78 | 0.19 | 95.23 | A | -1.88 |
| A43 | 105.07 | 0.16 | 71.39 | A | -3.98 |
| A44 | 102.07 | 0.20 | 24.84 | B | -3.58 |
| A45 | 79.00 | 0.06 | 94.62 | A | 0.83 |
| A46 | 107.50 | 0.19 | 94.31 | A | -0.55 |
| A47 | 91.63 | 0.11 | 95.93 | A | -0.55 |
| A48 | 64.27 | 0.08 | 94.62 | A | 0.65 |
| B1 | 91.81 | 0.114 | 94.25 | A | -1.26 |
| B2 | 104.5 | 0.15 | 85.90 | A | -6.41 |
| B3 | 85.75 | 0.306 | 79.83 | A | -6.91 |
| B4 | 72.08 | 0.159 | 96.14 | A | 10.58 |
| B5 | 55.64 | 0.163 | 95.00 | A | 6.08 |
| B6 | 61.81 | 0.183 | 75.47 | A | -0.39 |
| B7 | 58.86 | 0.116 | 85.36 | A | -2.80 |
| B8 | 60.47 | 0.152 | 91.38 | A | -7.30 |
| B9 | 92.84 | 0.143 | 91.00 | A | 0.32 |
| B10 | 66.9 | 0.166 | 46.91 | A | 2.18 |
| B11 | 70.27 | 0.205 | 81.83 | A | -9.69 |
| B12 | 65.03 | 0.183 | 93.45 | A | -9.02 |
| B13 | 79.06 | 0.246 | 91.93 | A | 0.30 |
| B14 | 76.67 | 0.13 | 89.01 | A | 2.73 |
| B15 | 87.64 | 0.182 | 95.93 | A | 2.43 |
| B16 | 57.19 | 0.593 | 94.62 | A | 5.42 |
| B17 | 64.2 | 0.185 | 92.99 | A | 14.20 |
| B18 | 80.68 | 0.187 | 81.99 | A | 4.79 |
| B19 | 104.1 | 0.149 | 94.63 | A | -5.68 |
| B20 | 93.23 | 0.391 | 93.73 | A | -2.36 |
| B21 | 84.45 | 0.388 | 93.97 | B | 6.22 |
| B22 | 141.8 | 0.165 | 95.03 | B | -3.48 |
| B23 | 68.16 | 0.214 | 93.95 | B | 12.50 |
| B24 | 68.83 | 0.245 | 93.57 | A | -9.96 |
| B25 | 64.2 | 0.095 | 69.52 | A | -3.38 |
| B26 | 105.63 | 0.22 | 92.99 | D | -1.65 |
| B27 | 70.04 | 0.21 | 81.99 | B | 1.50 |
| B28 | 59.38 | 0.25 | 94.63 | A | -0.77 |
| B29 | 62.10 | 0.10 | 93.73 | A | 1.69 |
| B30 | 64.27 | 0.08 | 93.97 | A | 3.81 |
| B31 | 61.00 | 0.10 | 95.03 | A | 4.83 |
| B32 | 119.93 | 0.32 | 93.95 | A | 7.87 |
| B33 | 72.74 | 0.21 | 93.57 | C | -2.58 |
| B34 | 85.75 | 0.15 | 96.22 | C | -10.28 |
| B35 | 72.08 | 0.31 | 95.37 | A | -3.98 |
| B36 | 55.64 | 0.16 | 93.88 | A | -3.58 |
| B37 | 61.81 | 0.16 | 94.77 | B | 0.83 |
| B38 | 58.86 | 0.18 | 75.84 | B | -0.55 |
| B39 | 60.47 | 0.12 | 92.29 | A | -0.55 |
| B40 | 92.82 | 0.15 | 96.22 | A | 0.65 |
| B41 | 66.90 | 0.14 | 95.37 | A | -1.65 |
| B42 | 70.27 | 0.17 | 94.38 | A | 1.50 |
| C1 | 60.62 | 0.108 | 82.31 | B | -9.90 |
| C2 | 55.75 | 0.5 | 93.88 | B | 7.20 |
| C3 | 62.53 | 0.041 | 98.57 | C | -9.90 |
| C4 | 66.98 | 0.259 | 96.34 | B | 14.67 |
| C5 | 64.58 | 0.198 | 91.26 | D | -14.75 |
| C6 | 61.16 | 0.442 | 90.65 | D | -10.22 |
| C7 | 225.6 | 0.23 | 88.48 | D | -5.62 |
| C8 | 78.44 | 0.24 | 82.17 | A | -0.77 |
| D1 | 63.48 | 0.173 | 95.31 | C | -7.18 |
| D2 | 118.5 | 0.195 | 91.46 | C | -10.34 |
| D3 | 78.76 | 0.159 | 89.56 | C | -2.05 |
| D4 | 113.2 | 0.163 | 43.24 | C | -5.91 |
| D5 | 78.42 | 0.183 | 96.72 | A | -17.80 |
| D6 | 60.77 | 0.116 | 92.04 | A | -12.28 |
| E1 | 78.14 | 0.152 | 90.04 | C | -6.75 |
| E2 | 78.64 | 0.143 | 91.65 | C | -9.45 |
| E3 | 93.07 | 0.166 | 89.28 | C | -2.06 |
| E4 | 95.73 | 0.205 | 95.18 | A | 5.03 |
| E5 | 84.36 | 0.183 | 93.90 | B | -19.40 |
| E6 | 81.59 | 0.246 | 96.54 | C | -6.48 |
| E7 | 95.52 | 0.13 | 92.11 | C | -16.13 |
| E8 | 92.75 | 0.182 | 96.30 | B | -18.58 |
| E9 | 91.81 | 0.593 | 82.13 | C | -15.91 |
| E10 | 104.5 | 0.185 | 69.71 | C | -16.71 |
| E11 | 85.75 | 0.158 | 93.77 | B | 0.85 |
| DLin-MC3 | 87.75 | 0.036 | 95.24 | B | -3.25 |
| SM-102 | 89.08 | 0.058 | 96.25 | A | -1.23 |
| ALC-0315 | 70.12 | 0.082 | 93.27 | A | -0.76 |

| | | | | | |
|---|---|---|---|---|---|
| **Note:** A, B, C, and D represent the normalized results of the compound's luminescence flux compared to MC3, with the following scaling ranges: **A:** ≥2 **B:** ≥1 and <2 **C:** ≥0.1 and <1 **D:** <0.1. | | | | | |

### Example 6.8 TEM Structural Characterization of LNPs

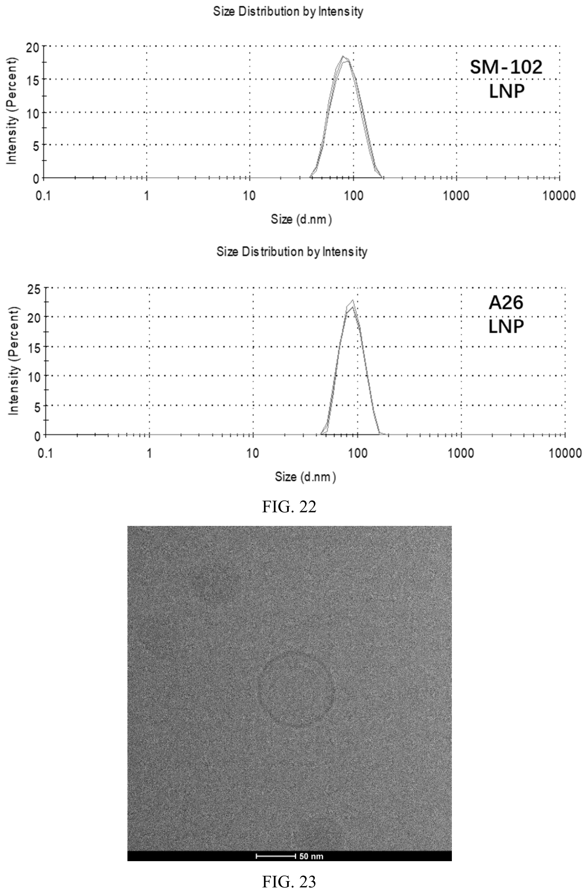

Freshly prepared mRNA-LNPs were concentrated to a total lipid concentration of 20-25 mg/mL and applied to glow-discharged copper grids (3-5 µL). Rapid freezing was performed using an FEI Mark IV Vitrobot (FEI, Hillsboro, OR, USA) to form vitreous ice. The grids were transferred into a Gatan 70° cryo-transfer system pre-equilibrated to -180°C, followed by imaging. All samples, unless otherwise specified, were imaged at 55,000× magnification with a nominal underfocus of 1-2 µm to enhance contrast. As shown in Figure 22, dynamic light scattering measurements from the aforementioned Example 6.6 indicate that the particle sizes of SM102 and A26 lipids are approximately 100 nm. TEM analysis, as depicted in Figure 23, also shows particle sizes of around 100 nm.

### Example 6.9 High-Throughput Screening of Compounds In Vitro

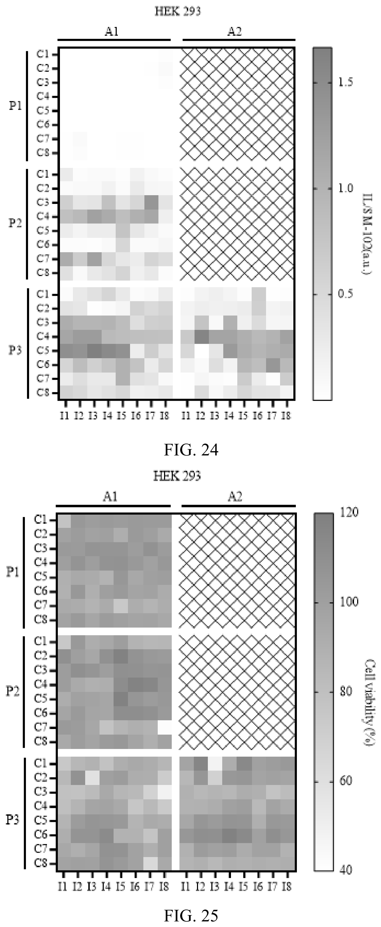

Lipids were synthesized via the Ugi reaction in 96-well plates. After a 6-hour reaction, ethanol solutions of the lipids were directly combined with acidic aqueous solutions of mRNA encoding F-Luc to form LNPs. For in vitro transfection, LNPs were added to HEK293 cells pre-plated in a 100 ng/3×10⁴ cell format. After overnight incubation, the transfection efficiency of F-Luc was measured using the One-Glo Luciferase Assay System (Promega) following the manufacturer's instructions. Luminescence was quantified using a PerkinElmer microplate reader, enabling rapid assessment of transfection efficiency for a batch of compounds.The specific compounds evaluated are listed in the table below. As shown in Figure 24, lipid nanoparticles can deliver mRNA into 293T cells for expression of the target fluorescent protein. Some compounds achieved luminescence flux levels comparable to SM102-LNP.

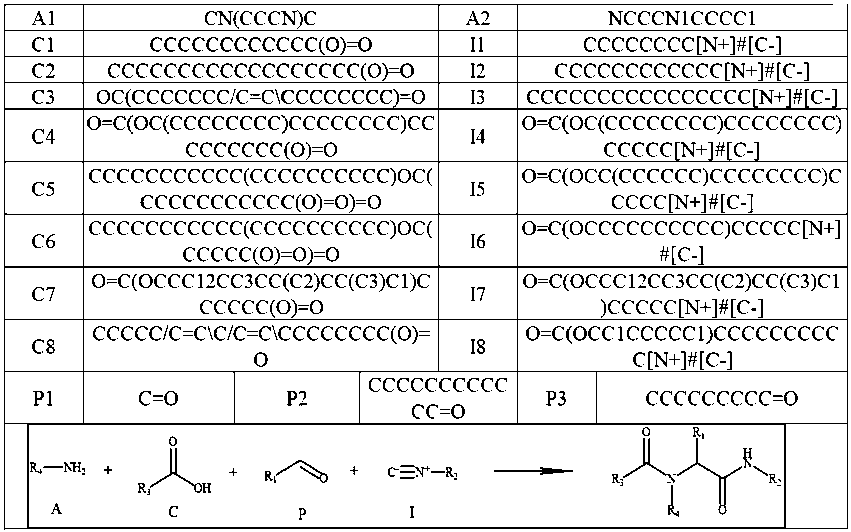

### Example 6.10 High-Throughput In Vitro Cytotoxicity Assay

Viability testing was conducted following the same preparation and transfection steps as described above. Cell viability was assessed using the One-Glo-Tox reagent (Promega) according to the manufacturer's instructions. Absorbance values were quantified using a PerkinElmer microplate reader to rapidly evaluate the cytotoxicity of a batch of compounds. As shown in Figure 25, the viability of cells exposed to lipid nanoparticles was largely maintained above 90%. This indicates that the lower transfection efficiency observed for some compounds in Example 6.9 was not due to cell death. Additionally, it demonstrates that most of the synthesized compounds exhibit low cytotoxicity.

### Example 6.12 In Vivo Imaging via Intramuscular Injection

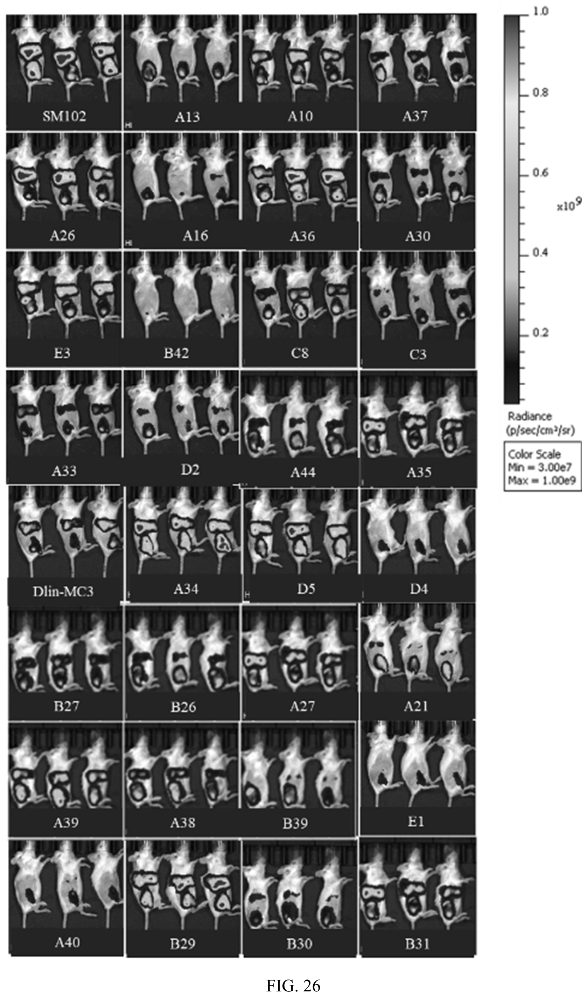

Formulations containing compounds according to formula (I-1) or (I-2) were administered via intramuscular injection at a dose of 0.25 mg/kg. Female SPF-grade BALB/c mice aged 6-8 weeks (n=3) were housed in SPF-grade animal facilities with controlled temperature (20-26°C) and humidity (40-70%), and provided with adequate food and water. A commercial lipid group was included as a control for in vivo comparison. Six hours post-dose, 200 µL of 15 mg/mL D-luciferin potassium salt was administered intraperitoneally, followed by in vivo imaging 5 minutes later. As shown in Figure 26, compounds A26, A27, A34, A35, A39, B31, B29, and D5 achieved Radiance levels of 5×10⁸-1×10⁸ p/sec/cm²/sr, matching commercial lipid standards.

### Example 6.13 In Vivo Organ Imaging via Tail Vein Injection

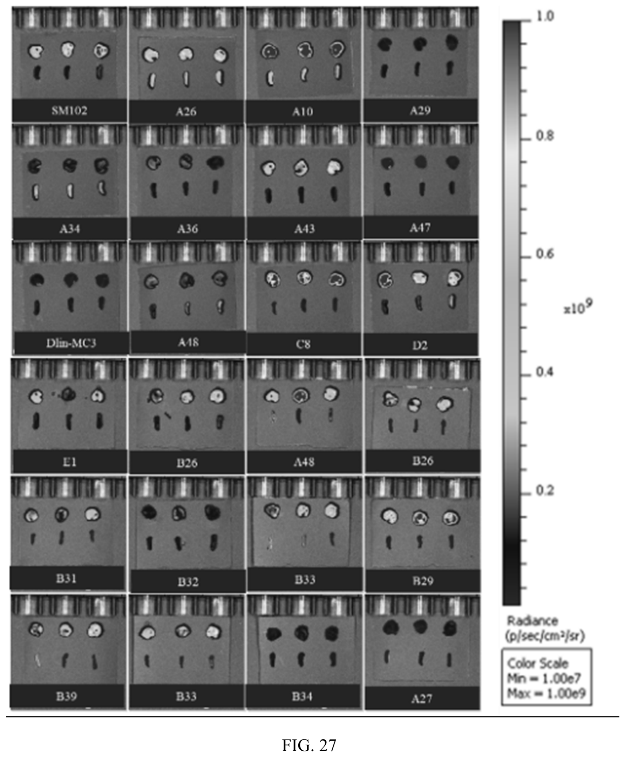

Formulations containing compounds according to formula (I-1) or (I-2) were administered via tail vein injection at a dose of 0.25 mg/kg. Female SPF-grade BALB/c mice aged 6-8 weeks (n=3) were housed under the same conditions as above. SM-102 and MC3-LNP were used as controls. Six hours post-dose, 200 µL of 15 mg/mL D-luciferin potassium salt was administered intraperitoneally, followed by in vivo imaging 5 minutes later. After all in vivo imaging was completed, D-luciferin potassium salt was re-administered, and organ imaging was performed 5 minutes later. As shown in Figure 27, compounds A26, A27, A34, A35, A39, B31, B29, and D5 demonstrated liver expression levels of 8×10⁸-1×10⁸ p/sec/cm²/sr (Radiance), matching commercial lipid standards. Some lipids exhibited higher spleen targeting than commercial lipids. For instance, compounds A10, A26, A34, A48, B34, D2 showed spleen expression levels of 6×10⁸-8×10⁸ p/sec/cm²/sr (Radiance), far exceeding the commercial lipid level of 1×10⁸ p/sec/cm²/sr (Radiance).

### Example 6.14 In Vivo Imaging with Optimized Formulations of Different Compounds

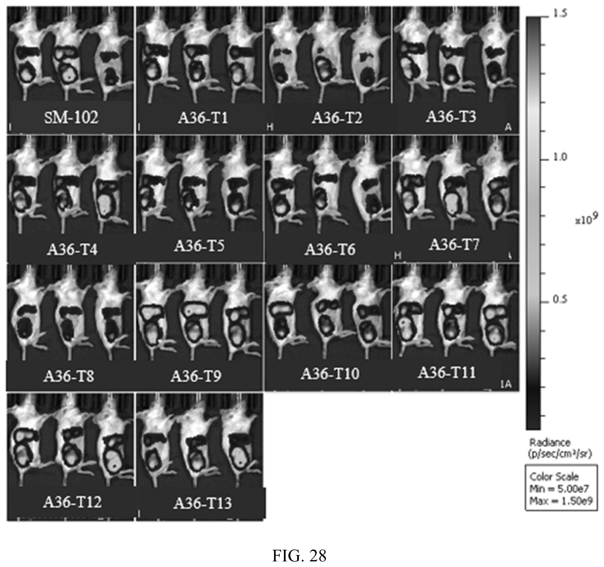

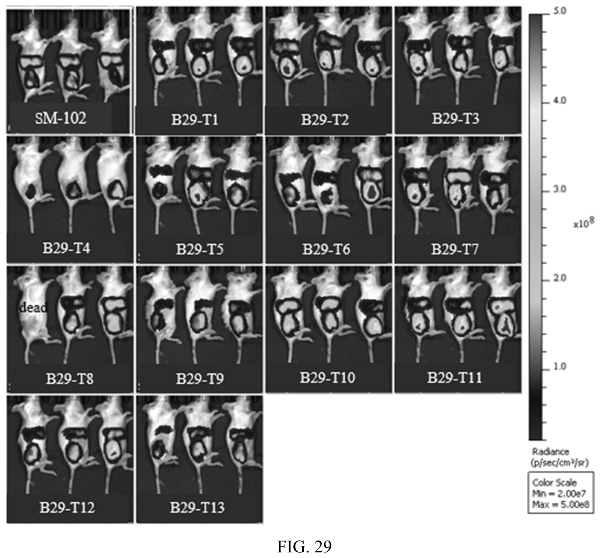

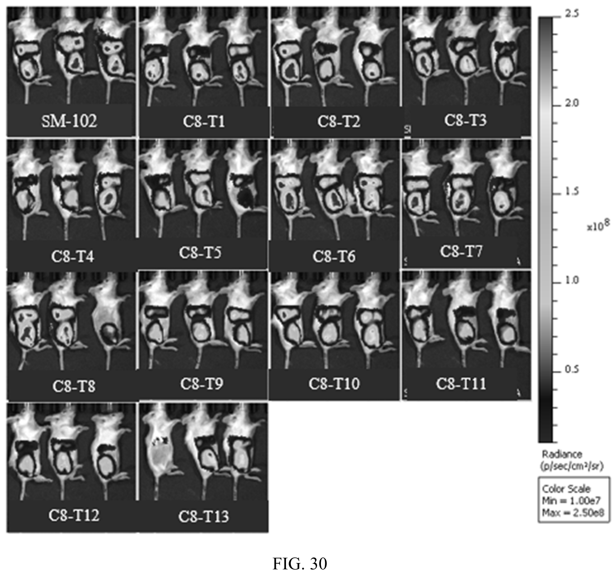

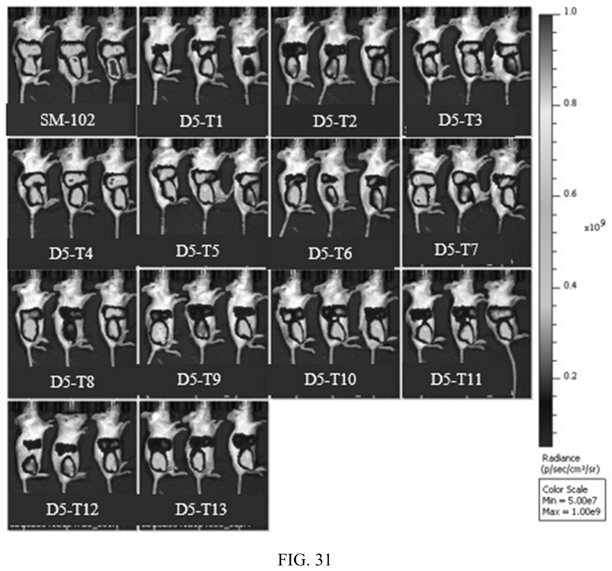

The relative amount of phospholipid in the lipid component of nanoparticles was adjusted to further optimize the formulation. Compounds according to formula (I-1) or (I-2) were selected for use in nanoparticles with DSPC as the phospholipid. Other phospholipids could also be evaluated. Nanoparticle formulations were prepared with phospholipid content varying between 0 mol% and 30 mol%. Parameters such as size, encapsulation efficiency, Luc or hEPO expression levels, and cytokine distribution were evaluated. Figures 28, 29, 30, 31, and 32 show that optimized formulations significantly enhanced expression compared to commercial lipid ratios. For example, A36-T1 showed an expression level of only 3-5×10⁸ p/sec/cm²/sr (Radiance), while optimized A36-T9 achieved 1-1.5×10⁹ p/sec/cm²/sr (Radiance). Similar improvements were observed in other optimized lipids like B29, with varying optimization preferences. Some lipids exhibited higher expression at lower phospholipid content, while others showed more stable physicochemical parameters and expression effects at higher cholesterol content.

| **Serial Number** | IL | | DSPC | Chol | PEG-DMG |
|---|---|---|---|---|---|
| SM-102 | SM-102 | 0.5 | 0.1 | 0.385 | 0.015 |
| A36-T1 | A36 | 0.5 | 0.1 | 0.385 | 0.015 |
| A36-T2 | A36 | 0.314 | 0.171 | 0.498 | 0.016 |
| A36-T3 | A36 | 0.328 | 0.019 | 0.631 | 0.022 |
| A36-T4 | A36 | 0.173 | 0.086 | 0.716 | 0.025 |
| A36-T5 | A36 | 0.494 | 0.071 | 0.418 | 0.016 |
| A36-T6 | A36 | 0.343 | 0 | 0.633 | 0.024 |
| A36-T7 | A36 | 0.3 | 0.022 | 0.666 | 0.018 |
| A36-T8 | A36 | 0.303 | 0.182 | 0.492 | 0.023 |
| A36-T9 | A36 | 0.347 | 0.2 | 0.439 | 0.014 |
| A36-T10 | A36 | 0.492 | 0.093 | 0.403 | 0.012 |
| A36-T11 | A36 | 0.388 | 0.034 | 0.56 | 0.018 |
| A36-T12 | A36 | 0.469 | 0.188 | 0.324 | 0.019 |
| A36-T13 | A36 | 0.477 | 0.045 | 0.456 | 0.022 |
| B29-T1 | B29 | 0.5 | 0.1 | 0.385 | 0.015 |
| B29-T2 | B29 | 0.505 | 0.057 | 0.414 | 0.024 |
| B29-T3 | B29 | 0.514 | 0.122 | 0.348 | 0.016 |
| B29-T4 | B29 | 0.529 | 0.198 | 0.255 | 0.018 |
| B29-T5 | B29 | 0.55 | 0.246 | 0.182 | 0.022 |
| B29-T6 | B29 | 0.584 | 0.109 | 0.283 | 0.023 |
| B29-T7 | B29 | 0.591 | 0.294 | 0.098 | 0.017 |
| B29-T8 | B29 | 0.592 | 0.069 | 0.332 | 0.007 |
| B29-T9 | B29 | 0.598 | 0.16 | 0.23 | 0.012 |
| B29-T10 | B29 | 0.415 | 0.14 | 0.431 | 0.014 |
| B29-T11 | B29 | 0.384 | 0.124 | 0.47 | 0.022 |
| B29-T12 | B29 | 0.39 | 0.077 | 0.516 | 0.017 |
| B29-T13 | B29 | 0.447 | 0.131 | 0.397 | 0.025 |
| C8-T1 | C8 | 0.5 | 0.1 | 0.385 | 0.015 |
| C8-T2 | C8 | 0.337 | 0.216 | 0.436 | 0.011 |
| C8-T3 | C8 | 0.223 | 0.238 | 0.518 | 0.021 |
| C8-T4 | C8 | 0.278 | 0.285 | 0.418 | 0.019 |
| C8-T5 | C8 | 0.326 | 0.102 | 0.553 | 0.019 |
| C8-T6 | C8 | 0.269 | 0.176 | 0.542 | 0.013 |
| C8-T7 | C8 | 0.236 | 0.135 | 0.605 | 0.024 |
| C8-T8 | C8 | 0.387 | 0.128 | 0.468 | 0.017 |
| C8-T9 | C8 | 0.305 | 0.193 | 0.479 | 0.023 |
| C8-T10 | C8 | 0.202 | 0.3 | 0.484 | 0.014 |
| C8-T11 | C8 | 0.407 | 0.158 | 0.41 | 0.025 |
| C8-T12 | C8 | 0.479 | 0.105 | 0.403 | 0.013 |
| C8-T13 | C8 | 0.208 | 0.113 | 0.667 | 0.012 |
| D5-T1 | D5 | 0.5 | 0.1 | 0.385 | 0.015 |
| D5-T2 | D5 | 0.494 | 0.077 | 0.418 | 0.011 |
| D5-T3 | D5 | 0.393 | 0.166 | 0.418 | 0.023 |
| D5-T4 | D5 | 0.3 | 0.099 | 0.584 | 0.017 |
| D5-T5 | D5 | 0.372 | 0.153 | 0.457 | 0.018 |
| D5-T6 | D5 | 0.454 | 0.128 | 0.398 | 0.02 |
| D5-T7 | D5 | 0.307 | 0.148 | 0.534 | 0.011 |
| D5-T8 | D5 | 0.405 | 0.114 | 0.469 | 0.012 |
| D5-T9 | D5 | 0.319 | 0.176 | 0.48 | 0.025 |
| D5-T10 | D5 | 0.359 | 0.092 | 0.527 | 0.022 |
| D5-T11 | D5 | 0.417 | 0.178 | 0.39 | 0.015 |
| D5-T12 | D5 | 0.485 | 0.09 | 0.411 | 0.014 |
| D5-T13 | D5 | 0.434 | 0.104 | 0.437 | 0.025 |
| E4-T1 | E4 | 0.5 | 0.1 | 0.385 | 0.015 |
| E4-T2 | E4 | 0.494 | 0.077 | 0.418 | 0.011 |
| E4-T3 | E4 | 0.473 | 0.081 | 0.422 | 0.023 |
| E4-T4 | E4 | 0.373 | 0.165 | 0.443 | 0.019 |
| E4-T5 | E4 | 0.331 | 0.243 | 0.404 | 0.021 |
| E4-T6 | E4 | 0.213 | 0.179 | 0.595 | 0.013 |
| E4-T7 | E4 | 0.444 | 0.142 | 0.401 | 0.013 |
| E4-T8 | E4 | 0.295 | 0.185 | 0.494 | 0.025 |
| E4-T9 | E4 | 0.411 | 0.098 | 0.475 | 0.016 |
| E4-T10 | E4 | 0.278 | 0.151 | 0.555 | 0.016 |
| E4-T11 | E4 | 0.307 | 0.087 | 0.586 | 0.02 |
| E4-T12 | E4 | 0.256 | 0.248 | 0.485 | 0.011 |
| E4-T13 | E4 | 0.348 | 0.12 | 0.52 | 0.012 |

### Example 6.15 Dose-Response Study of Different Compounds

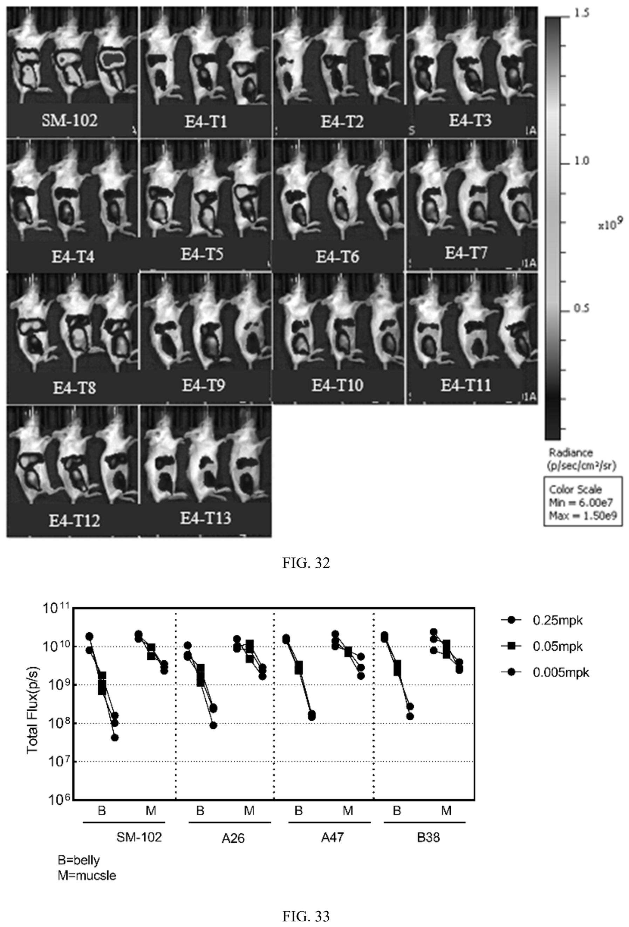

Samples containing firefly luciferase (Luc) mRNA were administered intramuscularly to evaluate protein expression. Formulations of compounds according to formula (I-1) or (I-2) were administered at different mRNA doses (0.25 mg/kg, 0.05 mg/kg, and 0.01 mg/kg). As shown in Figure 33, compounds A26, A47, and B38 demonstrated a clear dose-response gradient. The abdominal region (liver and spleen areas) showed more pronounced dose effects compared to the injection site. As the dose decreased, the injection site's expression proportion increased. A26, A47, and B38 maintained injection site flux levels of 0.3-3×10¹⁰ (p/s) across all doses.

### Example 6.16 In Vivo Distribution Study of Different Compounds

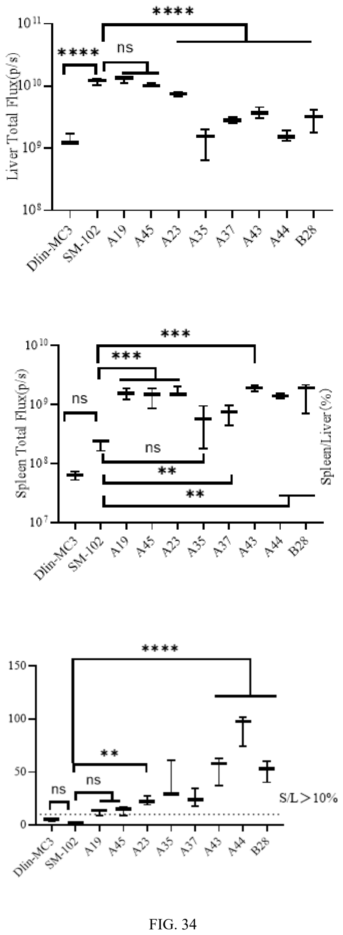

Formulations of compounds according to formula (I-1) or (I-2) were administered via tail vein injection at a dose of 0.25 mg/kg. Female SPF-grade BALB/c mice aged 6-8 weeks (n=3) were housed under standard conditions. SM-102 and MC3-LNP served as controls. Six hours post-dose, 200 µL of 15 mg/mL D-luciferin potassium salt was administered intraperitoneally, followed by in vivo imaging 5 minutes later. After all in vivo imaging, D-luciferin potassium salt was re-administered, and organ imaging was performed 5 minutes later. As shown in Figure 34, compound A19 exhibited liver expression levels of 1×1-2×10¹⁰ p/s, matching commercial lipids, and spleen expression levels of 1×1-2×10⁹ p/s, surpassing commercial lipids. Compound A44 showed liver and spleen expression levels of 1×1-2×10⁹ p/s, with lower liver expression than commercial lipids but higher spleen expression and a higher spleen-liver ratio, beneficial for diseases requiring spleen protein expression.

### Example 6.17 hEPO-mRNA Quantitative Expression Assay In Vivo

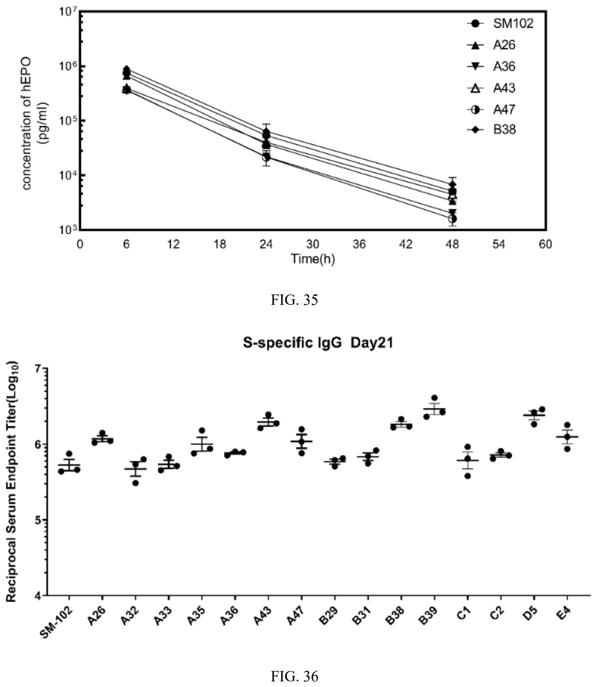

Formulations containing compounds according to formula (I-1) or (I-2) (encapsulating human erythropoietin (hEPO) mRNA) were administered via tail vein injection at a dose of 0.25 mg/kg to 6-8-week-old female BALB/c mice (n=3). Serum samples were collected at 6, 24, and 48 hours post-dose and analyzed for hEPO expression using an hEPO ELISA kit (Beyotime). Results in Figure 35 show that selected compounds from Series A and B demonstrated robust protein expression, with potential as protein replacement therapy carriers. At 6 hours, A26 and B38 showed protein expression levels of 0.8-1×10⁶ pg/mL.

### Example 6.18 Quantitative Detection of S-Protein-Binding Antibodies

Formulations of compounds according to formula (I-1) or (I-2) (encapsulating COVID-19 Spike-Protein mRNA) were administered intramuscularly at 0.05 mg/kg to 6-8-week-old female BALB/c mice (n=3). Mice were randomly divided into groups and vaccinated twice on days 0 and 14. Seven days after the booster dose, mouse serum was collected and analyzed for specific IgG antibodies via indirect ELISA. Results in Figure 36 show that compounds from Series A-E induced strong humoral immune responses, with groups A26, A35, A43, A47, B38, B39, C1, C2, D5, E4, etc., achieving antibody levels above 10⁶, exceeding commercial lipid levels.

### Example 6.19 Quantitative Detection of S-Protein Neutralizing Antibodies

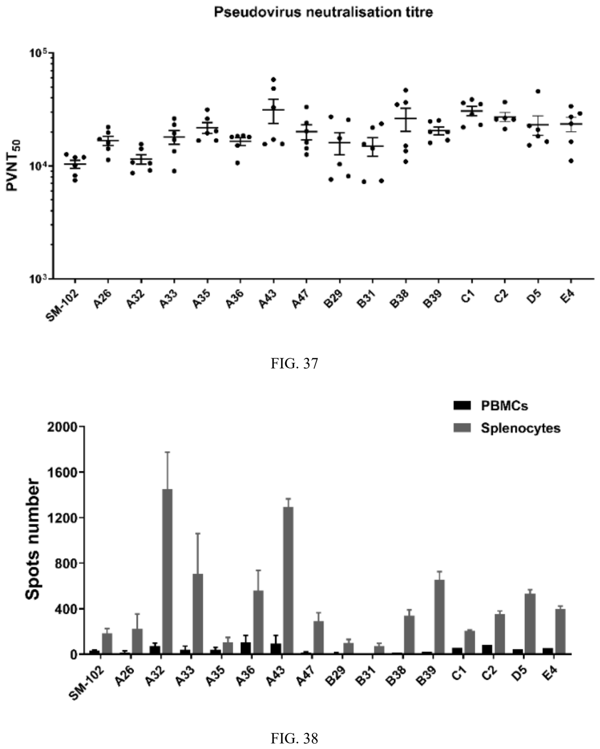

Mice were dosed and immunized as in Example 6.18. Seven days after the booster dose, mouse serum was collected and analyzed for S-protein-specific neutralizing antibodies via pseudovirus neutralization assays. Results in Figure 37 show that compounds from Series A-E induced strong humoral immune responses, with groups A26, A35, A43, A47, B38, B39, C1, C2, D5, E4, etc., achieving neutralizing antibody titers above 10⁴, surpassing commercial lipids. Data from Figures 36 and 37 indicate a strong correlation between binding and neutralizing antibodies.

### Example 6.20 ELISPOT Immune Spot Assay

Mice were dosed and immunized as in Example 6.18. At the study endpoint, mice were euthanized, and spleens and peripheral blood were collected. Splenocytes and peripheral blood immune cells were diluted to 2×10⁵ cells/mL in complete medium with 10% fetal bovine serum and added to 96-well ELISPOT plates pre-coated with anti-mouse IFN-γ antibody. Overlapping peptides (10 µg/mL) were added, and plates were incubated at 37°C for 36 hours. After washing, biotinylated anti-mouse IFN-γ antibody (1 µg/mL) was added and incubated for 2 hours. Streptavidin-HRP was added and incubated for 1 hour, followed by TMB substrate development. Spots were counted using an ELISPOT analyzer, and spot-forming cells per 1×10⁶ cells were calculated. Results in Figure 38 show that compounds from Series A-E induced strong cellular immune responses, with several formulations inducing higher splenocyte immune responses than commercial lipids.

### Example 6.21 Safety Study of Different Compounds

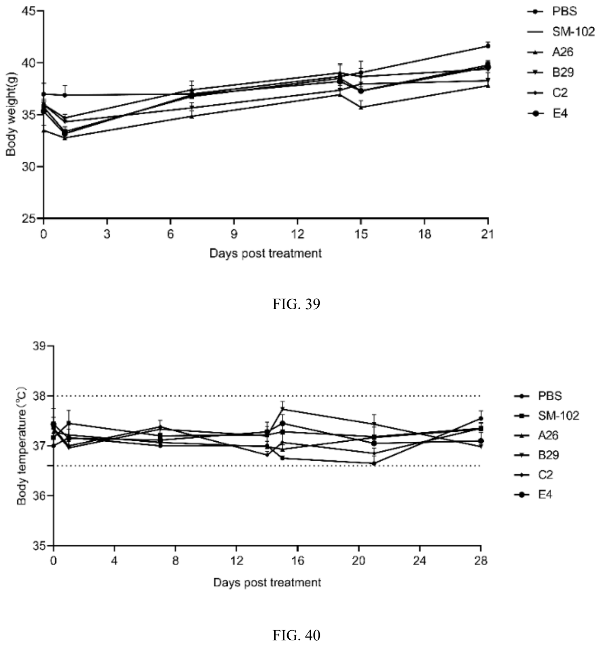

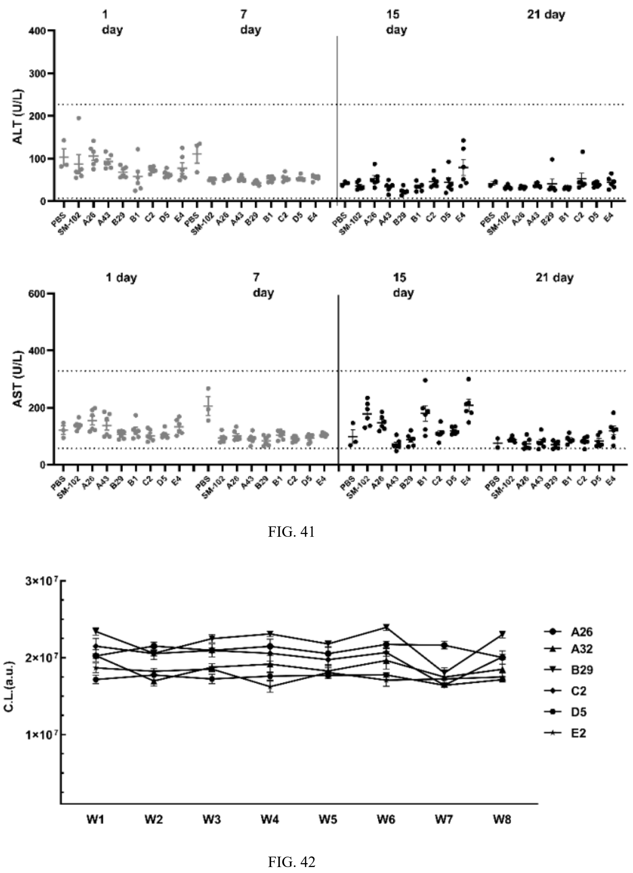

Formulations of compounds according to formula (I-1) or (I-2) were administered intramuscularly at 2.5 mg/kg to 6-8-week-old female SPF-grade BALB/c mice. Mice were observed for body weight and temperature at 0d, 1d, 7d, 14d, 15d, 21d, and 28d. SM-102 served as a positive control. Figures 39 and 40 show that all dosed groups maintained normal weight and temperature fluctuations within acceptable ranges compared to the PBS 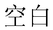 group. Additionally, ALT and AST levels were measured at 1d, 7d, 15d, and 21d post-dose. Figure 41 shows that ALT and AST levels remained within normal ranges seven days post-dose, with no acute elevation within 24 hours post-injection, indicating good safety profiles for the compounds.

Example 6.23 Stability Study of Different Compound Formulations

Formulations of compounds according to formula (I-1) or (I-2) were stored at 4°C for stability assessment. Transfection conditions were the same as in Example 6.9. Stability results after eight weeks (Figure 42) show no significant decline in F-Luc protein expression, indicating good stability and high transfection efficiency comparable to commercial products like lipo3000 (2×10⁷ transfection level in 293T cells).

### Example 6.24 siRNA Delivery Efficiency of Different Compounds

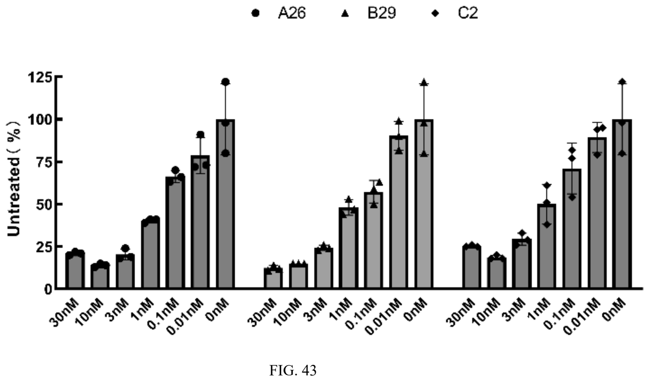

293T-Luc stably transfected cells were seeded in 96-well white plates at 3×10⁴ cells per well. Formulations of compounds according to formula (I-1) or (I-2) (encapsulating siRNA-FLuc) were added at different concentrations. After incubation, cells were processed, and Bright-Lite detection was used to calculate cell viability and Luc firefly luciferase expression efficiency. Results in Figure 43 show that all compounds demonstrated robust mRNA knockdown efficacy with IC50 values around 1 nM, indicating that the compounds can also deliver siRNA effectively.

While the embodiments of the invention have been illustrated and described above, it should be understood that the above embodiments are exemplary and are not to be construed as limiting the present invention. Those of ordinary skill in the art may make variations, modifications, and substitutions to the above embodiments within the scope of the present invention

## Claims

1. A compound, **characterized in that** it comprises at least one structural formula (I-1): 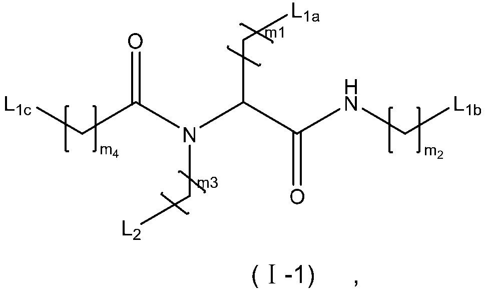
or the compound is a pharmaceutically acceptable salt, prodrug, or isomer thereof;
wherein,
m1, m2, m3, and m4 are each independently selected from integers between 1 and 18, inclusive;
L₁ₐ, L_{1b}, and L_{1c} are each independently selected from the group consisting of: -H, -S-SR1, -SR1, -C(=O)OR1, -OC(=O)R1, -N(R2)R1-, -C(=O)N(R2)R1-, -N(R2)C(=O)R1-, -C(=O)NR1, -OR1;
R1 and R2 are each independently selected from the group consisting of: -H, alicyclic hydrocarbons, 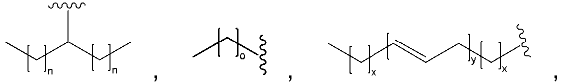 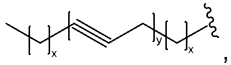 wherein n, x, and o are each independently selected from integers of 0 to 12,y is independently selected from 1, 2, 3, or 4;
L2 is independently selected from the group consisting of: -R3XR4Y, -R3Y, -R3YR5, -Y, -R3XR4(Z)Y;
R3, R4, and R5 are each independently selected from the group consisting of: C1-C5 alkane and C2-C5 alkene;
X is independently selected from the group consisting of: -O-, -N(Rx)- , -S-, -S-S-, -OC(=O)- , -C(=O)O-, -OP(=O)O-, -OS(=O)O-, -C(=O)N-, -N(C(=O))-;
Rₓ is independently selected from the group consisting of: -H, -(CH2)m-OH, -(CH2)m-CN, -(CH2)m, and alicyclic hydrocarbons, wherein m is independently selected from 0, 1, 2, 3, 4, or 5;
Y is independently selected from the group consisting of: -(CH2)m-OH, -(CH2)m-CN, -(CH2)m-N(R**)R*, -(CH2)m-G-(CH2)n, wherein m and n are each independently selected from 0, 1, 2, 3, 4, or 5;
G is independently selected from aryl or heteroaryl;
R* and R** are each independently selected from the group consisting of H, C1-C3 alkyl, -(CH2)mQ, wherein m is independently selected from 1, 2, 3, or 4;
Q is independently selected from the group consisting of: -OH, -CN;
Z is independently selected from the group consisting of: -(CH2)m-OH, -(CH2)m-CN, -(CH2)m-N(R**)R*, -(CH2)m-G-(CH2)n, wherein m and n are each independently selected from 0, 1, 2, 3, 4, or 5;
G is independently selected from aryl or heteroaryl;
R* and R** are each independently selected from the group consisting of: H, C1-C3 alkyl, -(CH2)ₐQ, wherein a is independently selected from 1, 2, 3, or 4;
Q is independently selected from the group consisting of: -OH, -CN;
R is independently selected from the group consisting of: -(CH2)m-, -N-, -S-, -C(=O)O-, -OC(=O)- , -N((CH2)m)- , -C(=O)N-, -O-, wherein m is independently selected from 1, 2, 3, or 4.

2. The compound of claim 1, wherein the structures L₁ₐ, L_{1b}, and L_{1c} are each interchangeably or replaceably connectable with L₂, and/or the structure L₂ is interchangeably or replaceably connectable with L₁ₐ, L_{1b}, or L_{1c}; and wherein the number of L₂ groups in each structural formula is at least one and no more than two.

3. The compound of claim 1 or 2, wherein Rₓ is alicyclic, and Rₓ is alicyclic having at least one of the structures (II-1), (II-2), (II-3), and (II-4), the structures (II-1), (II-2), (II-3), and (II-4) are as follows: 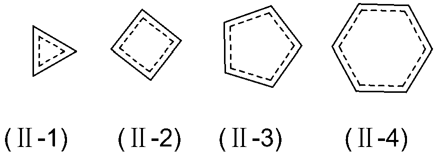
or Rx is a pharmaceutically acceptable salt, prodrug, or isomer of the structural formula (II-1), (II-2), (II-3) or (II-4);
wherein, the"------"bond in the structural formula (II-1), (II-2), (II-3) and (II -4) is a single or double bond.

4. The compound of claim 1 or 2, wherein R1 is alicyclic, and R1 is a vitamin E succinate, adamantane or its derivative; R2 is alicyclic;
R2 is a vitamin E succinate, adamantane or its derivative.

5. The compound of claim 1 or 2, wherein G is a heterocycle, and G is a nitrogen-containing heterocycle, oxygen-containing heterocycle or sulfur-containing heterocycle compound.

6. The compound of claim 1 or 2, wherein the parent structure of the compound is prepared by the Ugi reaction (i.e., Ugi reaction) from amine, carboxylic acid, aldehyde and isonitrile.

7. A preparation method of the compound of any of claims 1-6, specifically comprises following steps:
dissolving a first compound in a reaction device containing a first solvent, adding a second substance to the reaction device, after stirring for 0.5-2h, adding a third substance, continuing stirring for 0.5-2h, adding a fourth substance, continuing stirring for 4-48h, detecting the completion of the reaction, terminating the reaction, evaporating the organic solvent in the reaction device under reduced pressure, and then purifying to obtain the compound; the first compound is an amine;
the first solvent is ethanol or dichloromethane/methanol, the second substance is an aldehyde, the third substance is a carboxylic acid, and the fourth substance is an isonitrile.

8. A composition, comprising a steroid, a structural lipid, a polymer-bound lipid and the compound of any of claims 1-6.

9. The composition of claim 8, wherein the steroid is a steroid of formula III-1 structure:

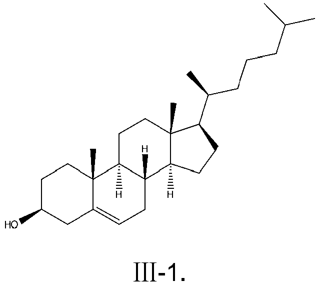

10. The composition of claim 8 or 9, wherein the molar ratio of the compound to the steroid is in the range of 1:2 to 6:1.

11. The composition of claim 8, wherein the polymer-bound lipid comprises one or more of the following: polyinosinic acid, DSPE-PCB20, DMG-PEG2000, DMPE-PEG2000, or C14-PEG2000.

12. The composition of claim 8 or 11, wherein the molar ratio of the compound to the polymer-bound lipid is in the range of 10:1 to 100:1.

13. The composition of claim 8, wherein the structural lipid includes at least one selected from the group consisting of formula III-2, III-3, and III-4:

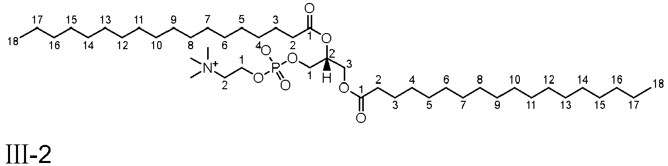

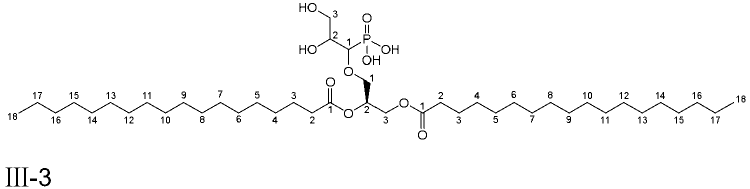

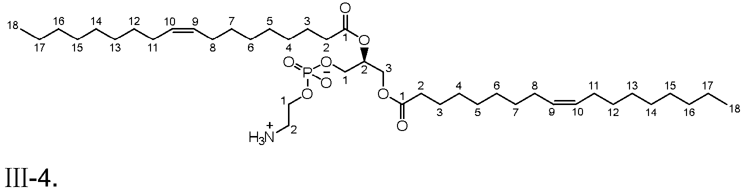

14. The composition of claim 8 or 13, wherein the molar ratio of the compound to the structural lipid is in the range of 2:1 to 10:1.

15. The composition of claim 8, wherein the composition further comprises a therapeutic or prophylactic agent; the therapeutic or prophylactic agent is a nucleic acid-based drug or a nucleic acid-like drug.

16. The composition of claim 15, wherein the nucleic acid-based drug includes aptamers, circular RNA, ASO, siRNA, sgRNA, tRNA, or mRNA; the mRNA comprises at least one mRNA encoding an antigen or its fragment or epitope; the mRNA is a monocistronic mRNA or a bicistronic mRNA; the antigen is a pathogenic antigen or a tumor-related antigen.

17. The composition of claim 15, wherein the nucleic acid-like drug comprises Locked Nucleic Acid (LNA), Peptide Nucleic Acid (PNA), or Morpholino.

18. The composition of claim 15 or 16, wherein the mRNA comprises one or more functional nucleotide analogs; the functional nucleotide analogs are selected from one or more of the following: pseudouridine, 1-methyl-pseudouridine, and 5-methylcytidine.

19. A lipid nanoparticle, comprising the compound of any of claims 1-6 or the composition of any of claims 8-18.

20. A pharmaceutical composition, comprising the compound of any of claims 1-6, the composition of any of claims 8-18, or the lipid nanoparticle of claim 19, and a pharmaceutically acceptable excipient or diluent.

21. A compound, comprising at least one structural formula (I-1): 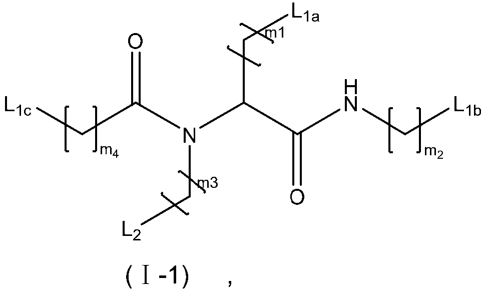
or the compound is a pharmaceutically acceptable salt, prodrug, or isomer thereof;
wherein,
m1, m2, m3, and m4 are each independently selected from integers between 1 and 18, inclusive;
L1a, L1b, and L1c are each independently selected from the group consisting of: -H, -S-SR1, -SR1, -C(=O)OR1, -OC(=O)R1, -N(R2)R1, -C(=O)N(R2)R1, -N(R2)C(=O)R1, -C(=O)NR1, and -OR1;
R1 and R2 are each independently selected from the group consisting of: -H , alicyclic hydrocarbons, 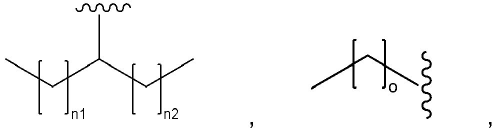 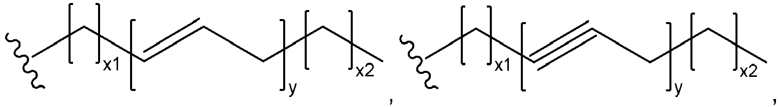 wherein n1, n2, x1, x2, and o are each independently selected from integers of 0 to 12, and y is independently selected from 1, 2, 3, or 4;
L2 is independently selected from the group consisting of: -R3XR4M, -R3XR4Y-H, -R3M, -R3YR5, -M, -R3XR4(Z)M;
R3, R4, and R5 are each independently selected from the group consisting of: C1-C5 alkane and C2-C5 alkene;
X is independently selected from the group consisting of: -O-, -N(Rx)-, -S-, -S-S-, -OC(=O)-, -C(=O)O-, -OP(=O)O-, -OS(=O)O-, -C(=O)N-, -N(C(=O))-;
Rx is independently selected from the group consisting of: -H, -(CH2)m-OH, -(CH2)m-CN, -(CH2)mH, and alicyclic hydrocarbons, wherein m is independently selected from 0, 1, 2, 3, 4, or 5;
M is independently selected from the group consisting of: -(CH2)m-OH, -(CH2)m-CN, -(CH2)m-N(R**)R*, wherein m is independently selected from 0, 1, 2, 3, 4, or 5;
Y is independently selected from the group consisting of: -(CH2)m-G-(CH2)n-,
wherein m and n are each independently selected from 0, 1, 2, 3, 4, or 5;
G is independently selected from aryl or heteroaryl;
R* and R** are each independently selected from the group consisting of: H, C1-C3 alkyl, and -(CH2)mQ, wherein m is independently selected from 1, 2, 3, or 4;
Q is independently selected from the group consisting of: -OH , -CN, 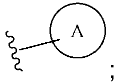 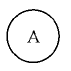 is independently selected from the group consisting of: 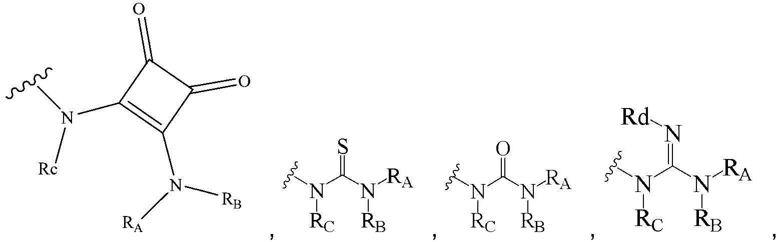 aryl or heterocyclic compounds;
RA, RB, and RC are each independently selected from the group consisting of: -H, -(CH2)m-H; m is independently selected from 1, 2, and 3;
RD is independently selected from the group consisting of: -H, -(CH2)n-H, -CN, -NO2, -NH2, -N(CH3)CH3; n is independently selected from 1, 2, and 3;
Z is independently selected from the group consisting of: -(CH2)m-OH, -(CH2)m-CN, -(CH2)m-N(R**)R*, -(CH2)m-G-(CH2)n-H, wherein m and n are each independently selected from 0, 1, 2, 3, 4, or 5;
G is independently selected from aryl or heteroaryl;
R* and R** are each independently selected from the group consisting of: H, C1-C3 alkyl, -(CH2)aQ, wherein a is independently selected from 1, 2, 3, or 4;
Q is independently selected from the group consisting of: -OH, -CN, 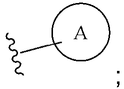 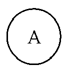 is independently selected from the group consisting of: 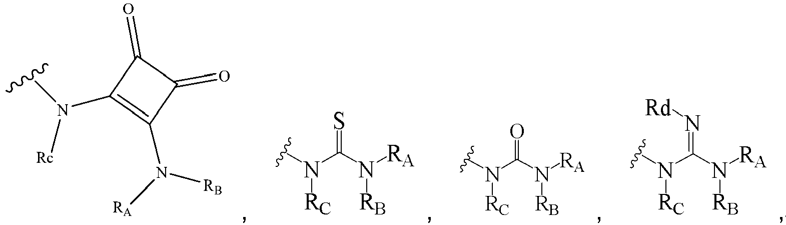 aryl or heterocyclic compounds;
a compound, wherein RA, RB, and RC are each independently selected from the group consisting of: -H, -(CH2)m-H;
m is independently selected from 1, 2, and 3;
R_{d} is independently selected from the group consisting of: -H, -(CH2)n-H, -CN, -NO2, -NH2, -N(CH3)CH3; n is independently selected from 1, 2, and 3.

22. The compound of claim 21, wherein the structures L1a, L1b, and L1c are each interchangeably or replaceably connectable with L2, and/or the structure L2 is interchangeably or replaceably connectable with L1a, L1b, or L1c; and wherein the number of L2 groups in each structural formula is at least one and no more than two.

23. The compound of claim 21 or 22, wherein Rx is alicyclic, and Rx is alicyclic having at least one of the structures (II-1), (II-2), (II-3), and (II-4), the structures (II-1), (II-2), (II-3), and (II-4) are as follows: 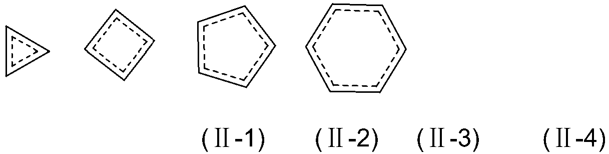
or Rx is a pharmaceutically acceptable salt, prodrug, or isomer of the structural formula (II-1), (II-2), (II-3) or (II-4);
wherein, the "------" bond in the structural formula (II-1), (II-2), (II-3) and (II -4) is a single or double bond.

24. The compound of claim 21 or 22, wherein:
R1 is alicyclic, and R1 is a vitamin E succinate, adamantane or its derivative; and/or
R2 is alicyclic, and R2 is a vitamin E succinate, adamantane or its derivative; and/or
G is a heterocycle, and G is a nitrogen-containing heterocycle, oxygen-containing heterocycle or sulfur-containing heterocycle compound.
